(19) **Europäisches Patentamt**
**European Patent Office**
**Office européen des brevets**

(11) **EP 1 951 748 B1**

(12) **EUROPEAN PATENT SPECIFICATION**

(45) Date of publication and mention
of the grant of the patent:
**24.07.2013 Bulletin 2013/30**

(51) Int Cl.:
*C07K 14/18* (2006.01)    *C12N 15/51* (2006.01)
*C07K 16/10* (2006.01)    *C07K 16/40* (2006.01)
*G01N 33/576* (2006.01)    *C12Q 1/37* (2006.01)
*C12Q 1/68* (2006.01)

(21) Application number: **06837685.4**

(22) Date of filing: **13.11.2006**

(86) International application number:
**PCT/US2006/044363**

(87) International publication number:
**WO 2007/059221 (24.05.2007 Gazette 2007/21)**

(54) **HEPATITIS C VIRUS VARIANTS**

VARIANTEN DES HEPATITIS-C-VIRUS

VARIANTES DU VIRUS DE L'HÉPATITE C

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **11.11.2005 US 735577 P**
**25.10.2006 US 854598 P**

(43) Date of publication of application:
**06.08.2008 Bulletin 2008/32**

(60) Divisional application:
**11169123.4 / 2 392 588**
**11169127.5 / 2 392 589**
**11169130.9 / 2 392 590**

(73) Proprietor: **Vertex Pharmaceuticals, Inc.**
**Cambridge, MA 02139 (US)**

(72) Inventors:
• **LIN, Chao**
**Winchester, MA 01890 (US)**
• **KIEFFER, Tara**
**Brookline, MA 02446 (US)**
• **SARRAZIN, Christoph,**
**c/o J.W. Goethe University Hospital Frankfurt**
**60590 Frankfurt am Main (DE)**
• **KWONG, Ann**
**Cambridge, MA 02138 (US)**

(74) Representative: **Vossius & Partner**
**Siebertstrasse 4**
**81675 München (DE)**

(56) References cited:
**WO-A-2006/112482    WO-A-2007/031867**

• **UENO TAKAMASA ET AL: "Isolation and characterization of monoclonal antibodies that inhibit hepatitis C virus NS3 protease" JOURNAL OF VIROLOGY, THE AMERICAN SOCIETY FOR MICROBIOLOGY, US, vol. 74, no. 14, July 2000 (2000-07), pages 6300-6308, XP002398322 ISSN: 0022-538X**
• **LIN CHAO ET AL: "In vitro resistance studies of hepatitis C virus serine protease inhibitors, VX-950 and BILN 2061: structural analysis indicates different resistance mechanisms." THE JOURNAL OF BIOLOGICAL CHEMISTRY 23 APR 2004, vol. 279, no. 17, 23 April 2004 (2004-04-23), pages 17508-17514, XP002407557 ISSN: 0021-9258 cited in the application**
• **LIN CHAO ET AL: "In vitro studies of cross-resistance mutations against two hepatitis C virus serine protease inhibitors, VX-950 and BILN 2061." THE JOURNAL OF BIOLOGICAL CHEMISTRY 4 NOV 2005, vol. 280, no. 44, 4 November 2005 (2005-11-04), pages 36784-36791, XP002441097 ISSN: 0021-9258 cited in the application**
• **HOLLAND-STALEY C A ET AL: "Genetic diversity and response to IFN of the NS3 protease gene from clinicalstrains of the hepatitis C virus" ARCHIVES OF VIROLOGY, NEW YORK, NY, US, vol. 147, no. 7, July 2002 (2002-07), pages 1385-1406, XP008080962 ISSN: 0304-8608**

**(Cont. next page)**

EP 1 951 748 B1

- SARRAZIN CHRISTOPH ET AL: "Clinical significance of in vitro replication-enhancing mutations of the hepatitis C virus (HCV) replicon in patients with chronic HCV infection" JOURNAL OF INFECTIOUS DISEASES, CHICAGO, IL, US, [Online] vol. 192, no. 10, 14 October 2005 (2005-10-14), pages 1710-1719, XP008080829 ISSN: 0022-1899
- SARRAZIN CHRISTOPH ET AL: "Dynamic hepatitis C virus genotypic and phenotypic changes in patients treated with the protease inhibitor telaprevir." GASTROENTEROLOGY MAY 2007, vol. 132, no. 5, May 2007 (2007-05), pages 1767-1777, XP002442095 ISSN: 0016-5085
- ZHOU YI ET AL: "Phenotypic characterization of resistant Val36 variants of hepatitis C virus NS3-4A serine protease.", ANTIMICROBIAL AGENTS AND CHEMOTHERAPY JAN 2008 LNKD-PUBMED:17938182, vol. 52, no. 1, January 2008 (2008-01), pages 110-120, ISSN: 0066-4804

Remarks:
The file contains technical information submitted after the application was filed and not included in this specification

**Description**

**Field of the Invention**

The present invention relates to an isolated HCV polynucleotide encoding an HCV NS3 protease, or a fragment thereof; wherein the protease or the fragment thereof has serine protease activity; and, wherein a codon in the polynucleotide encodes M or G at the codon position 36 of a polynucleotide sequence encoding a wild-type 181-amino acid HCV NS3 protease domain. The disclosure generally relates to hepatitis C virus (HCV) variants.

**Background of the Invention**

Hepatitis C virus (HCV) infects more than 170 million people worldwide and is the leading cause of chronic hepatitis, which can ultimately lead to end-stage liver cirrhosis and hepatocellular carcinoma. The standard treatment for HCV infection is currently pegylated interferon alpha (Peg-IFN) in combination with ribavirin (RBV). The goal of HCV therapy is to eliminate viral infection by obtaining a sustained viral response (SVR) as defined by having undetectable HCV-RNA in the blood after 6 months of antiviral treatment. Unfortunately, the current treatment is not effective in about 50% of subjects with genotype 1, and the side effects are significant. Thus, new antiviral targets and improved treatment strategies are needed (Pawlotsky, J. M., and J. G. McHutchison, 2004, Hepatitis C. Development of new drugs and clinical trials: promises and pitfalls. Summary of an AASLD hepatitis single topic conference, Chicago, IL, February 27-March 1, 2003, Hepatology 39:554-67; Strader, et al., 2004, Diagnosis, management, and treatment of hepatitis C. Hepatology 39:1147-71).

The non-structural (NS) 3-4A protease is essential for HCV replication and a promising target for new anti-HCV therapy. VX-950, a potent and specific NS3-4A protease inhibitor demonstrated substantial antiviral activity in a phase 1b trial of subjects infected with HCV genotype 1 (Study VX04-950-101). The degree to which a subject responds to treatment and the rate at which viral rebound is observed could in part be due to genotypic differences in sensitivity to the protease inhibitor. The rapid replication rate of HCV, along with the poor fidelity of its polymerase, gives rise to an accumulation of mutations throughout its genome (Simmonds, P., 2004, Genetic diversity and evolution of hepatitis C virus - 15 years on. J. Gen. Virol. 85:3173-88). The degree to which sequence variability in the protease region affects the catalytic efficiency of the enzyme or the binding of an inhibitor is not known. Additionally, the generation of numerous viral genomes with remarkable sequence variation presents potential problems of emerging drug resistant virus in subjects treated with antiviral therapy. Indeed, drug resistance against antiviral drugs, such as HIV protease inhibitors, is well documented (Johnson, et al., 2004, Top. HIV Med. 12:119-24). Drug resistant mutations have already been shown to develop in vitro in the presence of HCV protease inhibitors (Lin, et al., 2005, In vitro studies of cross-resistance mutations against two hepatitis C virus serine protease inhibitors, VX-950 and BILN 2061. J. Biol. Chem. 280: 36784-36791; Lin, et al., 2004, In vitro resistance studies of hepatitis C virus serine protease inhibitors, VX-950 and BILN 2061: Structural analysis indicates different resistance mechanisms. J. Biol. Chem. 279:17508-17514; Lu, et al., 2004, Antimicrob. Agents Chemother. 48:2260-6; Trozzi, et al., 2003, In vitro selection and characterization of hepatitis C virus serine protease variants resistant to an active-site peptide inhibitor. J. Virol. 77:3669-79). Mutations resistant to the protease inhibitor BILN 2061 have been found at positions R155Q, AI56T, and D168V/A/Y in the NS3 gene, but no mutations have yet been observed in the NS4 region or in the protease cleavage sites. A VX-950 resistance mutation has also been found in vitro at position A156S. Cross-resistant mutations against both VX-950 and BILN 2061 have also been shown to develop in vitro at position 156 (A156V/T) (Lin, et al., 2005, *supra*).

Accordingly, there exists a need in identifying mutated HCVs or other viruses that exhibit resistance to drugs or other therapies and in developing new viral therapeutics effective against these mutated viruses.

**Summary of the Invention**

Accordingly, the disclosure describes HCV variants, and related methods and compositions. In particular, HCV variants and variant HCV proteases that have reduced sensitivity to one or more protease inhibitors such as VX-950 are described.

The present invention relates to the following items:

[1] An isolated HCV polynucleotide encoding an HCV NS3 protease, or a fragment thereof; wherein the protease or the fragment thereof has serine protease activity; and, wherein a codon in the polynucleotide encodes M or G at the codon position that corresponds to codon position 36 of a polynucleotide sequence encoding a wild-type 181-amino acid HCV NS3 protease domain.

[2] The HCV polynucleotide of [1], wherein the HCV polynucleotide comprises a portion of a polynucleotide sequence encoding a wild-type HCV NS3 protease; wherein said portion encodes a fragment of an HCV NS3 protease that has serine protease activity.

[3] An HCV variant comprising a polynucleotide according to any of [1] or [2].

[4] An isolated HCV NS3 protease comprising an amino acid sequence in which the amino acid at the position corresponding to amino acid position 36 of a wild-type 181-amino acid HCV NS3 protease domain is an M or G.

[5] The HCV protease of [4], wherein the HCV NS3 protease comprises a portion of a wild-type HCV NS3 protease amino acid sequence; wherein said portion encodes a fragment of the HCV NS3 protease that has serine protease activity.

[6] The HCV protease of [4] comprising an NS4A cofactor.

[7] An anti-HCV protease antibody that is specific to an HCV NS3 protease encoded by the polynucleotide of [1].

[8] An expression system comprising the HCV polynucleotide of any one of [1] or [2].

[9] The expression system of [8] comprising a vector, wherein the vector comprises the HCV polynucleotide of any one of [1] or [2] operably linked to a promoter.

[10] A host cell transfected, transformed, or transduced with the vector of [9].

[11] The expression system of [8] that is an mRNA display system.

[12] A method for identifying a candidate compound for treating an HCV infection in a patient comprising:

(a) providing a sample infected with an HCV variant; wherein the HCV variant comprises a nucleic acid encoding an HCV NS3 protease that comprises the polynucleotide of [1];
(b) assaying the ability of the candidate compound in inhibiting an activity of the HCV variant in the sample.

[13] The method of [12], wherein the activity of the HCV variant is replication.

[14] An *in vitro* method for identifying a candidate compound for treating or preventing an HCV infection in a patient comprising:

(a) providing a replicon RNA comprising the polynucleotide of [1 ];
(b) determining whether the candidate compound inhibits replication of the replicon RNA of (a).

[15] An in vitro method for identifying a candidate compound for treating an HCV infection in a patient comprising:

(a) providing an HCV NS3 protease and a protease substrate, wherein the protease is encoded by the polynucleotide of [1], and wherein the protease and the substrate are in a cell-based system or in a cell-free system;
(b) contacting the HCV NS3 protease with the candidate compound in the presence of the substrate; and
(c) determining whether the HCV NS3 protease activity is reduced.

[16] A method for evaluating a candidate compound for treating an HCV infection in a patient comprising:

(a) introducing a vector comprising a polynucleotide encoding an HCV NS3 protease, or a fragment thereof; wherein the protease or the fragment thereof has serine protease activity; wherein the protease or fragment thereof is encoded by the polynucleotide of [1], and an indicator gene encoding an indicator in vitro into a host cell;
(b) culturing the host cell; and
(c) measuring the indicator in the presence of the candidate compound and in the absence of the candidate compound.

[17] An *in vitro* method for identifying a compound able to rescue the activity of VX-950 against an HCV NS3 protease that has become resistant to VX-950 comprising:

(a) contacting the HCV NS3 protease with a candidate compound; wherein the protease is encoded by the polynucleotide of [1];

(b) assaying the ability of VX-950 to inhibit the activity of the protease of (a).

[18] A method for identifying a compound effective in reducing an HCV NS3 protease activity, comprising:

(a) obtaining a three dimensional model of an HCV NS3 protease encoded by the polynucleotide of [1];

(b) designing or selecting a compound;

(c) evaluating the ability of the compound to bind to or interact with the protease of (a).

[19] The method of [18] further comprising evaluating the ability of a designed or selected compound to inhibit an HCV NS3 protease activity in a cell-free or cell-based assay or assaying the ability of a designed or selected compound to inhibit HCV replication in an infected cell in vitro or in a sample.

[20] A method for detecting the presence of an HCV variant in a biological sample comprising detecting the presence of a polynucleotide in the biological sample, wherein said polynucleotide encodes an HCV NS3 protease, or a fragment thereof; wherein the protease or the fragment thereof has serine protease activity; and, wherein the protease or fragment thereof is encoded by the polynucleotide of [1].

[21] A diagnostic kit comprising the antibody of [7].

[22] The method of any one of [12] and [14], wherein the HCV polynucleotide comprises a portion of a polynucleotide sequence encoding a wild-type HCV NS3 protease; wherein the portion encodes a fragment of an HCV NS3 protease that has serine protease activity.

[23] The method of any one of [15] to [18], [19] and [20], wherein the wild-type HCV NS3 protease comprises a portion of a wild-type HCV NS3 protease amino acid sequence; wherein the portion encodes a fragment of the HCV NS3 protease that has serine protease activity.

## Brief Description of the Drawings

Figure 1 illustrates a phylogenetic analysis of baseline sequences of the N terminal 543 nucleotides of the NS3 protein from untreated genotype 1 HCV-infected subjects.

Figure 2 shows the baseline $IC_{50}$s of Telaprevir (VX-950) for genotype 1a and 1b protease variants.

Figure 3 illustrates the grouping of subjects based on viral response to VX-950.

Figure 4 (in color) summarizes viral responses corresponding to mutation patterns.

Figure 5 shows enzymatic $IC_{50}$s and fold change from the reference genotype 1a strain of HCV-H of protease single resistance mutants to VX-950.

Figure 6 shows enzymatic $IC_{50}$s and fold change from the reference genotype 1a strain of HCV-H of protease double resistance mutants to VX-950.

Figure 7 shows the inverse correlation between resistance to VX-950 and fitness.

Figure 8 illustrates the structure of two HCV protease inhibitors: VX-950 and BILN 2061.

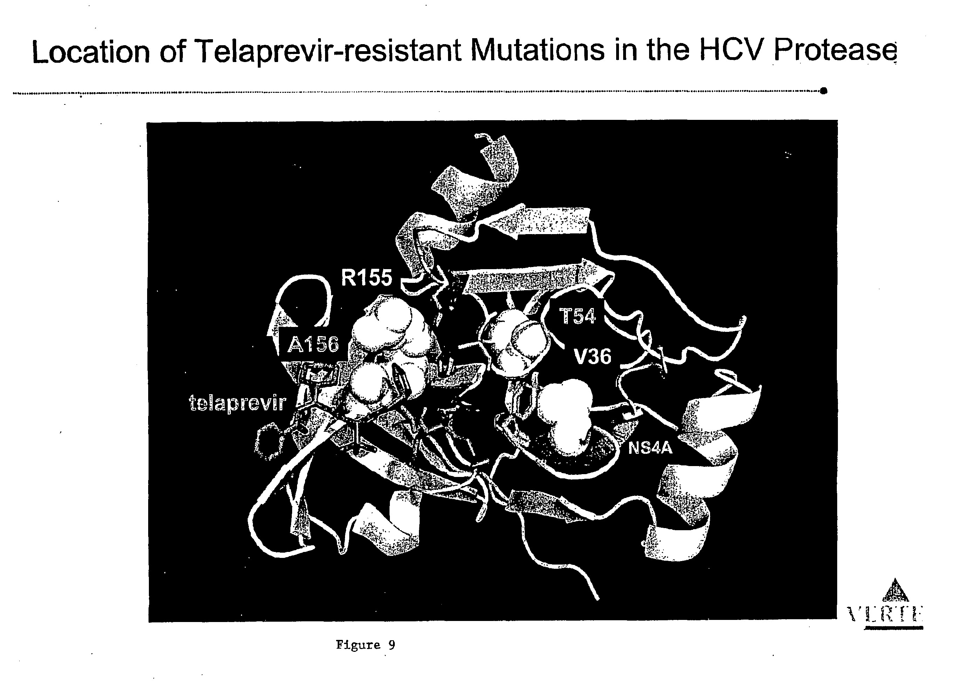

Figure 9 illustrates the location of VX-950 variations in the HCV protease according to structural studies.

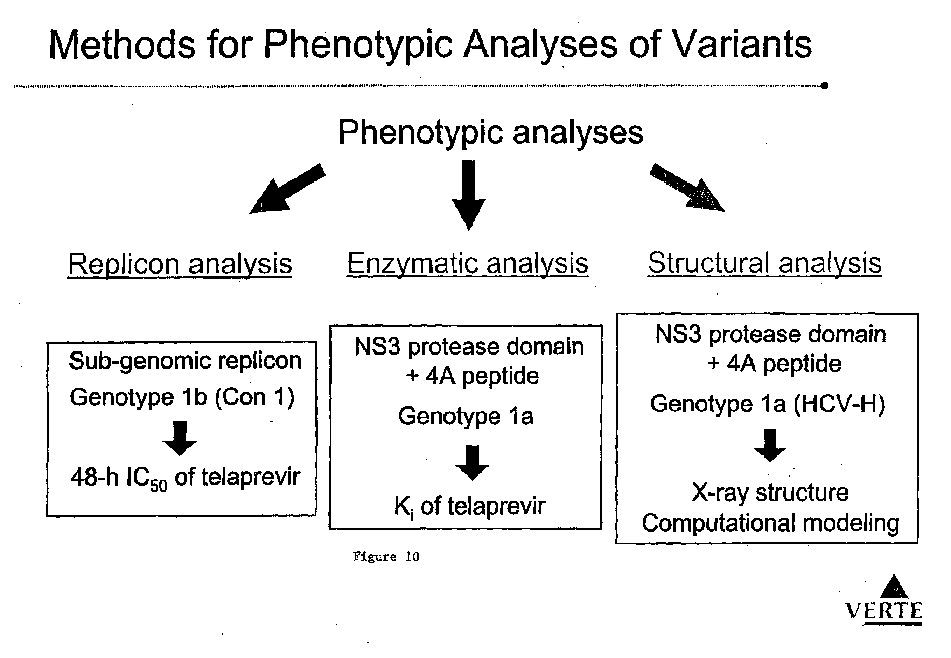

Figure 10 outlines the methods for phenotypic analysis of HCV viral variants.

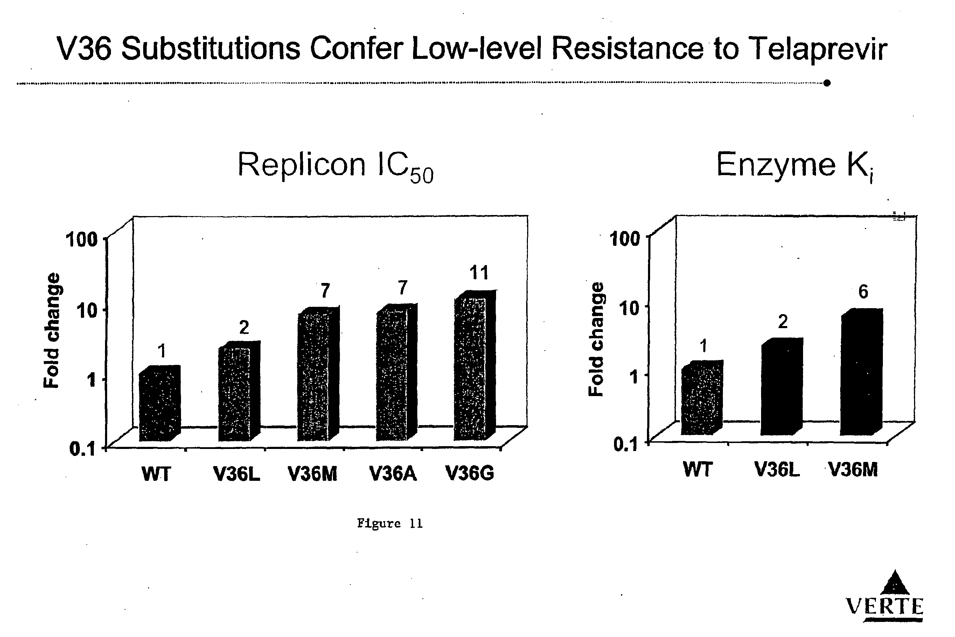

Figure 11 shows that V36 substitutions confer low-level resistance to VX-950.

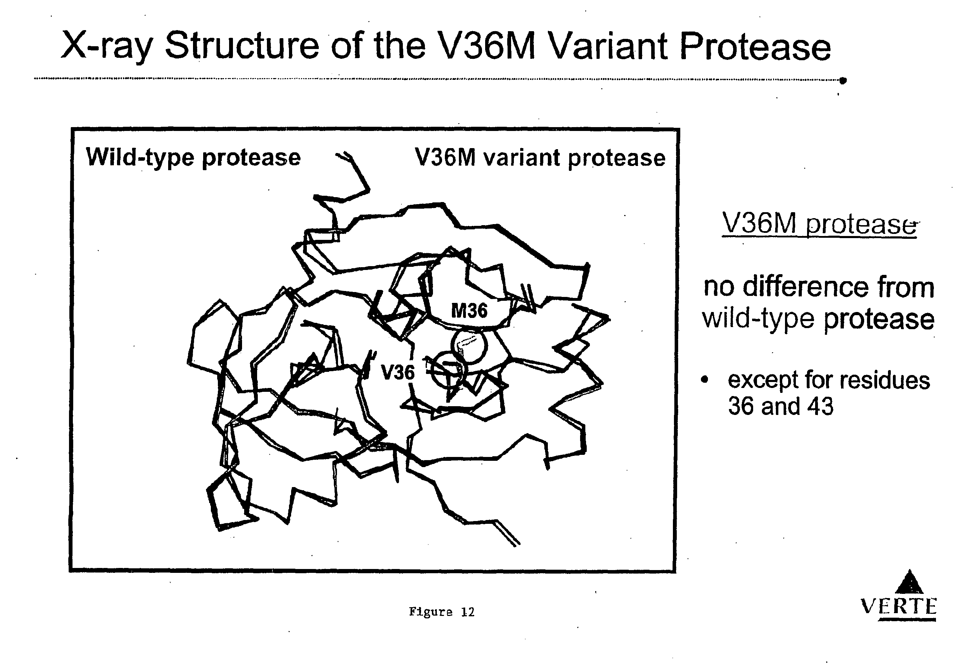

Figure 12 shows X-ray structure of the V36M variant protease.

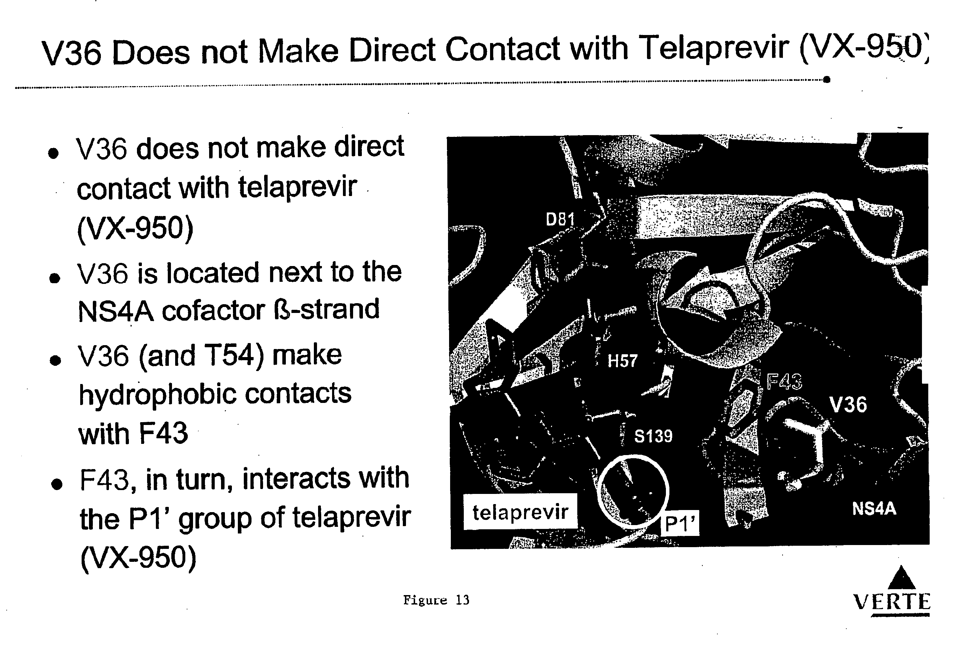

Figure 13 shows that V36 does not make direct contact with VX-950.

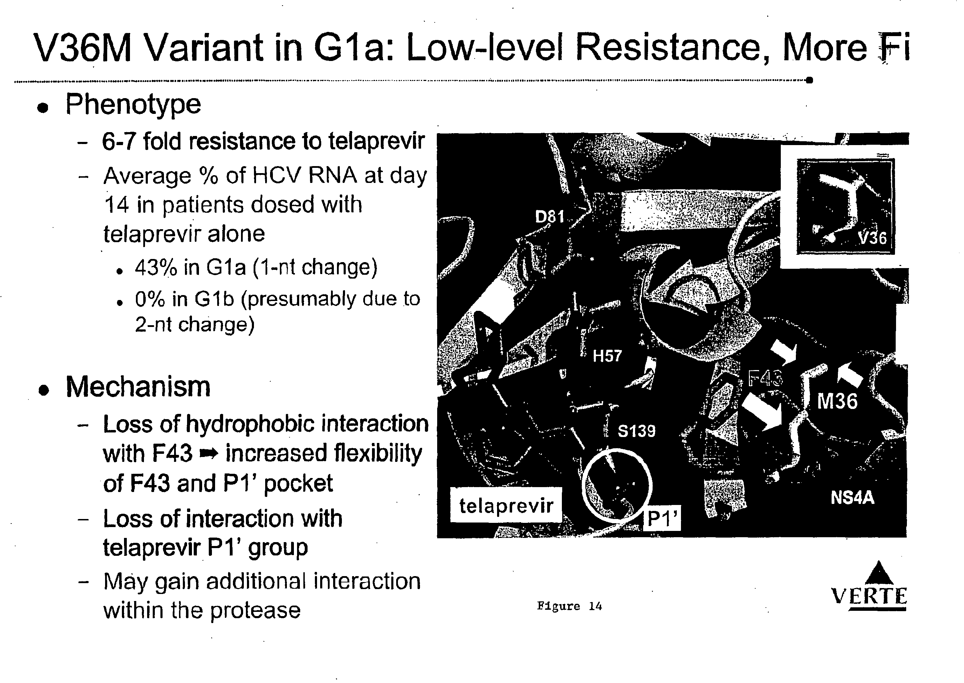

Figure 14 shows the V36M variant in G1a with low-level resistance and better fitness.

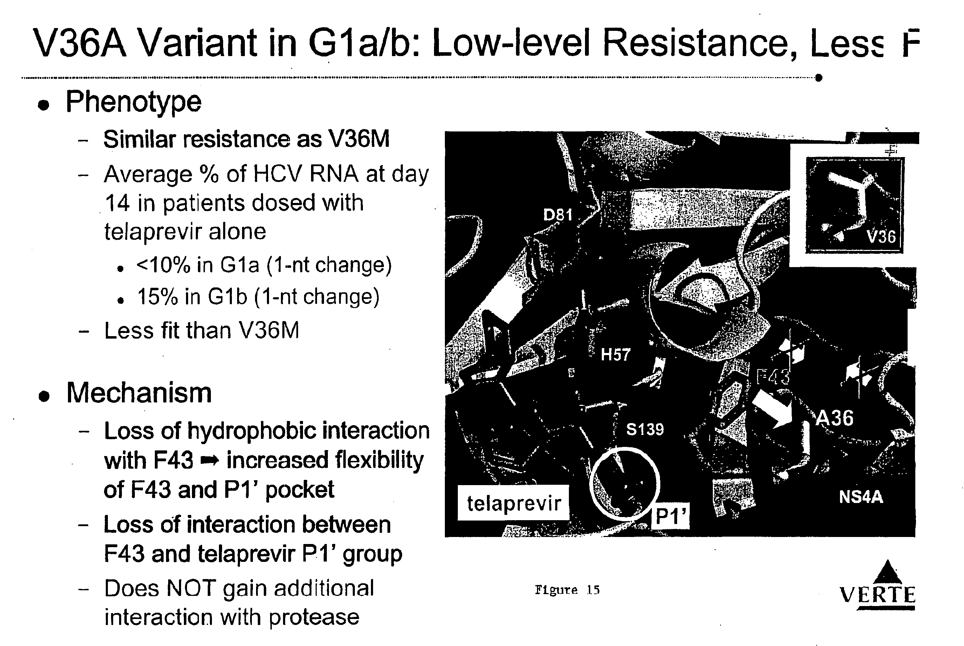

Figure 15 shows the V36A variant in G1a/b with low-level resistance and worse fitness.

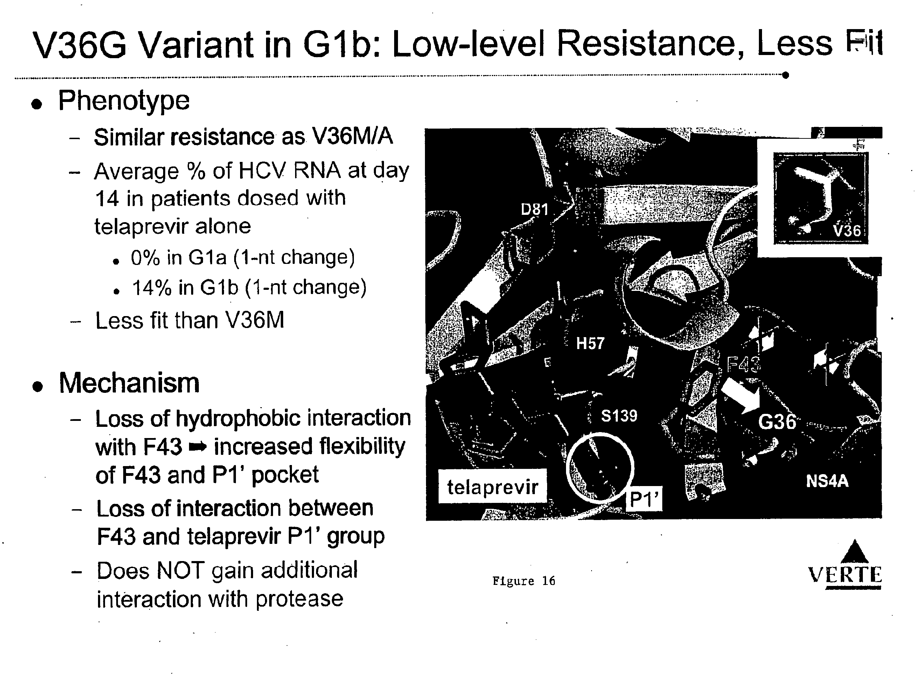

Figure 16 shows the V36G variant in G1b with low-level resistance and worse fitness.

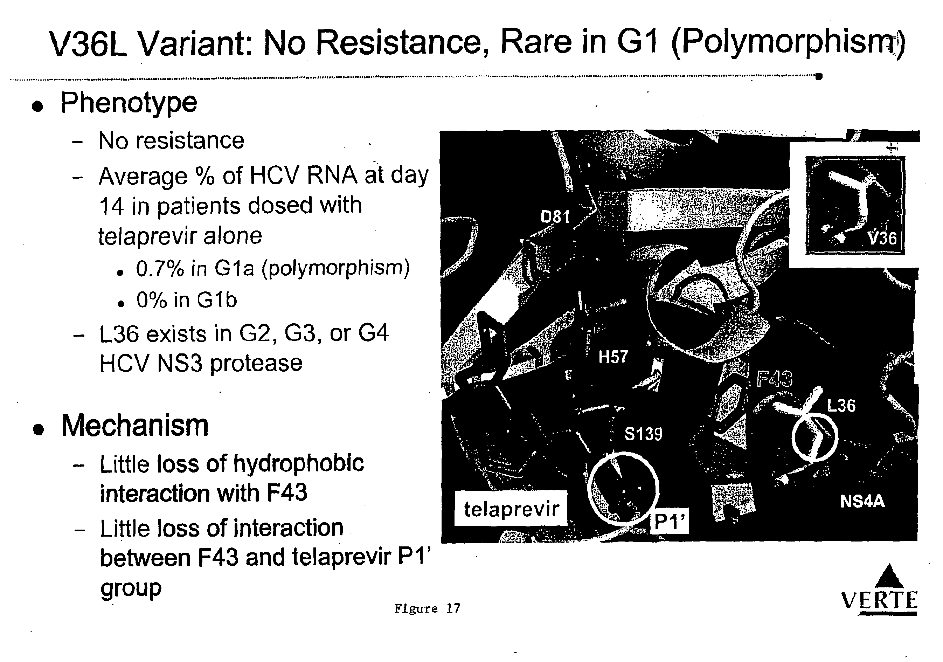

Figure 17 shows the V36L variant with no resistance, which is also rare in G1.

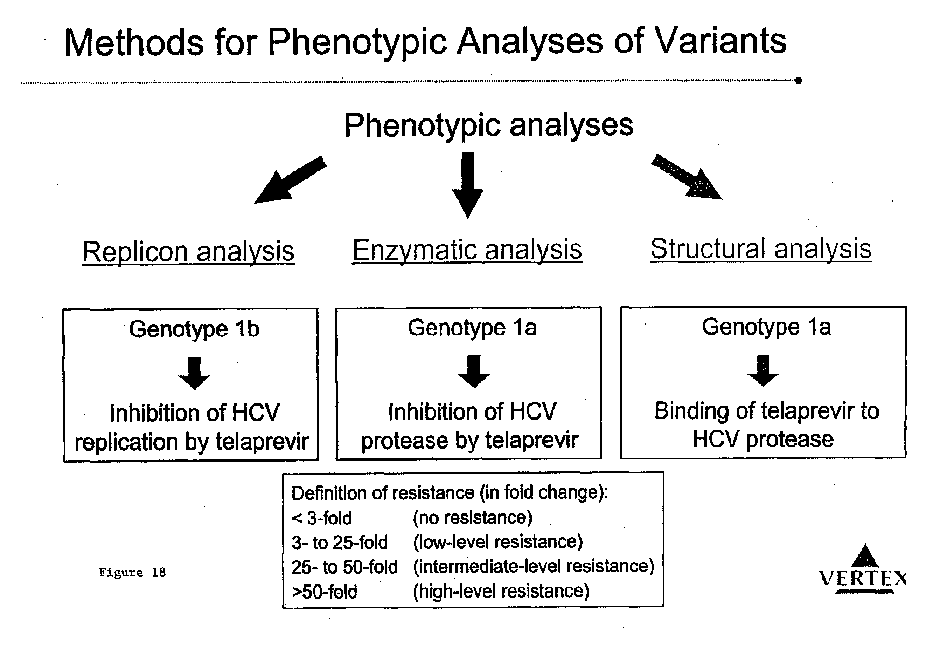

Figure 18 also outlines the methods for phenotypic analyses of HCV viral variants.

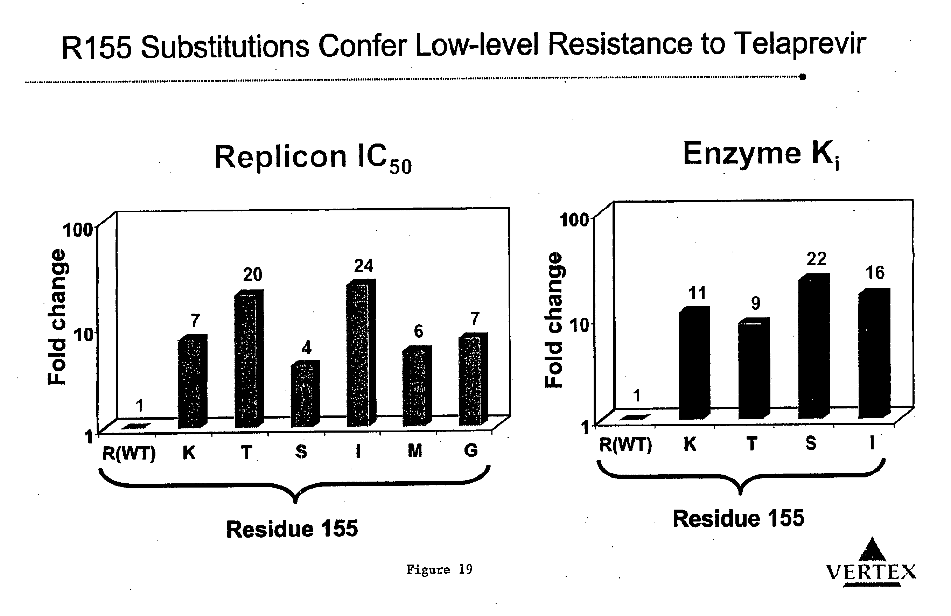

Figure 19 shows that R155 substitutions confer low-level resistance to VX-950.

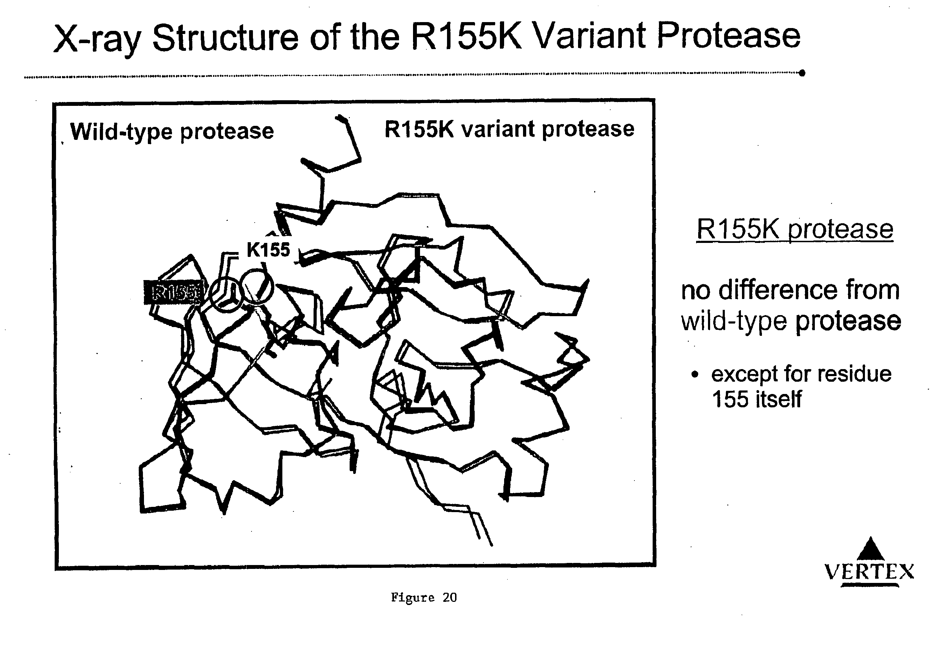

Figure 20 shows the X-ray structure of the R155K variant protease.

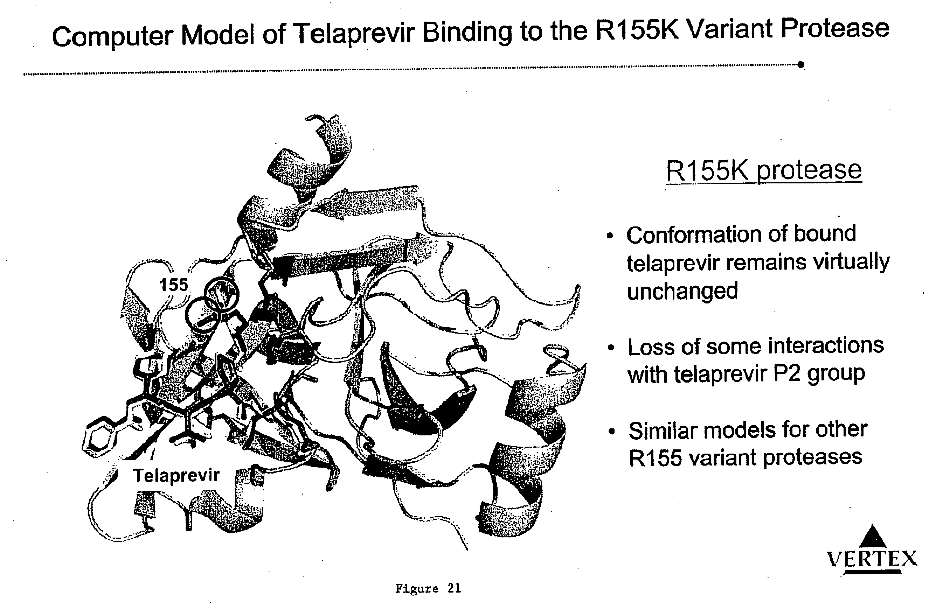

Figure 21 shows the computer model of VX-950 binding to the R155K variant protease.

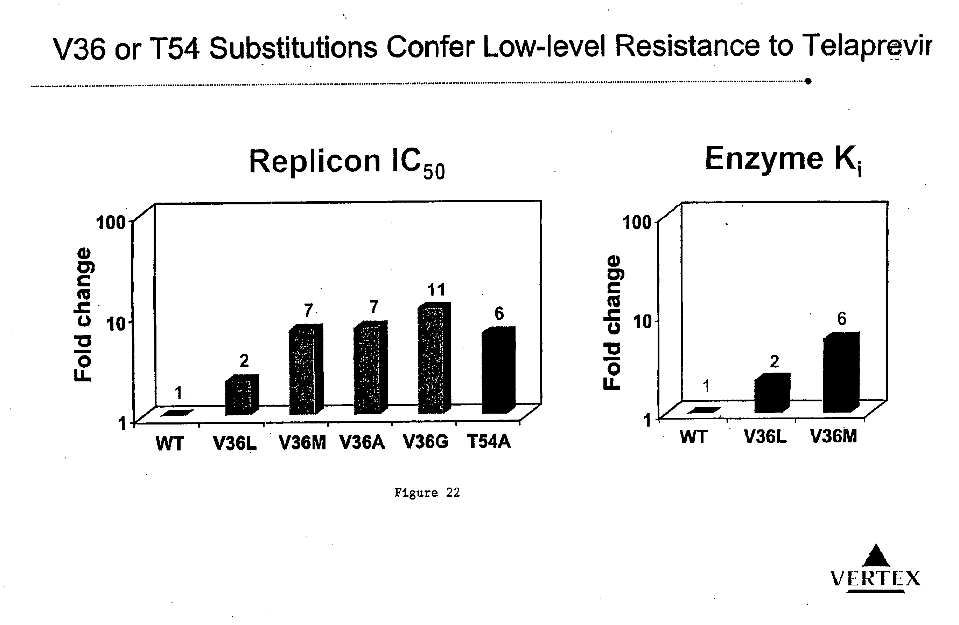

Figure 22 shows that V36 or T54 substitutions confer low-level resistance to VX-950.

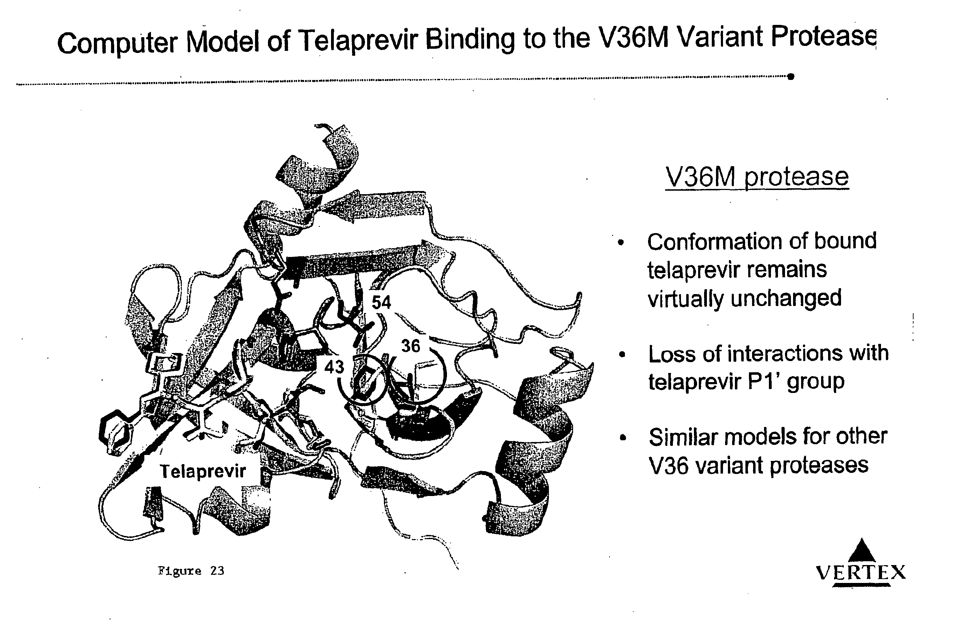

Figure 23 shows the computer model of VX-950 binding to the V36M variant protease.

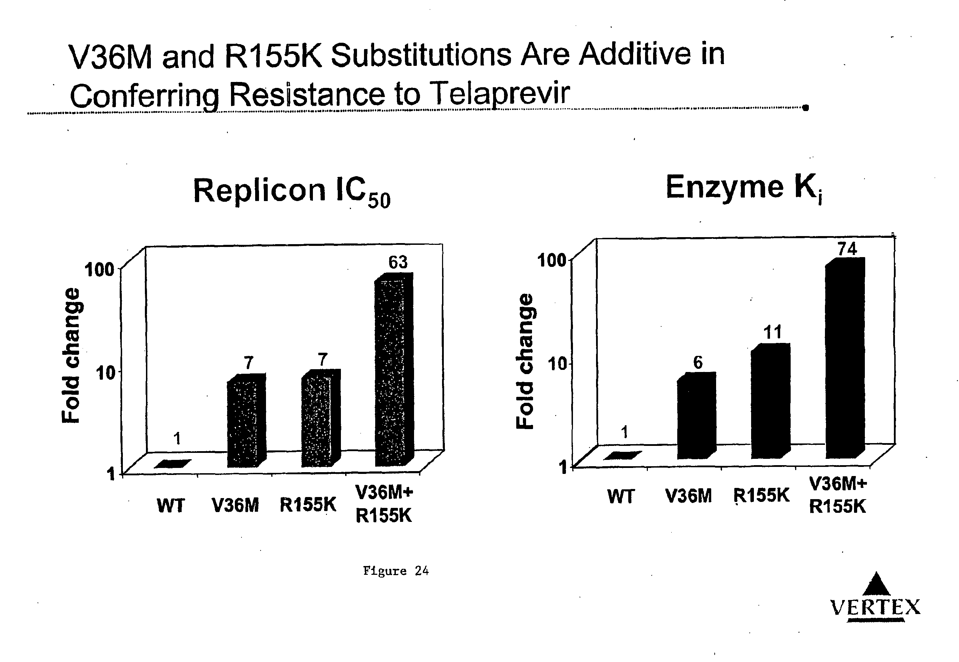

Figure 24 shows that the V36M and R155K substitutions are additive in conferring resistance to VX-950.

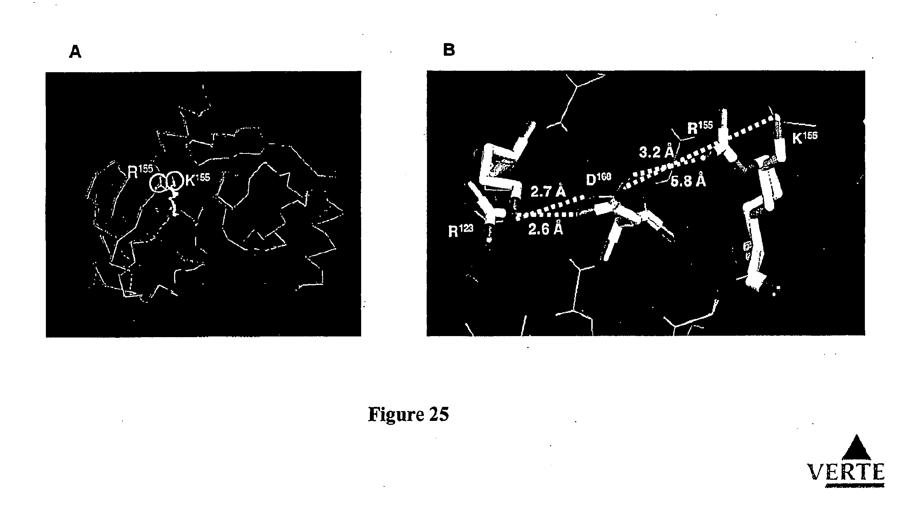

Figure 25 shows results of structural studies: (A) Superimposition of the X-ray structure of the $Lys^{155}$ variant and the $Arg^{155}$ wild-type NS3 protease domain in a complex with the NS4A co-factor. The $C\alpha$ atom traces of both the wild-type (in blue) and the R155K variant (in red) proteases are shown as lines. The residue 155 is highlighted with either ball and stick model ($Arg^{I55}$) or Liquorice model ($Lys^{155}$) with nitrogens in blue and oxygens in red. (B) Superposition of side chains of $Arg^{155}$, $Asp^{168}$ and $Arg^{123}$ in the wild type NS3-4A with that of corresponding $Lys^{155}$, $Asp^{168}$ and $Arg^{123}$ in the R155K variant. Three residues of the R155K variant protease ($Arg^{123}$, $Asp^{168}$, and $Lys^{155}$) are shown in the Liquorice model, so is the $Arg^{155}$ of the wild-type protease. The $Arg^{123}$ and $Asp^{168}$ residues of the wild-type protease are shown as thin lines. All nitrogens are colored in blue and oxygens in red.

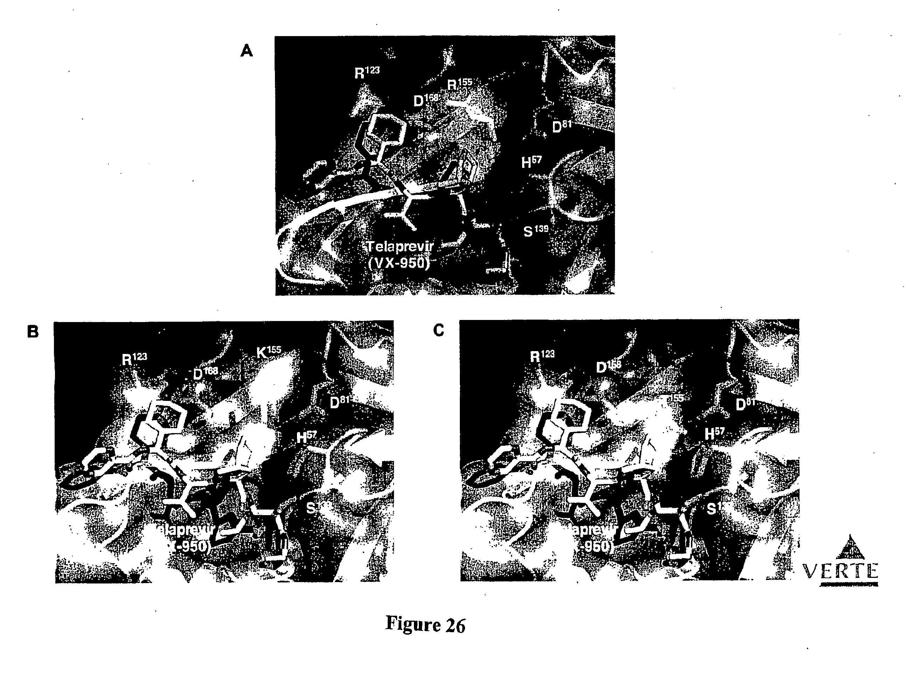

Figure 26 shows computational models of a co-complex of telaprevir with the HCV NS3 protease domains in a complex with an NS4A cofactor. In all three models, including the wild-type (A), R155K (B) or R155T (C) variant proteases, telaprevir is shown in a stick diagram colored in light blue with nitrogens in blue and oxygens in red. The active site residues ($His^{57}$, $Asp^{81}$, and $Ser^{139}$) are shown as gray sticks. The $Arg^{123}$ and $Asp^{168}$ residues are colored in purple, while residue 155 side-chain is colored in yellow. The $Lys^{155}$ or $Thr^{155}$ side-chain remains in the extended conformation making minimal contacts with the P2 group of telaprevir.

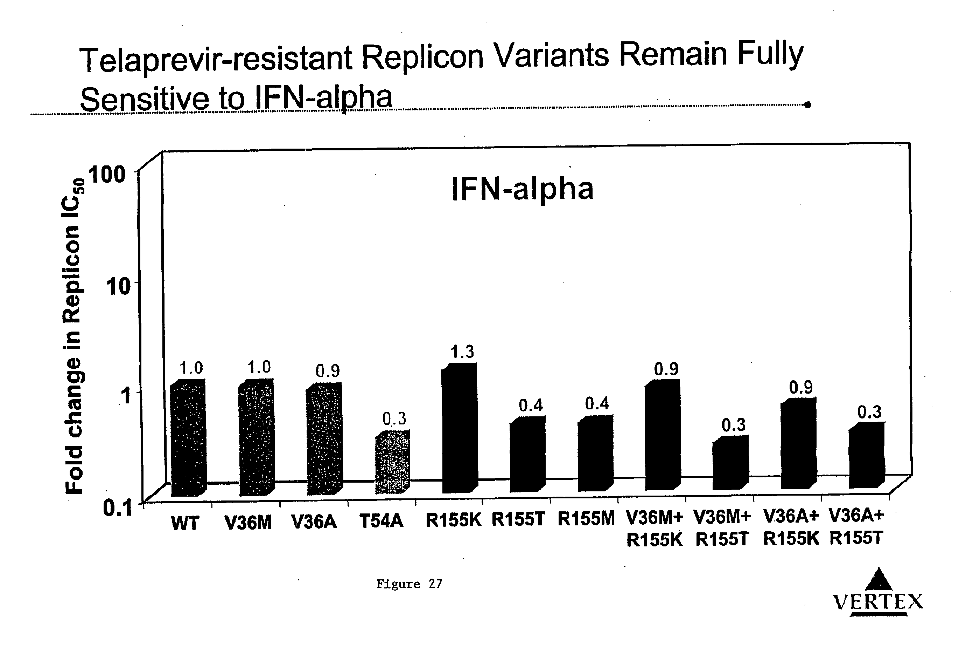

Figure 27 shows that the VX-950 resistant replicon variants remain fully sensitive to IFN-alpha.

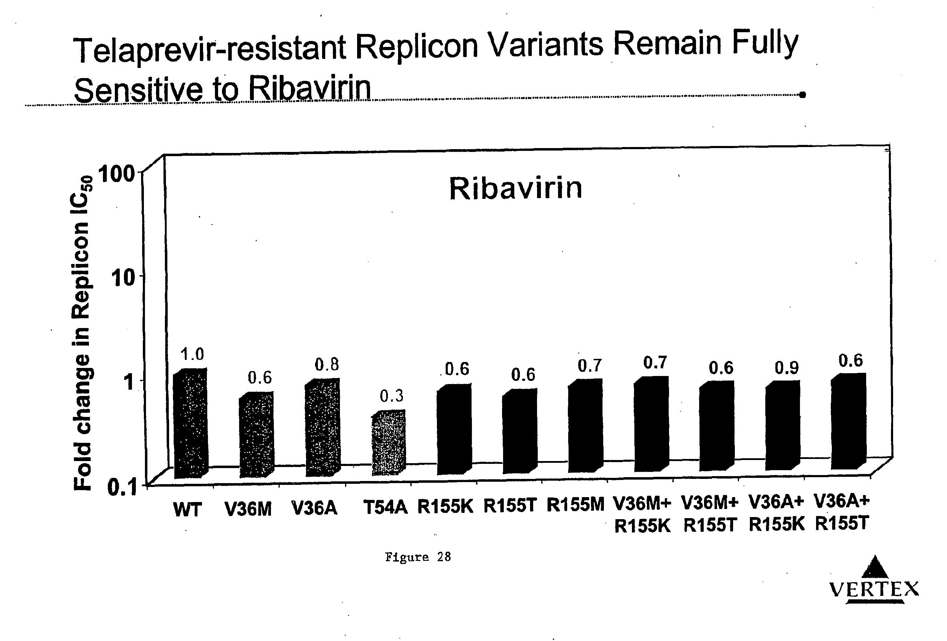

Figure 28 shows that the VX-950 resistant replicon variants remain fully sensitive to Ribavirin.

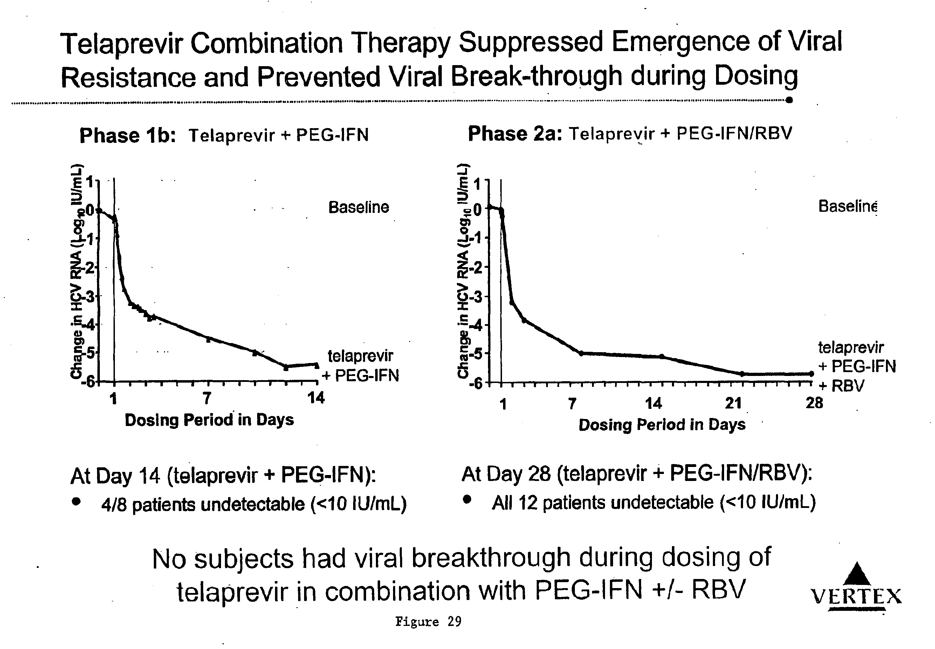

Figure 29 shows that VX-950 combination therapy suppressed emergence of viral resistance and prevented viral breakthrough during dosing.

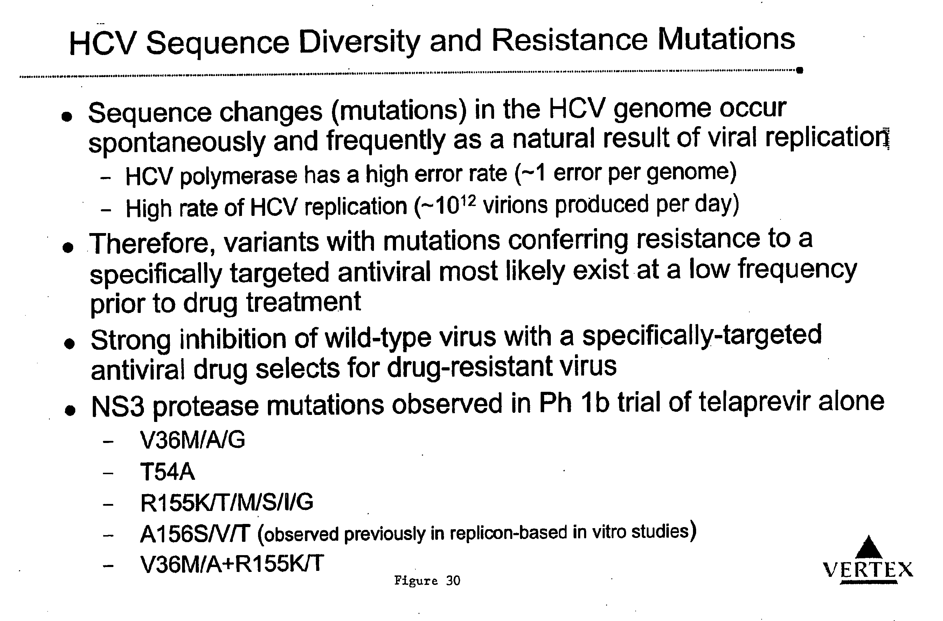

Figure 30 provides summary points regarding HCV sequence diversity and resistance mutations.

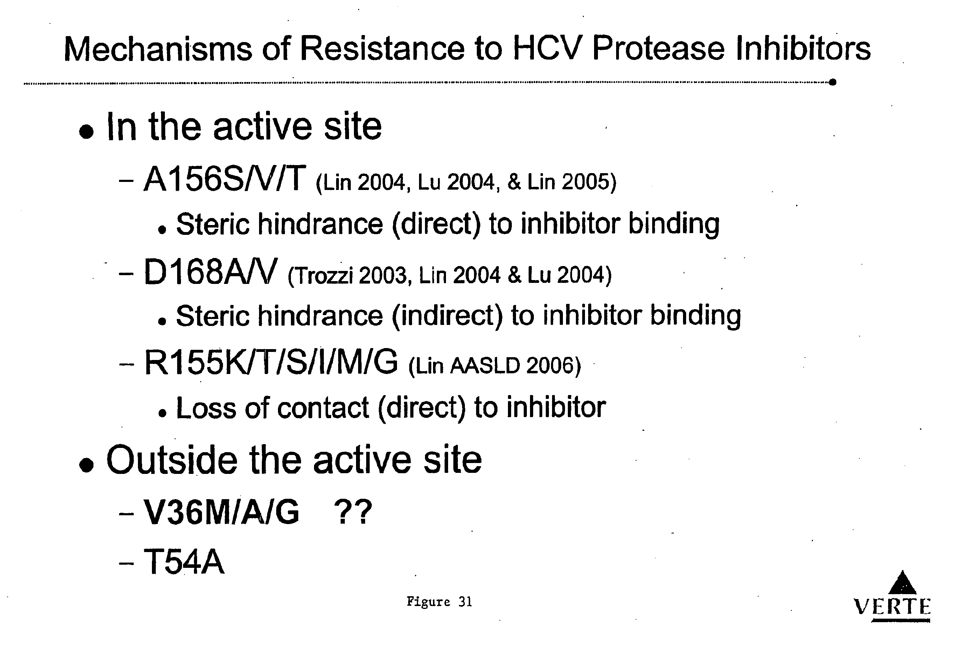

Figure 31 summarizes the mechanisms of viral variants resistance to HCV protease inhibitors including previous studies.

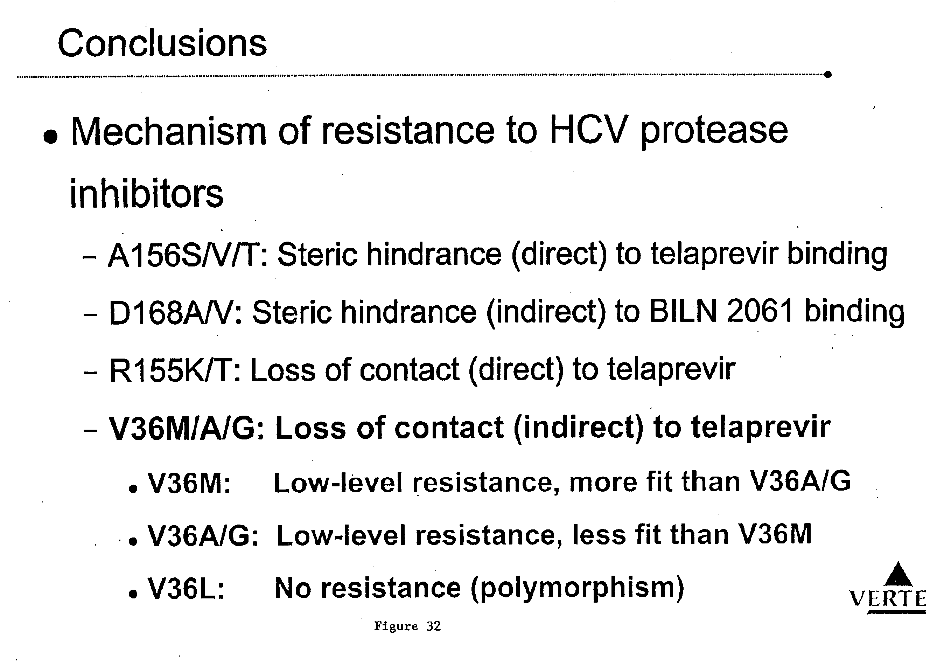

Figure 32 outlines conclusions regarding the mechanisms of viral variants resistance to HCV protease inhibitors including previous and present studies.

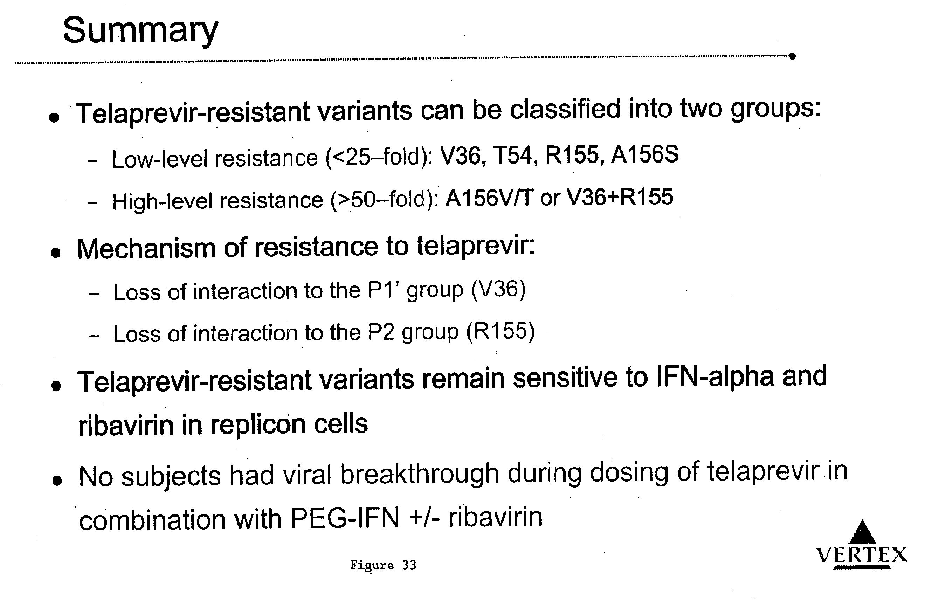

Figure 33 summarizes certain conclusions based on the present studies.

**Detailed Description of the Invention**

The disclosure relates to HCV variants. In particular, HCV variants that exhibit resistance to a protease inhibitor are described. Also disclosed are methods and compositions related to the HCV variants. The methods and compositions are useful in identifying viral variants, including variants of an HCV and other viruses, evaluating and identifying anti-viral compounds, and developing and optimizing therapeutics against viral infections.

The disclosure is based on a study that first characterized the extent of sequence diversity within the NS3 protease domain of an HCV isolated from 34 subjects enrolled in a clinical trial, Study VX04-950-101, before dosing with VX-950. Emergence of resistance to VX-950 in vivo was then monitored by sequence analysis of the protease NS3-4A region in the subjects after 14 days of dosing with VX-950. A follow-up sample was further collected 7 to 10 days after the end of dosing to see whether any drug-resistant mutations that developed during dosing was maintained in the plasma after removal of VX-950. Any mutations found to have increased in the population above baseline were considered potential drug resistant mutations. Because drug-resistance mutations may take some time to accumulate to a measurable level, the study included a new method to detect minor populations of variants (instead the dominant species in a population of wild-type viruses and viral variants), which involved obtaining sequences from many (e.g., 80-85) individual viral clones per subject per time point, so that viral variants that may emerge in 14 days of dosing with VX-950 with a sensitivity of down to about 5% of the population can be detected and identified. Such 80/85 individual viral clones may represent up to 80/85 different virions.

*HCV Variants and Related Polynucleotides and Proteases*

The disclosure describes HCV variants. As described herein, an HCV variant includes a polynucleotide sequence that encodes an HCV protease with reduced sensitivity to a protease inhibitor (also termed "a variant HCV protease"), such as VX-950. As used herein, a wild-type HCV refers to an HCV comprising a polynucleotide (also termed "a wild-type polynucleotide") that encodes an HCV protease with normal or desirable sensitivity to a protease inhibitor, in particular the protease inhibitor VX-950. Similarly, a wild-type HCV protease refers to an HCV protease with normal or desirable sensitivity to a protease inhibitor, in particular the protease inhibitor VX-950.

As used here in, an HCV can be an HCV of any genotype or subtype, for example, genotypes 1-6.

As used herein, an "NS3 protease" or an "HCV NS3 protease" refers to an HCV NS protein 3 or a portion thereof that has serine protease activity. For example, an NS3 protease can be the NS3 protein as represented by the first 631 amino acid sequence of a wild-type NS3 protein (685 amino acids); alternatively, an NS3 protease can be a protein as represented by the first 181 amino acids of a wild-type NS3 protein(685 amino acids); the 181-amino acid fragment is also referred to as the NS3 protease domain in the art. An NS3 protease can also be an NS3-NS4A protein complex, such as the complexes described in U.S. Patent Nos. 6,653,127; 6,211,338. An "NS3 protease activity" means the protease activity of an HCV NS protein 3 or a portion thereof in the presence or absence of an NS4A protein or a biologically active portion thereof. An NS4A protein, such as for example as represented by the last 54 amino acid sequence of a wild-type|NS3 protein (685 amino acids), usually functions as a co-factor for an NS3 protease and can form an NS3-NS4A serine protease complex; a biologically active portion of an NS4A protein refers to a fragment of an NS4A protein that retains the NS4A protein's function as a co-factor for an NS3 protease.

The disclosure also provides isolated HCV variants, isolated variant HCV NS3 proteases, and isolated polynucleotide that encodes a variant HCV NS3 protease. The term "isolated" generally means separated and/or recovered from a component of natural environment of a subject virus, protease, or polynucleotide.

In the invention, a variant HCV protease is a variant HCV NS3 protease that comprises an amino acid sequence in which the amino acid(s) at position 36 of a wild-type HCV NS3 protease is different from the amino acid at said corresponding position of the wild-type HCV NS3 protease as defined in the claims. The wild-type HCV NS3 protease may comprise an amino acid sequence of a wild-type NS3 proteins (685 amino acids) or a portion thereof such as for example the first 181 amino acids of a wild-typeNS3 protein (685 amino acids). The isolated HCV NS3 protease may comprise a biologically active analog or fragment of an HCV NS3 protease, for example, the isolated HCV NS3 protease may not have the N-terminal 5,10,15, 20, 30, 35, 40, 45, or 48 amino acids of a wild-type NS3 protein (685 amino acids).

Examples of amino acid substitutions or mutations at various positions of a variant HCV NS3 protease are shown in Tables 1-4. The Tables, Figures, and Examples herein also provide various data obtained with variant HCV NS3 proteases or HCV viral variants as compared to wild-type HCV NS3 proteases or wild-type HCVs.

Biologically active fragments of a variant HCV NS3 protease described herein are also disclosed. Bartenschlager et al. (1994, J. Virology 68: 5045-55) described various fragments of HCV NS3 proteins, for example, the deletion of N-terminal 7 or 23 residues abolished cleavage at NS4B/5A site, but no effect on other cleave sites subjected to the NS3 protease activity; and the deletion ofN-terminal 39 residues abolished cleavage at NS4B/5A and NS5A/5B sites and decreased the NS3 protease activity on the NS4A/4B site. Failla et al. (1995, J. Virology 69: 1769-77) described that the deletion of N-terminal 10 residues of a wild-type NS3 protein had no effect on the NS3 protease activity, the deletion ofN-terminal 15 or 28 residues resulted in a NS3 protein with partial protease activity (normal cleavage at NS5A/5B, but lower at NS4A/4B and NS4B/5A sites), the deletion of N-terminal 49 residues resulted in a completely inactive NS3 protease, and the deletion of C-terminal 10 residues of the NS3 protease domain in the NS3 protein also resulted in a completely inactive NS3 proteases.

Expression systems are described, for example, to make the variant HCV proteases described herein. An expression system may include an expression vector that comprises an HCV polynucleotide described herein Suitable prokaryotic or eukaryotic vectors (e.g., expression vectors) comprising an HCV polynucleotide (or "nucleic acid," used interchangeably herein) described herein can be introduced into a suitable host cell by an appropriate method (e.g., transformation, transfection, electroporation, infection), such that the polynucleotide is operably linked to one or more expression control elements (e.g., in the vector or integrated into the host cell genome). For production, host cells can be maintained under conditions suitable for expression (e.g., in the presence of inducer, suitable media supplemented with appropriate salts, growth factors, antibiotic, nutritional supplements, etc.), whereby the encoded polypeptide is produced. If desired, the encoded protein can be recovered and/or isolated (e.g., from the host cells or medium). It will be appreciated that the method of production encompasses expression in a host cell of a transgenic animal (see e.g., WO 92/03918). An expression system may be based on a cell-free system such as the RNA-protein fusion technology described in U.S. Patent No. 6,258,558 or the in vitro "virus" described in U.S. Patent No. 6,361,943. Ribosome display method can also be used, such as the method described in U.S. Patent No. 5,843,701.

Various assays are provided, for example, assays suitable for phenotyping HCVs. The assays may be directed to measuring a viral activity (e.g., infection, replication, and/or release of viral particles) or an enzymatic activity (e.g. protease activity). Viral activity assays may employ cells or samples infected with a virus or viral variant of which the activity is to be measured. The cells or samples may be obtained from a patient such as a human patient. Alternatively, the cells or samples may be cultured and infected with a virus or viral variant in vitro. Viral activity assays may employ a replicon-based system, such as the replicon-based assays described in Trozzi et al. (13) and U.S. patent application publication No. 20050136400.

Enzymatic activity can be determined in cell-free or cell-based systems which generally include the enzyme of interest or a biologically active fragment or analog thereof and a substrate for the enzyme of interest. For example, U.S. patent application publication No. 20030162169 describes a surrogate cell-based system and method for assaying the activity of HCV NS3 protease. Trozzi et al. (13) describes an in vitro, cell-free protease assay that employs peptide substrates and HPLC systems.

The disclosure takes advantage of the fact that the three-dimensional structure of NS3/4A protease has been resolved (see e.g., WO 98/11134). A three dimensional model of the variant protease of the invention can be obtained; compounds are designed or selected, for example based on their ability to interact with the three-dimensional structure of the variant protease, and the ability to bind to or interact with the protease is evaluated by modeling in silico and can be further evaluated by in vitro or in vivo assays.

The compound may be one identified from a combinatorial chemical library or prepared through rational drug design. The compound may be a compound prepared through rational drug design and derived from the structure of a know protease inhibitor such as VX-950. Rational drug design also may be combined with a systematic method of large-scale screening experiments where potential protease inhibitor drug targets are tested with compounds from combinatorial libraries. Rational drug design is a focused approach, which uses information about the structure of a drug receptor or one of its natural ligands to identify or create candidate drugs. The three-dimensional structure of a protein can be determined using methods such as X-ray crystallography or nuclear magnetic resonance spectroscopy. In the disclosure the three dimensional structure of a variant HCV NS3 protease that contain the mutation of residue 36 may now readily be determined using routine X-ray crystallographic and/or NMR spectroscopy techniques. Rational drug design also may be combined with a systematic method of large-scale screening experiments where potential protease inhibitor drug targets are tested with compounds from combinatorial libraries. Computer programs can be devised to search through databases containing the structures of many different chemical compounds. The computer can select those compounds that are most likely to interact with the variant HCV NS3 proteases, and such identified compound can be tested in assays (e.g., viral or enzymatic assays) suitable for evaluating protease inhibitors.

The identified compound may be formulated into a composition comprising the compound and a pharmaceutically acceptable carrier, adjuvant or vehicle. Preferably the composition contains the compound in an amount effective to reduce the activity of an HCV NS3 serine protease. Even more preferably the composition is formulated for administration to a patient. The compositions also may comprise an additional agent selected from an immunomodulatory agent; an anti-viral agent; a second inhibitor of HCV protease; an inhibitor of another target in the HCV life cycle; a cytochrome P-450 inhibitor; or combinations thereof. The various compositions are described in greater details below.

In another aspect, the disclosure describes antibodies that are specific to an HCV protease, in particular, an HCV NS3 protease with one or more amino acids altered as compared to a wild type HCV NS3 protease. The term "antibody" is used in the broadest sense and specifically covers, without limitation, intact monoclonal antibodies, polyclonal antibodies, chimeric antibodies, multispecific antibodies (e.g., bispecific antibodies) formed from at least two intact antibodies, and antibody fragments, so long as they exhibit the desired biological activity. The term "immunoglobulin" includes a variety of structurally related proteins that are not necessarily antibodies.

"Antibody fragments" comprise a portion of an intact antibody, preferably the antigen-binding or variable region of the intact antibody. Examples of antibody fragments include Fab, Fab", $F(ab')_2$, and Fv fragments; diabodies; linear antibodies (Zapata et al., Protein Eng., 8(10): 1057-1062 (1995)); single-chain antibody molecules; and multispecific antibodies formed from antibody fragments.

"Single-chain Fv" or "scFv" antibody fragments comprise the VH and VL domains of an antibody, wherein these domains are present in a single polypeptide chain. Preferably, the Fv polypeptide further comprises a polypeptide linker between the VH and VL domains that enables the scFv to form the desired structure for antigen binding. For a review of scFv see Pluckthun in The Pharmacology of Monoclonal Antibodies, vol. 113, Rosenburg and Moore, eds. (Springer-Verlag: New York, 1994), pp. 269-315.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90: 6444-6448 (1993).

An antibody against a variant HCV protease may be developed from a known antibody against an HCV NS3 protein, for example through molecular evolution. U.S. patent application publication No. 20040214994 describes an human recombinant antibody against the HCV NS3 protein. Amino acid sequence variants of are prepared by introducing appropriate nucleotide changes into the DNA of a known antibody, or by peptide synthesis. Such variants include, for example, deletions from, and/or insertions into and/or substitutions of, residues within the amino acid sequences of the known antibody. Any combination of deletion, insertion, and substitution is made to arrive at the final construct, provided that the final construct possesses the desired characteristics. The amino acid changes also may alter post-translational processes of the antibody, such as changing the number or position of glycosylation sites.

An antibody of the invention may have diagnostic as well as therapeutic applications. An antibody of the invention may be labeled. The various antibodies of the present disclosure can be used to detect or measure the expression of a variant HCV NS3 protease, and therefore, they are also useful in applications such as cell sorting and imaging (e.g., flow cytometry, and fluorescence activated cell sorting), for diagnostic or research purposes. As used herein, the terms "label" or "labeled" refers to incorporation of another molecule in the antibody. The label may be a detectable marker, e.g., incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). The label or marker can be therapeutic, e.g., a drug conjugate or toxin. Various methods of labeling polypeptides and glycoproteins are known in the art and may be used. Examples of labels for polypeptides include, but are not limited to, the following: radioisotopes or radionuclides ($_e$.g., $^3H$, $^{14}C$, $^{15}N$, $^{35}S$, $^{90}Y$, $^{99}Tc$, $^{111}In$, $^{125}I$, $^{131}I$), fluorescent labels (e.g., FITC, rhodamine, lanthanide phosphors), enzymatic labels (e.g., horseradish peroxidase, beta-galactosidase, luciferase, alkaline phosphatase), chemiluminescent markers, biotinyl groups, predetermined polypeptide epitopes recognized by a secondary reporter (e.g., leucine zipper pair sequences, binding sites for secondary antibodies, metal binding domains, epitope tags), magnetic agents, such as gadolinium chelates, toxins such as pertussis toxin, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Labels may be attached by spacer arms of various lengths to reduce potential steric hindrance.

In certain aspects, kits for use in detecting the presence of an HCV viral, a variant HCV NS3 polynucleotide, or a variant HCV protease in a biological sample can also be prepared. Such kits may include an antibody that recognizes a variant HCV NS3 protease of the invention, as well as one or more ancillary reagents suitable for detecting the presence of a complex between the antibody and the variant protease or a portion thereof. Alternatively, such kits may include a probe or primer, such a probe or primer can hybridize with a variant HCV NS3 polynucleotide of the invention under stringent conditions. A probe or primer may be suitable for PCR or RT-PCR that can be employed to detect a subject of interest. Alternatively, such kits may be based on PCR or non-PCR based HCV diagnostic kits available commercially, e.g., Roche Cobas Amplicor system and Bayer Versant system, including RNA 3.0 assay (bDNA) and RNA Qualitative Assay (TMA). The AMPLICOR HCV MONITOR® Test, v2.0 is an in vitro nucleic acid amplification test for the quantification of HCV RNA in human serum or plasma. The VERSANT® HCV RNA 3.0 Assay (bDNA) is a viral load assay that has been proven to reliably detect a 2 log10 drop. The VERSANT® HCV RNA Qualitative Assay is based on state-of-the-art Transcription-Mediated Amplification (TMA) technology.

*Pharmaceutical Compositions and Formulations*

Another aspect of the disclosure describes pharmaceutical compositions or formulations including a compound described herein, for example, a secondary compound that is identified as being able to rescue the activity of VX-950, or a compound that is identified as effective against an HCV variant (e.g., capable of reducing replication of the viral variant) and/or a variant HCV NS3 protease (e.g., capable of reducing the enzymatic activity of the variant protease).

Another aspect of the disclosure describes uses of a compound described herein in the manufacture of a medicament, such as a medicament for treating an HCV infection in a patient.

Another aspect of the disclosure relates to a pharmaceutically or therapeutically effective amount of a compound described herein alone or in combination (sequentially or contemporaneously) with another anti-viral agent for use in methods for treating an HCV infection in a patient "Effective amount" of a compound or agent generally refers to those amounts effective to reproducibly reduce HCV NS3 protease expression or activity, HCV production, replication, or virulence, HCV infection, or produce an amelioration or alleviation of one or more of the symptoms of HCV infection in comparison to the levels of these parameters in the absence of such a compound or agent

In another aspect, the compounds and compositions as described herein include a protease inhibitor (e.g., VX-950) and another anti-viral agent, preferably an anti-HCV agent. Combination therapy targeting HCV is also described in U.S. Patent Nos. 6,924,270; 6,849,254.

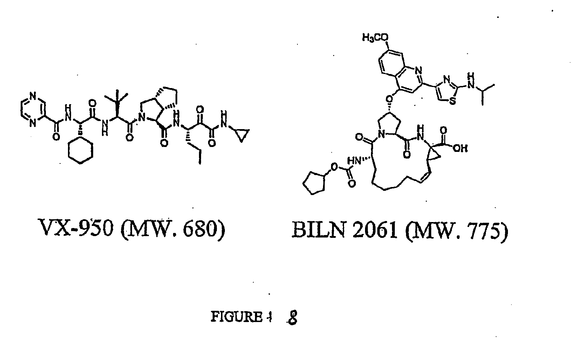

Another anti-viral agent may also be a protease inhibitor, particularly an HCV protease inhibitor. HCV protease inhibitors known in the art include VX-950 (Figure 8), BILN 2061 (Figure 8, see also PCT Publication No. WO 00/59929; U.S. Pat. No. 6,608,027), compound 1 (13), Inhibitors A, B, and C (PCT Publication No. WO 04/039970). Potential HCV protease inhibitors have also been described in PCT and U.S. patent application publication Nos. WO 97/43310, US 20020016294, WO 01/81325, WO 02/08198, WO 01/77113, WO 02/08187, WO 02/08256, WO 02/08244, WO 03/006490, WO O1/74768, WO 99/50230, WO 98/17679, WO 02/48157, US 20020177725, WO 02/060926, US 20030008828, WO 02/48116, WO 01/64678, WO 01/07407, WO 98/46630, WO 00/59929, WO 99/07733, WO 00/09588, US 20020016442, WO 00/09543, WO 99/07734, US 20020032175, US 20050080017, WO 98/22496, WO 02/079234, WO 00/31129, WO 99/38888, WO 99/64442, WO 2004072243, and WO 02/18369, and U.S. Patent Nos. 6,018,020; 6,265,380; 6,608,027; 5,866,684; M. Llinas-Brunet et al., Bioorg. Med. Chem. Lett., 8, pp. 1713-18 (1998); W. Han et al., Bioorg. Med. Chem. Lett., 10,711-13 (2000); R. Dunsdon et al., Bioorg. Med. Chem. Lett., 10, pp. 1571-79 (2000); M. Llinas-Brunet et al., Bioorg. Med. Chem. Lett., 10, pp. 2267-70 (2000); and S. LaPlante et al., Bioorg. Med. Chem. Lett., 10, pp. 2271-74 (2000). A number of NS3 protease inhibitors have also been developed by Schering Corp., Schering A.G., and other companies, and they are described in U.S. patent application publication Nos. 20050249702; 20050153900; 20050245458; 20050222047; 20050209164; 20050197301; 20050176648; 20050164921; 20050119168; 20050085425; 20050059606; 20030207861; 20020147139; 20050143439; 20050059606; 20050107304; 20050090450; 20040147483; 20040142876; 20040077600; 20040018986; 20030236242; 20030216325; 20030207861; U.S. Patent Nos. 6,962,932; 6,914,122; 6,911,428; 6,846,802; 6,838,475.

Anti-viral agents may also include, but are not limited to, immunomodulatory agents, such as alpha-, beta-, and gamma-interferons, pegylated derivatized interferon-alpha compounds, and thymosin; other anti-viral agents, such as ribavirin, amantadine, and telbivudine; other inhibitors of hepatitis C proteases (NS2-NS3 inhibitors and NS3-NS4A inhibitors); inhibitors of other targets in the HCV life cycle, including helicase and polymerase inhibitors; inhibitors of internal ribosome entry; broad-spectrum viral inhibitors, such as IMPDH inhibitors (e.g., compounds of U.S. Pat. Nos. 5,807,876, 6,498,178, 6,344,465, 6,054,472, WO 97/40028, WO 98/40381, WO 00/56331, and mycophenolic acid and derivatives thereof, and including, but not limited to VX-497, VX-148, and/or VX-944); or combinations of any of the above. See also W. Markland et al., Antimicrobial & Antiviral Chemotherapy, 44, p. 859 (2000) and U.S. Pat. No. 6,541,496.

The following definitions are used herein:

"Peg-Intron" means PEG-Intron®, peginteferon alfa-2b, available from Schering Corporation, Kenilworth, N.J.; "Intron" means Intron-A®, interferon alpha-2b available from Schering Corporation, Kenilworth, N.J.; "ribavirin" means ribavirin (1-beta-D-ribofuranosyl-1H-- 1,2,4-triazole-3-carboxamide, available from ICN Pharmaceuticals, Inc., Costa Mesa, Calif.; described in the Merck Index, entry 8365, Twelfth Edition; also available as Rebetol® from Schering Corporation, Kenilworth, N.J., or as Copegus® from Hoffmann-La Roche, Nutley, N.J.; "Pagasys" means Pegasys®, peg-interferon alfa-2a available Hoffmann-La Roche, Nutley, N.J.; "Roferon" means Roferon®, recombinant interferon alpha-2a available from Hoffmann-La Roche, Nutley, N.J.; "Berofor" means Berofor®, interferon alpha-2 available from Boehringer Ingelheim Pharmaceutical, Inc., Ridgefield, Conn.; Sumiferon®, a purified blend of natural alpha interferons such as Sumiferon available from Sumitomo, Japan; Wellferon®, interferon alpha n1 available from Glaxo Wellcome Ltd., Great Britain; Alferon®, a mixture of natural alpha interferons made by Interferon Sciences, and available from Purdue Frederick Co., CT.

The term "interferon" as used herein means a member of a family of highly homologous species-specific proteins that inhibit viral replication and cellular proliferation, and modulate immune response, such as interferon alpha, interferon beta, or interferon gamma. The Merck Index, entry 5015, Twelfth Edition. As described herein, the interferon may be alpha-interferon. A therapeutic combination described herein may utilize natural alpha interferon 2a. Alternatively, the therapeutic combination described herein utilizes natural alpha interferon 2b. The therapeutic combination may utilize recombinant alpha interferon 2a or 2b. The interferon may be pegylated alpha interferon 2a or 2b. Suitable interferons include: (a) Intron (interferon-alpha 2B, Schering Plough), (b) Peg-Intron, (c) Pegasys, (d) Roferon, (e) Berofor, (f) Sumiferon, (g) Wellferon, (h) consensus alpha interferon available from Amgen, Inc., Newbury Park, Calif., (i) Alferon; (j) Viraferon®; (k) Infergen®.

A protease inhibitor can be administered orally, whereas Interferon is not typically administered orally. Nevertheless, nothing herein limits the methods or combinations as described to any specific dosage forms or regime. Thus, each component of a combination described herein may be administered separately, together, sequentially or simultaneously, or in any combination thereof.

The protease inhibitor and interferon may be administered in separate dosage forms. Any additional agent may be administered as part of a single dosage form with the protease inhibitor or as a separate dosage form. As this disclosure involves a combination of compounds and/or agents, the specific amounts of each compound or agent may be dependent on the specific amounts of each other compound in the combination. Dosages of interferon are typically measured in IU (e.g., about 4 million IU to about 12 million IU).

Accordingly, agents (whether acting as an immunomodulatory agent or otherwise) that may be used in combination with a compound described herein include, but are not limited to, interferon-alpha 2B (Intron A, Schering Plough); Rebatron (Schering Plough, Inteferon-alpha 2B+Ribavirin); pegylated interferon alpha (Reddy, K. R. et al. "Effcacy and Safety of Pegylated (40-kd) interferon alpha-2a compared with interferon alpha-2a in noncirrhotic patients with chronic hepatitis C," Hepatology, 33, pp. 433-438 (2001)); consensus interferon (Kao, J. H., et al., "efficacy of Consensus Interferon in the Treatment of Chronic Hepatitis," J. Gastroenterol. Hepatol. 15, pp. 1418-1423 (2000)); interferon-alpha 2A (Roferon A; Roche), lymphoblastoid or "natural" interferon; interferon tau (Clayette, P. et al., "IFN-tau, A New Interferon Type I with Antiretroviral activity," Pathol. Biol. (Paris) 47, pp. 553-559 (1999)); interleukin 2 (Davis, G.L. et al., "Future Options for the Management of Hepatitis C," Seminars in Liver Disease, 19, pp. 103-112 (1999)); Interleukin 6 (Davis, G.L. et al., *supra*); interleukin 12 (Davis, G.L. et al., *supra*); Ribavirin; and compounds that enhance the development of type 1 helper T cell response (Davis, G.L., et al., *supra*). Interferons may ameliorate viral infections by exerting direct antiviral effects and/or by modifying the immune response to infection. The antiviral effects of interferons are often mediated through inhibition of viral penetration or uncoating, synthesis of viral RNA, translation of viral proteins, and/or viral assembly and release.

Compounds that stimulate the synthesis of interferon in cells (Tazulakhova, E. B. et al., "Russian Experience in Screening, analysis, and Clinical Application of Novel Interferon Inducers," J. Interferon Cytokine Res., 21 pp. 65-73) include, but are not limited to, double stranded RNA, alone or in combination with tobramycin, and Imiquimod (3M Pharmaceuticals; Sauder, D. N., "Immunomodulatory and Pharmacologic Properties of Imiquimod," J. Am. Acad. Dermatol., 43 pp. S6-11 1 (2000)).

Other non-immunomodulatory or immunomodulatory compounds may be used in combination with a compound as described including, but not limited to, those specified in WO 02/18369 (see, e.g., page 273, lines 9-22 and page 274, line 4 to page 276, line 11).

Compounds that stimulate the synthesis of interferon in cells (Tazulakhova et al., J. Interferon Cytokine Res. 21, 65-73), include, but are not limited to, double stranded RNA, alone or in combination with tobramycin and Imiquimod (3M Pharmaceuticals) (Sauder, J. Am. Arad. Dermatol. 43, S6-11 (2000)).

Other compounds known to have, or that may have, HCV antiviral activity include, but are not limited to, Ribavirin (ICN Pharmaceuticals); inosine 5'-monophosphate dehydrogenase inhibitors (VX-497 formula provided herein); amantadine and rimantadine (Younossi et al., In Seminars in Liver Disease 19, 95-102 (1999)); LY217896 (U.S. Pat. No. 4,835,168) (Colacino, et al., Antimicrobial Agents & Chemotherapy 34, 2156-2163 (1990)); and 9-Hydroxyimino-6-methoxy-1,4a-dimethyl 1,2,3,4,4a,9,10,10a-octahydro-phena- nthrene-1-carboxylic acid methyl ester; 6-Methoxy-1,4a dimethyl-9-(4-methyl-piperazin-1-ylimino)-1,2,3,4,4a,9,10,10a-octahydro-p-henanthrene-1 carboxylic acid methyl ester-hydrochloride; 1-(2-Chloro-phenyl)-3-(2,2-Biphenyl-ethyl)-urea (U.S. Pat. No. 6,127,422).

Formulations, doses, and routes of administration for the foregoing molecules are either taught in the references cited below, or are well-known in the art as disclosed, for example, in F. G. Hayden, in Goodman & Gilman's The Pharmacological Basis of Therapeutics, Ninth Edition, Hardman et al., Eds., McGraw-Hill, New York (1996), Chapter 50, pp. 1191-1223, and the references cited therein. Alternatively, once a compound that exhibits HCV antiviral activity, particularly antiviral activity against a drug-resistant strain of HCV, has been identified, a pharmaceutically effective amount of that compound can be determined using-techniques that are well-known to the skilled artisan. Note, for example, Benet et al., in Goodman & Gilman's The Pharmacological Basis of Therapeutics, Ninth Edition, Hardman et al., Eds., McGraw-Hill, New York (1996), Chapter 1, pp. 3-27, and the references cited therein. Thus, the appropriate formulations, dose(s) range, and dosing regimens, of such a compound can be easily determined by routine methods.

The compositions related to combination therapies described herein can be provided to a cell or cells, or to a human patient, either in separate pharmaceutically acceptable formulations administered simultaneously or sequentially, formulations containing more than one therapeutic agent, or by an assortment of single agent and multiple agent formulations. Regardless of the route of administration, these drug combinations form an anti-HCV effective amount of components of the pharmaceutically acceptable formulations.

A large number of other immunomodulators and immunostimulants that can be used in the compounds described herein are currently available and include: AA-2G; adamantylamide dipeptide; adenosine deaminase, Enzon adjuvant, Alliance; adjuvants, Ribi; adjuvants, Vaxcel; Adjuvax; agelasphin-11; AIDS therapy, Chiron; algal glucan, SRI; alganunulin, Anutech; Anginlyc; anticellular factors, Yeda; Anticort; antigastrin-17 immunogen, Ap; antigen delivery system, Vac; antigen formulation, IDBC; antiGnRH immunogen, Aphton; Antiherpin; Arbidol; azarole; Bay-q-8939; Bay-r-1005; BCH-1393; Betafectin; Biostim; BL-001; BL-009; Broncostat; Cantastim; CDRI-84-246; cefodizime; chemokine inhibitors, ICOS; CMV peptides, City of Hope; CN-5888; cytokine-releasing agent, St; DHEAS, Paradigm; DISC TA-HSV; J07B; I01A; I01Z; ditiocarb sodium; ECA-10-142; ELS-1; endotoxin, Novartis; FCE-20696; FCE-24089; FCE-24578; FLT-3 ligand, Immunex; FR-900483; FR-900494; FR-901235; FTS-Zn; G-proteins, Cadus; gludapcin; glutaurine; glycophosphopeptical; GM-2; GM-53; GMDP; growth factor vaccine, EntreM; H-BIG, NABI; H-CIG, NABI; HAB-439; Helicobacter pylori vaccine; herpes-specific immune factor; HIV therapy, United Biomed; HyperGAM+CF; ImmuMax; Immun BCG; immune therapy, Connective; immunomodulator, Evans; immunomodulators, Novacell; imreg-1; imreg-2; Indomune; inosine pranobex; interferon, Dong-A (alpha2); interferon, Genentech (gamma); interferon, Novartis (alpha); interleukin-12, Genetics Ins; interleukin-15, Immunex; interleukin-16, Research Cor; ISCAR-1; J005X; L-644257; licomarasminic acid; LipoTher; LK-409, LK-410; LP-2307; LT (R1926); LW-50020; MAF, Shionogi; MDP derivatives, Merck; metenkephalin, TNI; methylfurylbutyrolactones; MIMP; mirimostim; mixed bacterial vaccine, Tem, MM-1; moniliastat; MPLA, Ribi; MS-705; murabutide; marabutide, Vacsyn; muramyl dipeptide derivative; muramyl peptide derivatives myelopid; -563; NACOS-6; NH-765; NISV, Proteus; NPT-16416; NT-002; PA-485; PEFA-814; peptides, Scios; peptidoglycan, Pliva; Perthon, Advanced Plant; PGM derivative, Pliva; Pharmaprojects No. 1099; No. 1426; No. 1549; No. 1585; No. 1607; No. 1710; No. 1779; No. 2002; No. 2060; No. 2795; No. 3088; No. 3111; No. 3345; No. 3467; No. 3668; No. 3998; No. 3999; No. 4089; No. 4188; No. 4451; No. 4500; No. 4689; No. 4833; No. 494; No. 5217; No. 530; pidotimod; pimelautide; pinafide; PMD-589; podophyllotoxin, Conpharn; POL-509; poly-ICLC; poly-ICLC, Yamasa Shoyu; PolyA-PolyU; Polysaccharide A; protein A, Berlux Bioscience; PS34WO; Pseudomonas MAbs, Teijin; Psomaglobin; PTL-78419; Pyrexol; pyriferone; Retrogen; Retropep; RG-003; Rhinostat; rifamaxil; RM-06; Rollin; romurtide; RU-40555; RU-41821; Rubella antibodies, ResCo; S-27649; SB-73; SDZ-280-636; SDZ-MRL953; SK&F-107647; SL04; SL05; SM-4333; Solutein; SRI-62-834; SRL-172; ST-570; ST-789; staphage lysate; Stimulon; suppressin; T-150R1; T-LCEF; tabilautide; temurtide; Theradigm-HBV; Theradigm-HBV; Theradigm-HSV; THF, Pharm & Upjohn; THF, Yeda; thymalfasin; thymic hormone fractions; thymocartin; thymolymphotropin; thymopentin; thymopentin analogues; thymopentin, Peptech; thymosin fraction 5, Alpha; thymostimulin; thymotrinan; TMD-232; TO-115; transfer factor, Viragen; tuftsin, Selavo; ubenimex; Ulsastat; ANGG-; CD-4+; Collag+; COLSF+; COM+; DA-A+; GAST-; GF-TH+; GP-120-; IF+; IF-A+; IF-A-2+; IF-B+; IF-G+; IF-G-1B+; IL-2+; IL-12+; IL-15+; IM+; LHRH-; LIPCOR+L LYM-B+; LYM-NK+; LYM-T+; OPI+; PEP+; PHG-MA+; RNA-SYN-; SY-CW-; TH-A-I+; TH-5+; TNF+; UN.

Representative nucleoside and nucleotide compounds useful in the disclosure include, but are not limited to: (+)-cis-5-fluoro-1-[2-(hydroxymethyl)-[1,3-oxathiolan-5yl]cytosine; (-)-2'-deoxy-3'-thiocytidine-5'-triphospbate (3TC); (-)-cis-5-fluoro-1-[2(hydroxy-methyl)-[1,3-oxathiolan-5-yl]cytosine (FTC); (-)2', 3', dideoxy-3'-thiacytidine[(-)-SddC]; 1-(2'-deoxy-2'-fluoro-beta-D-arabinofinanosyl)-5-iodocytosine (FIAC); 1-(2'-deoxy-2'-fluoro-beta-D-arabinofuranosyl)-5-iodocytosine triphosphate (FIACTP); 1-(2'-deoxy-2'-fluoro-beta-D-arabinofuranosyl)-5-m- ethyluracil (FMAU); 1-beta-D-ribofuranosyl-1,2,4-triazole-3-carboxamide; 2',3'-dideoxy-3'-fluoro-5-methyl-dexocytidine (FddMeCyt); 2',3'-dideoxy-3'-chloro-5-methyl-dexocytidine (ClddMeCyt); 2',3'-dideoxy-3'-amino-S-methyl-dexocytidine (AddMeCyt); 2',3'-dideoxy-3'-fluoro-5-methyl-cytidine (FddMeCyt); 2',3'-dideoxy-3'-chloro-5-methyl-cytidine (ClddMeCyt); 2',3'-dideoxy-3'-amino-5-methyl-cytidine (AddMeCyt); 2',3'-dideoxy-3'-fluorothymidine (FddThd); 2',3'-dideoxy-beta-L-5-fluoroc- ytidine (beta-L-FddC) 2',3'-dideoxy-beta-L-5-thiacytidine; 2',3'-dideoxy-beta-L-5-cytidine (beta-L-ddC); 9-(1,3-dihydroxy-2-propoxymethyl) guanine; 2'-deoxy-3'-thia-5-fluorocytosine; 3'-amino-5-methyl-dexoc- ytidine (AddMeCyt); 2-amino-1,9-[(2-hydroxymethyl-1-(hydroxymethyl) ethoxy]methyl]-6H-purin-6-one (gancyclovir); 2-[2-(2-amino-9H-purin-9y)et- hyl]-1,3-propandil diacetate(famciclovir); 2-amino-1,9-dihydro-9-[(2-hydro- xy-ethoxy) methyl]6H-purin-6-one (acyclovir); 9-(4-hydroxy-3-hydroxymethyl- -but-1-yl) guanine (penciclovir); 9-(4-hydroxy-3-hydroxymethyl-but-1-yl)-6- -deoxy-guanine diacetate(famciclovir); 3'-azido-3'-deoxythymidine (AZT); 3'-chloro-5-methyl-dexocytidine (ClddMeCyt); 9-(2-phosphonyl-methoxyethyl-)-2',6-diaminopurine-2', 3'-dideoxyriboside; 9-(2-phosphonylmethoxyethyl)- adenine (PMEA); acyclovir triphosphate (ACVTP); D-carbocyclic-2'-deoxyguan- osine (CdG); dideoxy-cytidine; dideoxy-cytosine (ddC); dideoxy-guanine (ddG); dideoxy-inosine (ddI); E-5-(2-bromovinyl)-2'-deoxyuridine triphosphate; fluoro-arabinofuranosyl-iodouracil; 1-(2'-deoxy-2'-fluoro-1- -beta-D-arabinofuranosyl)-5-iodo-uracil (FIAU); stavudine; 9-beta-D-arabinofuranosyl-9H-purine-6-amine monohydrate (Ara-A); 9-beta-D-arabinofuranosyl-9H-purine-6-amine-5'-monophosphate monohydrate (Ara-AMP); 2-deoxy-3'-thia-5-fluorocytidine; 2',3'-dideoxy-guanine; and 2',3'-dideoxy-guanosine.

Synthetic methods for the preparation of nucleosides and nucleotides useful in the present disclosure are well known in the art as disclosed in Acta Biochim Pol., 43, 25-36 (1996); Swed. Nucleosides Nucleotides 15, 361-378 (1996); Synthesis 12, 1465-1479 (1995); Carbohyd. Chem. 27, 242-276 (1995); Chena Nucleosides Nucleotides 3, 421-535 (1994); Ann. Reports in Med. Chena, Academic Press; and Exp. Opin. Invest. Drugs 4, 95-115 (1995).

The chemical reactions described in the references cited above are generally disclosed in terms of their broadest application to the preparation of the compounds described herein. Occasionally, the reactions may not be applicable as described to each compound included within the scope of compounds disclosed herein. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional will be applicable to the preparation of the corresponding compounds described herein. In all preparative methods, all starting materials are known or readily preparable from known starting materials.

While nucleoside analogs are generally employed as anti-viral agents as is, nucleotides (nucleoside phosphates) sometimes have to be converted to nucleosides in order to facilitate their transport across cell membranes. An example of a chemically modified nucleotide capable of entering cells is S-1-3-hydroxy-2-phosphonylmethoxypropyl cytosine (HPMPC, Gilead Sciences). Nucleoside and nucleotide compounds used herein that are acids can form salts. Examples include salts with alkali metals or alkaline earth metals, such as sodium, potassium, calcium, or magnesium, or with organic bases or basic quaternary ammonium salts.

The administration of a cytochrome P450 monooxygenase inhibitor may also be chosen. Such inhibitors may be useful in increasing liver concentrations and/or increasing blood levels of compounds that are inhibited by cytochrome P450. For a use described herein that involves a CYP inhibitor, any CYP inhibitor that improves the pharmacokinetics of the relevant NS3/4A protease may be included in a composition and/or used in a method of treatment described herein. These CYP inhibitors include, but are not limited to, ritonavir (WO 94/14436), ketoconazole, troleandomycin, 4-methylpyrazole, cyclosporin, clomethiazole, cimetidine, itraconazole, fluconazole, miconazole, fluvoxamine, fluoxetine, nefazodone, sertraline, indinavir, nelfinavir, amprenavir, fosamprenavir, saquinavir, lopinavir, delavirdine, erythromycin, VX-944, and VX-497. Preferred CYP inhibitors include ritonavir, ketoconazole, troleandomycin, 4-methylpyrazole, cyclosporin, and clomethiazole. For preferred dosage forms of ritonavir, see U.S. Pat. No. 6,037,157, and the documents cited therein: U.S. Pat. No. 5,484,801, U.S. application Ser. No. 08/402,690, and International Applications WO 95/07696 and WO 95/09614).

Methods for measuring the ability of a compound to inhibit cytochrome P50 monooxygenase activity are known (see U.S. Pat. No. 6,037,157 and Yun, et al. Drug Metabolism & Disposition, vol. 21, pp. 403-407 (1993)).

Immunomodulators, immunostimulants and other agents useful in the combination therapy methods described herein can be administered in amounts lower than those conventional in the art. For example, interferon alpha is typically be administered to humans for the treatment of HCV infections in an amount of from about $1 \times 10^6$ units/person three times per week to about $10 \times 10^6$ units/person three times per week (Simon et al., Hepatology 25: 445-448 (1997)). In the methods and compositions described herein, this dose can be in the range of from about $0.1 \times 10^6$ units/person three times per week to about $7.5 \times 10^6$ units/person three times per week; more preferably from about $0.5 \times 10^6$ units/person three times per week to about $5 \times 10^6$ units/person three times per week; most preferably from about $1 \times 10^6$ units/person three times per week to about $3 \times 10^6$ units/person three times per week Due to the enhanced hepatitis C virus antiviral effectiveness of immunomodulators, immunostimulants or other anti-HCV agent in the presence of the HCV serine protease inhibitors described herein, reduced amounts of these immunomodulators/immunostimulants can be employed in the treatment methods and compositions contemplated herein. Similarly, due to the enhanced hepatitis C virus antiviral effectiveness of the present HCV serine protease inhibitors in the presence of immunomodulators and immunostimulants, reduced amounts of these HCV serine protease inhibitors can be employed in the methods and compositions contemplated herein. Such reduced amounts can be determined by routine monitoring of hepatitis C virus titers in infected patients undergoing therapy. This can be carried out by, for example, monitoring HCV RNA in patients' serum by slot-blot, dot-blot, or RT-PCR techniques, or by measurement of HCV surface or other antigens. Patients can be similarly monitored during combination therapy employing the HCV serine protease inhibitors disclosed herein and other compounds having anti-HCV activity, for example nucleoside and/or nucleotide anti-viral agents, to determine the lowest effective doses of each when used in combination.

In the methods of combination therapy disclosed herein, nucleoside or nucleotide antiviral compounds, or mixtures thereof, can be administered to humans in an amount in the range of from about 0.1 mg/person/day to about 500 mg/person/day; preferably from about 10 mg/person/day to about 300 mg/person/day; more preferably from about 25 mg/person/day to about 200 mg/person/day; even more preferably from about 50 mg/person/day to about 150 mg/person/day; and most preferably in the range of from about 1 mg/person/day to about 50 mg/person/day.

Doses of compounds can be administered to a patient in a single dose or in proportionate doses. In the latter case, dosage unit compositions can contain such amounts of submultiples thereof to make up the daily dose. Multiple doses per day can also increase the total daily dose should this be desired by the person prescribing the drug.

The regimen for treating a patient suffering from a HCV infection with the compounds and/or compositions described herein is selected in accordance with a variety of factors, including the age, weight, sex, diet, and medical condition of the patient, the severity of the infection, the route of administration, pharmacological considerations such as the activity, efficacy, pharmacokinetic, and toxicology profiles of the particular compounds employed, and whether a drug delivery system is utilized. Administration of the drug combinations disclosed herein should generally be continued over a period of several weeks to several months or years until virus titers reach acceptable levels, indicating that infection has been controlled or eradicated. Patients undergoing treatment with the drug combinations disclosed herein can be routinely monitored by measuring hepatitis viral RNA in patients' serum by slot-blot, dot-blot, or RT-PCR techniques, or by measurement of hepatitis C viral antigens, such as surface antigens, in serum to determine the effectiveness of therapy. Continuous analysis of the data obtained by these methods permits modification of the treatment regimen during therapy so that optimal amounts of each component in the combination are administered, and so that the duration of treatment can be determined as well. Thus, the treatment regimen/dosing schedule can be rationally modified over the course of therapy so that the lowest amounts of each of the antiviral compounds used in combination which together exhibit satisfactory anti-hepatitis C virus effectiveness are administered, and so that administration of such antiviral compounds in combination is continued only so long as is necessary to successfully treat the infection.

The disclosure encompasses the use of the HCV serine protease inhibitors disclosed herein in various combinations with the foregoing and similar types of compounds having anti-HCV activity to treat or prevent HCV infections in patients, particularly those patients that have HCV infections that have developed resistance to treatment by VX-950 and other standard protease inhibitors. For example, one or more HCV serine protease inhibitors can be used in combination with: one or more interferons or interferon derivatives having anti-HCV activity; one or more non-interferon compounds having anti-HCV activity; or one or more interferons or interferon derivatives having anti-HCV activity and one or more non-interferon compounds having anti-HCV activity. When used in combination to treat or prevent HCV infection in a human patient, any of the presently disclosed HCV serine protease inhibitors and foregoing compounds having anti-HCV activity can be present in a pharmaceutically or anti-HCV effective amount. By virtue of their additive or synergistic effects, when used in the combinations described above, each can also be present in a subclinical pharmaceutically effective or anti-HCV effective amount, i.e., an amount that, if used alone, provides reduced pharmaceutical effectiveness in completely inhibiting or reducing the accumulation of HCV virions and/or reducing or ameliorating conditions or symptoms associated with HCV infection or pathogenesis in patients compared to such HCV serine protease inhibitors and compounds having anti-HCV activity when used in pharmaceutically effective amounts. In addition, the disclosure encompasses the use of combinations of HCV serine protease inhibitors and compounds having anti-HCV activity as described above to treat or prevent HCV infections, where one or more of these inhibitors or compounds is present in a pharmaceutically effective amount, and the other(s) is(are) present in a subclinical pharmaceutically-effective or anti-HCV effective amount(s) owing to their additive or synergistic effects. As used herein, the term "additive effect" describes the combined effect of two (or more) pharmaceutically active agents that is equal to the sum of the effect of each agent given alone. A "synergistic effect" is one in which the combined effect of two (or more) pharmaceutically active agents is greater than the sum of the effect of each agent given alone.

Upon improvement of a patient's condition, a maintenance dose of a compound, composition or combination as described may be administered, if necessary. Subsequently, the dosage or frequency of administration, or both, may be reduced, as a function of the symptoms, to a level at which the improved condition is retained when the symptoms have been alleviated to the desired level, treatment should cease. Patients may, however, require intermittent treatment on a long-term basis upon any recurrence of disease symptoms.

A specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredients will also depend upon the particular described compound and the presence or absence and the nature of the additional anti-viral agent in the composition.

Accordingly, the agents described herein useful for therapeutic treatment can be administered alone, in a composition with a suitable pharmaceutical carrier, or in combination with other therapeutic agents. An effective amount of the agents to be administered can be determined on a case-by-case basis. Factors to be considered usually include age, body weight, stage of the condition, other disease conditions, duration of the treatment, and the response to the initial treatment. Typically, the agents are prepared as an injectable, either as a liquid solution or suspension. However, solid forms suitable for solution in, or suspension in, liquid vehicles prior to injection can also be prepared. The agent can also be formulated into an enteric-coated tablet or gel capsule according to known methods in the art. The agents described herein may be administered in any way which is medically acceptable which may depend on the identity of the agent and/or on the disease condition or injury being treated. Possible administration routes include injections, by parenteral routes such as intravascular, intravenous, intraepidural or others, as well as oral, nasal, ophthalmic, rectal, topical, or pulmonary, e.g., by inhalation, aerosolization or nebulization. The agents may also be directly applied to tissue surfaces, e.g., during surgery. Sustained release administration is also specifically included in the application, by such means as depot injections, transdermal patches, or erodible implants.

The disclosure also describes a pharmaceutically acceptable composition described herein for use in a method for treating a patient infected with or preventing infection by a virus characterized by a virally encoded serine protease that is necessary for the life cycle of the virus. Preferably, the methods for treating as described are used to treat a patient suffering from a HCV infection. Such treatment may completely eradicate the viral infection or reduce the severity thereof. More preferably, the patient is a human being.

The term "treating" includes prophylactic (e.g., preventing) and/or therapeutic treatments. The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic, (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

The methods described herein additionally comprise the step of administering to said patient an anti-viral agent preferably an anti-HCV agent. Such anti-viral agents include, but are not limited to, immunomodulatory agents, such as alpha-, beta-, and gamma-interferons, pegylated derivatized interferon-alpha compounds, and thymosin; other anti-viral agents, such as ribavirin and amantadine; other inhibitors of hepatitis C proteases (NS2-NS3 inhibitors and NS3-NS4A inhibitors); inhibitors of other targets in the HCV life cycle, including helicase and polymerase inhibitors; inhibitors of internal ribosome entry; broad-spectrum viral inhibitors, such as IMPDH inhibitors (e.g., VX-497 and other IMPDH inhibitors disclosed in U.S. Pat. No. 5,807,876, mycophenolic acid and derivatives thereof); or combinations of any of the above.

Such additional agent may be administered to said patient as part of a single dosage form comprising both a compound described herein and an additional anti-viral agent. Alternatively the additional agent may be administered separately from the compound described herein, as part of a multiple dosage form, wherein said additional agent is administered prior to, together with or following a composition comprising a compound described herein.

The disclosure also relates to a pharmaceutically acceptable composition comprising a compound described herein for use in a method of pre-treating a biological substance intended for administration to a patient comprising the step of contacting said biological substance with said pharmaceutically acceptable composition. Such biological substances include, but are not limited to, blood and components thereof such as plasma, platelets, subpopulations of blood cells and the like; organs such as kidney, liver, heart, lung, etc; sperm and ova; bone marrow and components thereof, and other fluids to be infused into a patient such as saline, dextrose, etc.

The disclosure also relates to a compound as described herein for use in methods of treating materials that may potentially come into contact with a virus characterized by a virally encoded serine protease necessary for its life cycle. Such materials include, but are not limited to, surgical instruments and garments; laboratory instruments and garments; blood collection apparatuses and materials; and invasive devices, such as shunts, stents, etc.

The compounds described herein may be used as laboratory tools to aid in the isolation of a virally encoded serine protease. This method comprises the steps of providing a compound described herein attached to a solid support; contacting said solid support with a sample containing a viral serine protease under conditions that cause said protease to bind to said solid support; and eluting said serine protease from said solid support. Preferably, the viral serine protease isolated by this method is HCV NS3 protease. More particularly, it is a mutant HCV NS3 protease that is resistant to treatment by VX-905 and/or BILN 2061 as described herein. Exemplary such proteases includes those described herein as having mutant (i.e., non-wild-type) residue: at position 36 of a protein of a wild-type HCV NS3 protein (685 amino acids).

Exemplification

The disclosure now being generally described, it will be more readily understood by reference to the following examples. Examples not relating to the V36M or V36G mutation are for illustration only.

*Example 1 Patient Population and Study Design*

Thirty four patients infected with genotype 1 HCV who were enrolled in a phase 1b randomized, blinded, dose-escalation clinical trial for VX-950 (Study VX04-950-101) were subjects of the study. All patients were between 18 and 65 years of age, had baseline HCV RNA levels of at least $10^5$ IU/mL, and were hepatitis B virus (HBV) and HIV negative. Patients were divided into 3 groups receiving 450 (q8h), 750 (q8h), or 1250 (q12h) mg VX-950 for 14 consecutive days, with 2 placebo patients in each dosing group. Four milliliter (mL) blood samples were collected from study patients at 3 time points: the day before dosing (baseline samples), at day 14 of dosing or end of treatment (ETR sample), and 7 to 10 days after the last dose of study drug (follow-up sample). Blood was collected by venipuncture of a forearm vein into tubes containing EDTA ($K_2$) anticoagulant. Plasma was separated by 10 min centrifugation, frozen, and stored at -80 °C for less than 6 months. Virions were isolated from this plasma for sequence analysis.

*Example 2 Amplification and Sequencing of the HCV NS3 Protease from Patient Plasma*

Sequence analysis of HCV was done by semi-nested reverse-transcriptase polymerase chain reaction (RT-PCR) amplification of a HCV RNA fragment containing the full 534 base pair (bp) NS3 serine protease region from plasma virus. The virions were lysed under denaturing conditions, and the HCV RNA was isolated using a standard commercial silica-gel membrane-binding method (QIAamp Viral RNA Minikit; Qiagen, Valencia, CA). A complementary DNA (cDNA) fragment containing the NS3 serine protease region was synthesized from viral RNA and amplified using a commercial 1-step reverse transcriptase PCR (Superscript III RNase H-Reverse Transcriptase with High Fidelity Platinum Taq DNA Polymerase; Invitrogen Corp, Carlsbad, CA). A 912 bp coding region of NS3 was amplified using primers flanking the NS3 region (NS3-1b-1s: GGCGTGTGGGGACATCATC; and NS3-1b-3a: GGTGGAGTACGTGATGGGGC. Two rounds of nest PCR were performed for each sample at a final concentration of 0.5 $\mu$M primer (Invitrogen Custom Primers), 0.2 mM dNTPs (Invitrogen Corp), 1.2 mM MgSO$_4$, and 34.8 units of RNA guard (Porcine RNase Inhibitor, Amersham Biosciences) in 1x proprietary reaction buffer. Reaction mixtures were initially incubated for a 30 min reverse transcription reaction at 47 °C followed by a 3 min denaturation step at 94 °C and then 30 cycles of 94 °C for 30 sec, 51 °C for 30 sec, and 68 °C for 45 sec. The first PCR product was diluted 1:10 and used in another semi-nested reaction using 1.25 units of AccuPrime *Pfx* DNA polymerase, 0.5 $\mu$M primer (NS3-1b-1s and NS3-1b-4a; CATATACGCTCCAAAGCCCA, 0.3 mM dNTPs, 1 mM MgSO$_4$, and 1x reaction buffer. The DNA products from the outer PCR were denatured for 3 min at 94 °C and amplified with 30 cycles of 94 °C for 30s, 53 °C for 30s, and 68 °C for 30s. The DNA from this PCR was then separated on a 1% agarose gel, and the appropriately sized product (830 bp) was purified using the QIAquick Gel Extraction Kit (Qiagen). Isolated DNA was then cloned using the Zero Blunt TOPO PCR Cloning Kit (Invitrogen Corp). Cloning plates were sent to SeqWright (Houston, TX) where 96 clones were amplified and sequenced per patient per time point.

*Example 3 Sequence Alignment and Phylogenetic Analysis*

Sequences were aligned and analyzed for mutations using the software Mutational Surveyor (SoftGenetics, State College, PA). The N-terminal 543 nucleotides (181 amino acids) of NS3 protease were analyzed. A consensus sequence for each patient was developed from an average of 84 baseline sequences, and an average of 81 sequences were obtained for each patient at Day 14 and at follow-up (7 to 10 days after the last dose of study drug). Phylogenetic trees were made using PHYLIP (Felsenstein, J. 1993. PHYLIP (Phylogeny Inference Package) version 3.5c. Distributed by the author. Department of Genetics, University of Washington, Seattle, WA) Dnadist and Quick Tree (http: //www.hcv.lanl.gov/content/hcv-db, accessed June 2005).

*Example 4 Expression and Purification of Recombinant NS3 Protease Proteins*

A DNA fragment encoding Met[1]-Ser[181] of the HCV NS3 protease was amplified from selected plasmid clones of patient isolates using oligonucleotides specific for each HCV variant. The DNA fragment was cloned into the *Escherichia coli* expression plasmid pBEV11 leading to a 181-residue HCV NS3 protease domain followed by a C-terminal hexa-histidine tag. Recombinant 6X-His-tagged NS3 proteases were then expressed in *E. coli* using a leaky expression method as previously published (4). Five to seven isolated colonies of *E. coli* BL21 (DE3) freshly transformed with the NS3 protease expression plasmids were used to inoculate a 5 mL LB medium with 100 $\mu$g/mL carbenicillin. These seed cultures were incubated at 37 °C with shaking (250 rpm) until reaching an OD$_{620}$ between 0.3 and 1, then used to inoculate 50 mL 4xTY broth (32 g/L tryptone, 20 g/L, yeast extract, 5 g/L NaCl) containing 100 $\mu$g/mL carbenicillin in 250 mL Erlenmeyer flasks at an initial OD$_{620}$ of ~0.010. The expression cultures were incubated for 24 hours at ambient temperature (~25 °C) with shaking at 250 rpm. The cells were harvested by centrifugation at 3000x *g* for 30 min, the pellets were rapidly frozen in an -80 °C ethanol bath and stored at -80 °C until the protease was purified.

Recombinant proteases were purified from *E. coli* using a modification of a published method (7). Frozen cell pellets were thawed and re-suspended in 6.8 mL of cold Buffer A (50 mM *N*-2-Hydroxyethyl piperizine-*N*'-ethanesulfonic acid [HEPES, pH 8.0]; 1 M NaCl; 10% [vol/vol] glycerol; 5 mM imidazole; 5 mM $\beta$-mercaptoethanol; 0.1% Octyl $\beta$-D-glucopyranoside [Sigma, Saint Louis, MO]; 2 $\mu$g/mL Leupeptin [Sigma, Saint Louis, MO]; 1 $\mu$g/mL E-64 [Sigma, Saint Louis, MO]; 2 $\mu$g/mL Pepstatin A [Sigma, Saint Louis, MO]). The cells were lysed by the addition of 0.8 mL 10X BugBuster reagent (Novogen/EMD Biosciences, Madison, WI) and 8 $\mu$L of 1000X Benzonuclease (Novogen/EMD Biosciences, Madison, WI) followed by gentle rocking at 4 °C for 30 min. Cell lysates were centrifuged at 16,000x *g* to remove insoluble material. Each supernatant was applied to a 0.25 mL bed volume of TALON metal affinity resin (BD Biosciences, Palo Alto, CA) equilibrated to Buffer A in disposable polypropylene columns (Biorad, Hercules, CA). The lysate/resin slurries were rocked at 4 °C for 30 min. The lysates were drained from the column and the resin washed with 3-5 mL volumes of Buffer A. Two aliquots (0.25 mL each) of Buffer B (50 mM HEPES [pH 8.0]; 1 M NaCl; 25% [vol/vol] glycerol; 300 mM imidazole; 5 mM β-mercaptoethanol; 0.1 % Octyl β-D-glucopyranoside; 2 μg/mL Leupeptin; 1 μg/mL E-64; 2 μg/mL Pepstatin A]) were used to elute protein bound to the column, the two fractions were pooled, divided into small aliquots and stored at -80 °C. The concentration of the eluted protein was determined using a Coomassie protein assay (Biorad, Hercules, CA) according to the manufacturers instructions with a bovine serum albumin standard. Purity of the protease was estimated using 1D Image Analysis Software (Kodak, Rochester, NY) from protein samples resolved on denaturing acrylamide gels (SDS-PAGE) stained with Biosafe Coomassie Blue (Biorad, Hercules, CA).

*Examples 5 Enzymatic Assay for the HCV NS3 Serine Protease Domain*

*In vitro* protease activity was assayed as published (7) in 96-well microtiter plates (Coming NBS 3990) with both VX-950 and BILN 2061 protease inhibitors. BILN 2061 is a HCV NS3·4A protease inhibitor discovered by Boehringher Ingelheim, Laval, Quebec, Canada. Briefly, protease was incubated with 5 μM co-factor KK4A at 25 °C for 10 min and at 30 °C for 10 min. Protease inhibitor (VX-950 or BILN 2061), serially diluted in DMSO, was added and incubated for an additional 15 min at 30 °C. The reaction was initiated by the addition of 5 μM RET-S 1 (Anaspec Inc. San Jose, CA), an internally quenched fluorogenic depsipeptide substrate, and incubated at 30 °C. Product release was monitored for 20 min (excitation at 360 nm and emission at 500 nm) in a Tecan SpectraFluorPlus plate reader. Data were fitted with a simple $IC_{50}$ equation: $Y = V_0/(1+(X/IC_{50}))$.

*Examples 6 $K_m$ Determination of HCV NS3 Serine. Protease Domain proteins*

Substrate kinetic parameters were determined with an internally quenched fluorogenic depsipeptide substrate, RET-S 1 (Ac-DED(EDANS)EEαAbuψ[COO]ASK (DABCYL)-NH2 (Taliani et al., (1996) Anal. Biochem. 240(1), 60-67). Protease was pre-incubated with 5 μM co-factor peptide KK4A (KKGSVVIVGRIVLSGK (Landro et al., (1997) Biochemistry 36(31), 9340-9348) in 50 mM HEPES (pH 7.8), 100 mM NaCl, 20% glycerol, 5 mM dithiothreitol at 25 °C for 10 min and at 30 °C for 10 min. The reaction was initiated by the addition of the RET-S1 substrate (Anaspec Incorporated, San Jose, CA) and incubated for 10 min at 30 °C. Total assay volume was 100 μL. The reaction was quenched by the addition of 25 μL 10% trifluoroacetic acid (TFA). Reaction products were separated on a reverse phase microbore high performance liquid chromatography column (Penomenex Jupiter 5 μ C18 300 A column, 150 x 2.0 mm), which was heated to 40°C. The flow rate was 0.2 ml/min, with H2O/0.1%TFA (solvent A) and acetonitrile/0.1% TFA (solvent B). A linear gradient was used as follows: 5% to 30% solvent B over 1 min, 30% to 40% solvent B over 15 min, then 40% to 100% solvent B over 1 min, 3 min isocratic, followed by 100% to 5% B in 1 min, and equilibration at 5% B for 10 min. The DABCYL-peptide product was detected at 500 nm and typically eluted around 17 min. $K_m$ was determined by fitting the data to the Michaelis-Menten equation with GraphPrism software.

*Example 7 Determination of Telaprevir (VX-950) $K_{i(app.lh)}$ of the HCV NS3 Serine Protease Domain Variants*

Sensitivity of the NS3 protease domain variants to telaprevir was determined in 96-well microtiter plates (Coming NBS 3990; Coming, NY) as published previously (Lin et al., (2004) J. Biol. Chem. 279(17), 17508-17514). Briefly, the NS3 protease domain was pre-incubated with 5 μM KK4A peptide in 50 mM HEPES (pH 7.8), 100 mM NaCl, 20% glycerol, mM dithiothreitol at 25°C for 10 min and at 30°C for 10 min. Telaprevir, serially diluted in DMSO, was added to the protease mixture and incubated for an additional 60 min at 30 °C. The reaction was started by the addition of 5 μM RET-S1 substrate and incubated at 30 °C. Product release was monitored for 20 min (excitation at 360 nm and emission at 500 nm) in a Tecan SpectraFluorPlus plate reader (Tecan US, Durham, NC). Total assay volume was 100 μl. Protease concentration was chosen such that 10-20% of the substrate was turned over during the course of the assay. To calculate apparent inhibition constant ($K_{i(app,lh)}$) values, data were fit to the integrated form of Morrison's equation for tight binding inhibition (38) using the GraphPrism software. Steady-state assays showed that the wild type enzyme and all R155K/T/I/S variants had a $K_m$ for RET-S1 that was higher than the limit of detection (100 μM). Thus, the $K_m$ was set to 100 μM for calculating $K_{i(app,lh)}$ values. Inhibitor studies were carried out at a substrate concentration (5 μM) that is significantly below the $K_m$. Therefore, the deviation between the true $K_m$ and the $K_m$ used in calculations should have a negligible effect in the calculation of $K_{i(app,lh)}$.

*Example 8 Sequence Analysis of Baseline Samples*

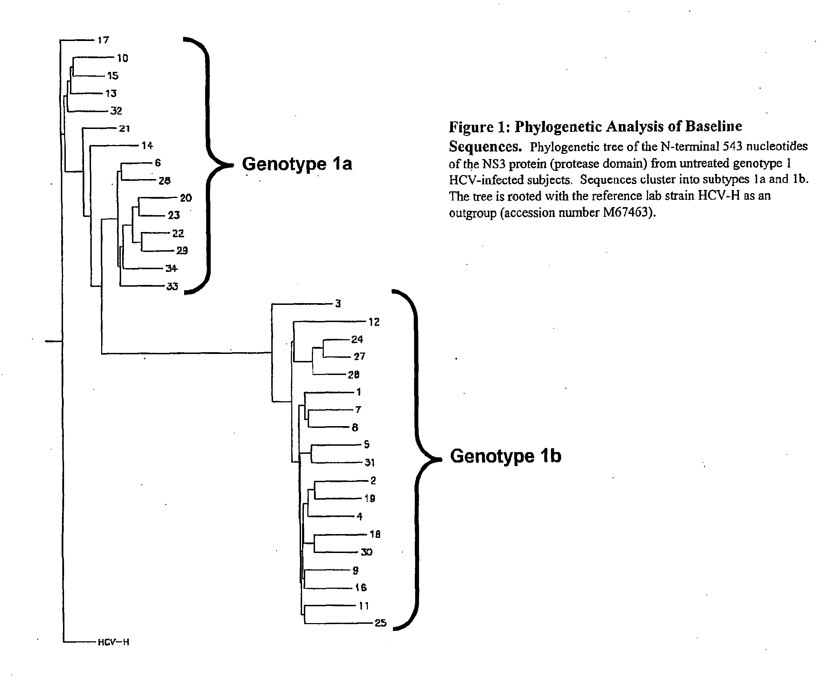

The consensus sequence for each patient's HCV population was derived from an average of 84 independent plasmid clones containing HCV cDNA. Phylogenetic analysis of the consensus sequences indicated that sequences were patient specific (Figure 1). The average intra-patient amino acid quasispecies complexity (Shannon entropy) and diversity (Hamming distance) were low (0.332 ± 0.109 and 0.421 ± 0.195, respectively), and no correlation of quasispecies heterogeneity with HCV RNA plasma concentration at baseline was observed. The inter-patient amino acid diversity (individual consensus compared to genotype 1a or 1b consensus sequence of the patients in this trial) was 1.3% for genotype 1a and 2% for genotype 1b. Structural modeling analysis predicted that amino acid differences observed between consensus sequences of all patients within a subtype would have little or no impact on VX-950 binding.

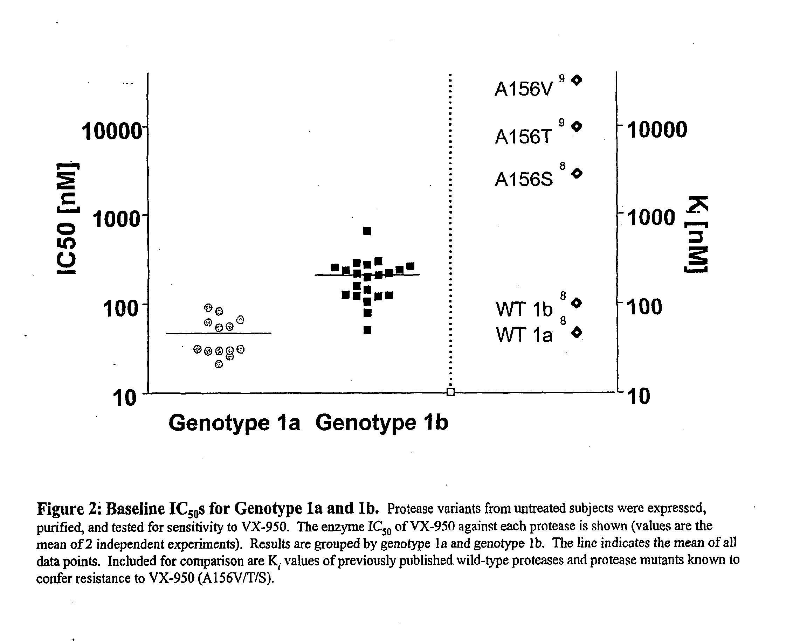

Patient-specific protease clones were then expressed and tested for inhibition by VX-950. In agreement with the modeling observation, there were no significant differences in the enzymatic $IC_{50}$ values of these proteases derived from different patient isolates within a specific subtype. However, the average $IC_{50}$ for genotype 1b patients was slightly higher than for genotype 1a patients (Figure 2). This finding is consistent with previous *in vitro* results measuring the $K_i$ value for HCV-H (1a) and HCV Con (1b) (7). Modeling analysis of 1a versus 1b genotypes suggested that the key difference that may affect the inhibitor/substrate binding is at the residue position 132, whereas other differences are located outside the binding pocket. The $Val^{132}$ side-chain of the genotype 1b protease makes only one van der Waals contact with the P3 terbutyl-glycine group of VX-950, while the $Ile^{132}$ side-chain of the genotype 1a protease makes 2 contacts. This structural difference in interactions is consistent with the experimental data that shows a lower enzyme $IC_{50}$ of VX-950 with the genotype 1a proteases compared to the genotype 1b proteases. Although there is a slight difference between subtypes, both subtypes are still clearly sensitive to VX-950. In conclusion, despite the observed sequence diversity in the HCV NS3 serine protease, genotype 1 patients are expected to be responsive to treatment with the protease inhibitor VX-950. The clinical data supports this finding as no significant difference in viral response to VX-950 was observed.

*Example 9. Genotypic Data: Sequence Analysis of ETR and Follow-up Samples*

The HCV NS3 protease sequences at end of treatment (ETR) were compared to the consensus sequences at baseline for each patient to identify potential resistance mutations. An average of 80 sequences was obtained for each ETR sample, and the percent of variants at each of 181 positions was calculated. Initially, an increase in frequency of 5% or greater at any single amino acid position of the ETR sample compared to the baseline was considered to be a potential resistance mutation. The 5% cut off value was used because this was the lower level of sensitivity of our sequencing protocol, based on the number of clones analyzed and the error rate of the PCR. Changes at sites that were polymorphic at baseline were not considered resistance mutations. Changes at sites which were only present at end of dosing and that were observed in multiple patients were considered potential resistance mutations and these were then analyzed in all the patients.

For analysis, patients were split into groups based on viral load (plasma HCV RNA levels) response to VX-950. Patients were grouped into "initial responders" or "continued responders" in the viral dynamic analysis. In viral sequence analysis, the "initial responder" group was further divided into two groups based on the increase in plasma HCV RNA after the initial decline. Patients who had less than a 0.75 $log_{10}$ increase from the lowest measured HCV RNA level to end of dosing (Day 14) were categorized as patients with an HCV RNA "plateau". Those who had greater than a $0.7510g_{10}$ increase in plasma HCV RNA were categorized as patients with HCV RNA "rebound". Normal fluctuation in HCV RNA in an untreated patient is about 0.5 $log_{10}$, and these groupings were based on antiviral response as well as viral mutational pattern. There were 2 patients for whom these categories were inconsistent between the 2 types of analyses. Patient 12308 had an increase of 0.05 $log_{10}$ from nadir to end of dosing (Day 14) and was categorized as "plateau" for the sequence analysis, while for the viral dynamic analysis 12308 was placed in the "continued responders" group. Patient 3112 had undetectable plasma HCV RNA (<10 IU/ml) at Day 11, but detectable plasma HCV RNA of 35 IU/ml at end of dosing (Day 14). This increase in HCV RNA caused the patient to be placed in "initial responders" group for viral dynamic analysis; however, in the sequence analysis the level of HCV RNA remained undetectable by the sequencing assay and the patient was therefore grouped into the "continued responders" group.

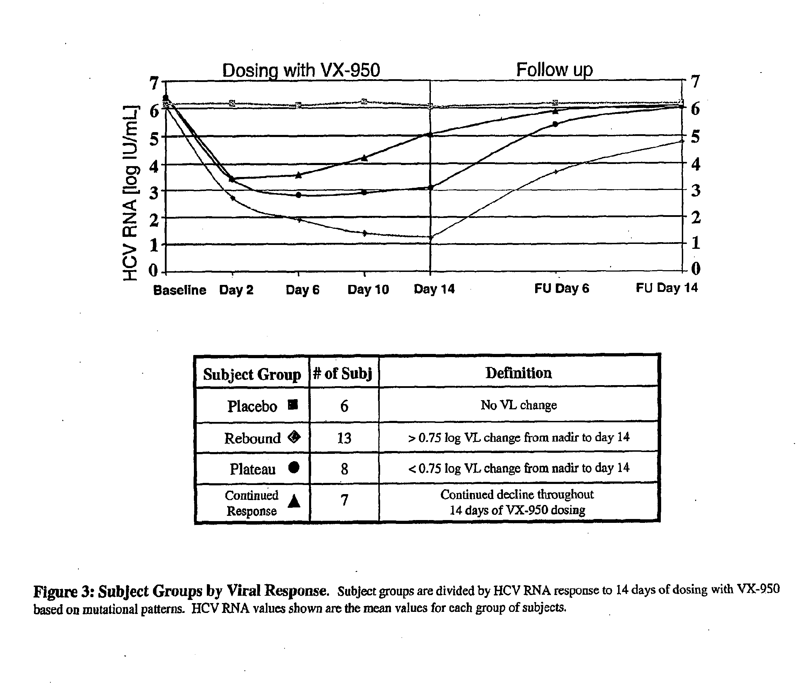

Sequence analysis grouped patients by: no response (placebo); decline followed by rebound during dosing (rebound); decline followed by plateau during dosing (plateau); and continued decline throughout dosing (continued responders) (Figure 3). Additionally, within these groups, patients were analyzed by dose group and genotype subgroup (1a or 1b). Lastly, mutations were analyzed in follow-up samples collected 7 to 10 days after withdrawal of VX-950 to monitor the persistence of any mutations as well as any shift from baseline variants. An average of 81 clones were analyzed for each follow-up sample. Complete sequence analysis is available for 28 of the patients, and analysis of the remaining 6 patients is currently in progress. Analysis is ongoing for 2 patients with rebound, 3 with plateau, and 1 with continued decline. In the first group of placebo patients (n=6), there were no significant changes from baseline at any position in any patient.

*Example 10 Patients with HCV RNA Rebound During Dosing*

There were 13 patients who initially responded to VX-950 with a greater than 2 $log_{10}$ drop in HCV RNA, but eventually began to rebound while still being dosed with VX-950. Of these 13 patients, 6 were in the 450 mg q8h dose group, 1 was in the 750 mg q8h dose group, and 6 were in the 1250 mg q12h dose group. Complete sequence analysis is available for 11 of these patients at Day 14 (Table 1). All of these patients had a significant increase in mutations at position 36. At position 36, the wild-type valine was mutated to an alanine (V36A) in genotype 1b patients (mean 60%, range 31%-86%) and to either an alanine or a methionine (V36M) in genotype 1a patients (mean 62%, range 18%-90%). The absence of V36M in subtype 1b is likely due to the requirement of 2 nucleotide substitutions in subtype 1b versus only a single change in subtype 1a. The V36A mutation requires a single nucleotide change in either subtype 1a or 1b. Mutation to glycine at position 36 (V36G) was also seen in genotype 1b patients and to a leucine (V36L) in 1a patients, but at a much lower frequency. Three patients also had a mutation at position 54 from a threonine to an alanine (T54A) (mean 35%, range 8%-67%) and less frequently to a serine (T54S). Interestingly, the mutations at positions 36 and 54 appear to be mutually exclusive. Additionally, all patients in this group who were genotype 1a contained a mutation at position 155 from an arginine to either lysine (R155K) or threonine (R155T) (mean 60%, range 22%-99%), and less frequently to isoleucine (R155I), serine (R155S), methionine (R155M), or glycine (R155G). The observation that these mutations at R155 is restricted to subtype 1a patients is again likely due to the requirement for 2 nucleotide changes from baseline in the 1b patients versus a single nucleotide change in 1a patients. In follow-up samples from this group, wild-type virus began to re-emerge, but all mutations seen at ETR were still present, although at different frequencies potentially due to differences in viral fitness. No sequencing samples are available for the time point at which rebound was first observed for each patient, so it is not clear if other mutations were present earlier.

*Example 11 Patients with Plateaued HCV RNA Response During Dosing*

In the next group (n=8), patients responded to VX-950 initially, but their HCV RNA response stabilized and did not continue to decline, although no increase in HCV RNA was seen. Two of these patients were in the 450 mg q8h dose group, 2 were in the 750 mg q8h dose group, and 4 were in the 1250 mg q12h dose group. Analysis is complete for 5 of these patients at Day 14 (Table 1). All patients developed a mutation at position 156 from an alanine to either valine (A156V) or threonine (A156T), and very infrequently to a serine (A156S) or isoleucine (A156I). The A156V/T mutation was seen at a very high frequency in 3 of the patients in this group (100%), and no other mutation was seen in these patients. The other 2 patients (02104 and 12308) only had 8% A156T and 30% A156V mutant virus, respectively, but these patients also had mutations at other positions (47% V36A/M and 36% R155K/T for patient 02104; 68% T54A for patient 12308). One patient from the rebound group, 02102, also had a similar frequency of mutations (11%) at position 156. At follow-up time points, the mutation at position 156 was almost completely replaced by either wild-type virus or virus with mutations at positions 36, 54, and/or 155. A change to serine at position 156 (A156S) was the dominant mutation seen during *in vitro* resistance studies of VX-950 (7). The A156V/T mutations were also identified as cross-resistant to both VX-950 and BILN 2061 in an *in vitro* study (7).

*Example 12 Patients with Continued Response During Dosing*

The remaining 7 patients responded to VX-950 with a continuous decline in HCV RNA levels throughout the 14-day dosing period. Five of these patients were in the 750 mg q8h dose group and 2 were in the 450 mg q8h dose group. These patients had very low levels of HCV RNA at Day 14 (64 IU/mL to undetectable (<10 IU/mL)), and sequencing data could not be obtained at this time point. However, HCV cDNA was successfully amplified from the follow-up samples for these patients, and analysis is complete for 6 of these 7 patients (Table 1). The genotype 1a patient (03205) who had reached undetectable levels by Day 14 had 3 mutations, V36A/M (67%), T54A (11%), and R155K/T (26%), at the follow-up time point. Two genotype 1b patients had the V36A (21% and 25%) and T54A mutations (54% and 20%), and another two 1b patients had only low levels of the V36A (3% and 9%) and the T54A (6% and 1%) mutations at follow up. In the last 1b patient who had undetectable HCV RNA levels at Day 14 (<10 IU/mL), no mutations were detected at follow up.

*Example 13 Frequency of Double Mutations*

Frequency of double mutations that were found in patients were also analyzed. The mutations at position 36 were found in combination with mutations at both positions 155 (36/155 double mutation) and 156 (36/156 double mutation). Only genotype 1a patients had the 36/155 double mutation, which was seen in all eight 1a patients analyzed in the rebound group (mean 27.6%, range 10%-77%) and in one of two 1a patients in the plateau group (9%) at Day 14 ETR. The 36/156 double mutation was much less frequent, and found in 3 patients in the rebound group (3%, range 1%-5%) and in 2 patients in the plateau group (1% and 4%) at Day 14 ETR. Double mutations were also found in the follow-up samples at a similar frequency for 36/155 but at a lower frequency for 36/156 as compared with the ETR samples. For the group of patients that continued to decline, only 1 patient had the 36/155 double mutation, which was present at 5%.

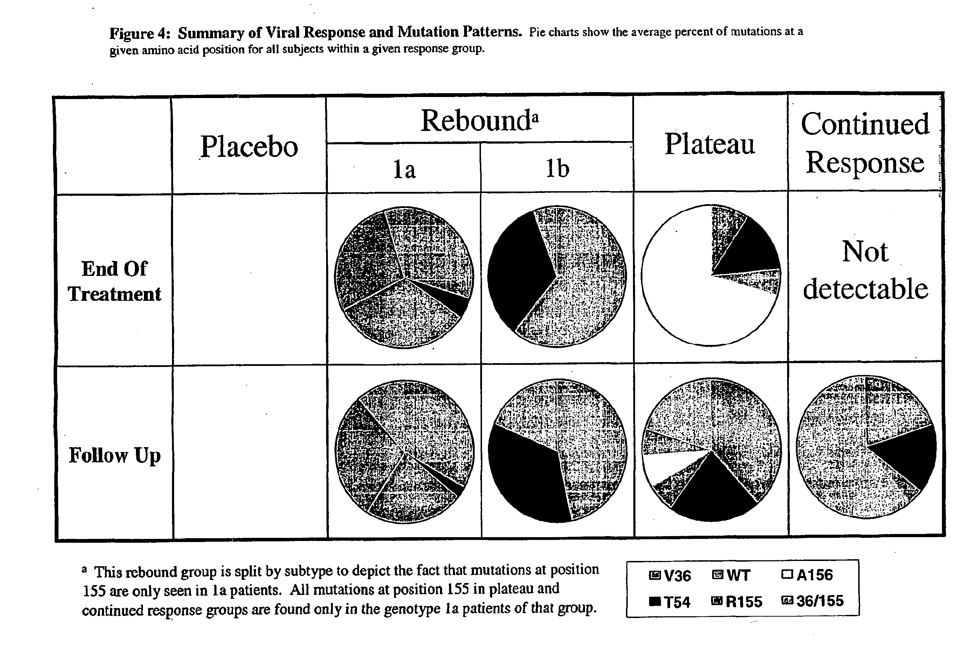

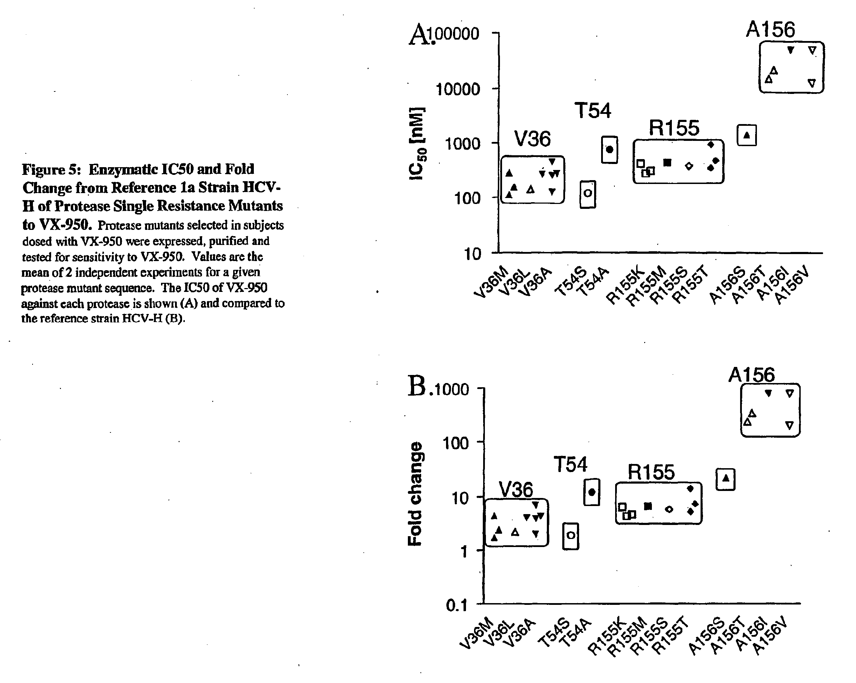

The frequency of mutations found either alone or in combination is shown in Figure 4. A summary of the resistance patterns is shown in Figure 5, which depicts the average percent of mutated amino acids for each patient group.

*Example 14 Phenotypic Data: Enzymatic $IC_{50}$ Analysis of Resistance Mutations*

Since an average of 82 independent sequencing clones were subjected to genotypic analysis, there was a mixture of virions in each patient, as shown in Table 1. The enzyme $IC_{50}$ of all mutants seen in the above sequence analysis (V36A/M/G, T54A/S, R155K/T/M/G/S, and A156T/V/S) as well as any observed combinations of these mutations found *in vivo* were determined in at least 2 different patient-specific genetic backgrounds. The baseline $IC_{50}$ for all patients within a genotype were similar, as reported in *Example 6.* Enzyme $IC_{50}$ values of resistant proteases are reported as fold change compared to the genotype 1a HCV-H reference strain. The $IC_{50}$ of this reference strain was found to be 64 nM in this enzyme assay.

Figure 5 shows the enzyme $IC_{50}$ values and the fold change over the reference strain for single mutants. The values that are similar for any amino acid change at a given position are grouped together in boxes in this figure. A single mutation at position 36 confers low levels of resistance to VX-950. The V36A/M/L mutations show about a 1.5- to 10-fold increase in enzyme $IC_{50}$, regardless of the specific amino acid change. The substitution at position 54 from a threonine to a serine (T54S) does not significantly increase $IC_{50}$. However the T54A mutation gives about a 10-fold increase in enzyme $IC_{50}$. The substitution at position 155 also confers fairly low levels of resistance (about a 5- to 15-fold increase in $IC_{50}$), for any of the changes from an arginine to a threonine (R155T), lysine (R155K), methionine (R155M), or serine (R155S). The mutation at position 156 from an alanine to a valine (A156V), threonine (A156T), or isoleucine (A156I) confers high levels of resistance (about a 400- to 500-fold increase in enzyme $IC_{50}$). Interestingly, the A156S mutation is much less resistant to VX-950 (only a 22-fold increase in $IC_{50}$) than the other amino acid changes at this position, which is consistent with the *in vitro* resistance studies (6, 7).

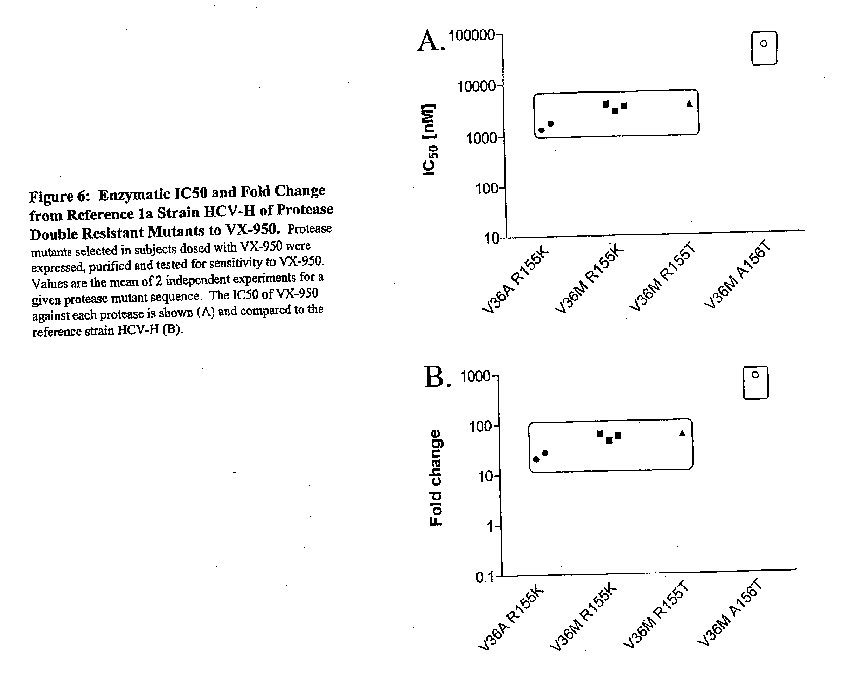

Figure 6 shows the actual enzyme $IC_{50}$ values as well as the fold change over the reference strain for double mutants. Double mutations at these positions give higher levels of resistance than any single mutation. A mutation at position 36 combined with either a mutation at position 155 or 156 gives about an additional 10-fold increase in resistance over the respective single mutants. Table 2 lists the actual $IC_{50}$ values for all mutants tested against both VX-950 as well as another HCV protease inhibitor, BILN 2061. The mutations at positions 36 and 54 affect susceptibility to VX-950 much more than BILN 2061, whereas the mutation at residue 155 confers much higher levels of resistance to BILN 2061. The mutations at position 156 as well as the double mutations all confer high levels of resistance against both inhibitors. However, the A156S mutant is more resistant to VX-950, which has been shown previously *in vitro* (7). Table 3 shows the mean and standard deviation values of the enzyme $IC_{50}$ and fold change over the reference strain of all mutations at a given position.

*Example 15 Average Phenotypes for Each Patient*

The genotypic analyses performed here allowed a detailed examination of the relative proportion of different resistant mutants within each patient. To better understand the level of phenotypic resistance conferred by these viral mixtures, exploratory analyses were done to obtain an average phenotype for each patient. The percent of virions with a given single or double mutation was multiplied by the average fold change in enzyme $IC_{50}$ for that given mutant (Table 3), and then all values were added for each patient. The relative number (value divided by 100) is shown in the last column of Table 1. The mean value for the rebound group of patients was 27 at Day 14 (range 6-70) and 15 at follow-up (range 3-29). The mean value for the plateau group of patients was 272 at Day 14 (range 26-466) and 32 at follow-up (range 1-126). The last group of patients that continued to decline had a mean value of 4 at follow-up (range 0-10). From these very preliminary calculations, it seems that the level of resistance in all groups of patients declines after the last dose (from Day 14 to follow-up), which is not unexpected, as the drug pressure is no longer present for selection of resistant virions. It also appears that the rebound group of patients has lower levels of resistance than the patients that plateaued. An analysis correlating pharmacokinetic data with resistance patterns is currently underway.

*Example 16 Fitness Analysis of Resistance Mutations*

Another important aspect of these viral variants in addition to their resistance profile is the level of fitness. The viral fitness of single and double mutants was calculated (Table 4). The absolute number of infectious units (IU/mL) of each HCV variant in an individual patient at a given time point was determined. For example, the absolute number of infectious units of variants with the A156V/T mutation in Patient 03201 at Day 14 was calculated as: $VL_{d14\ (A156V/T,\ 3201)} = VL_{d14}$ (whole, 3201) $\times$ % A156V/$T_{d14\ (3201)}$. Total HCV RNA at either end-of-dosing (Day 14, $VL_{d14}$ (whole, $_{3201}$)) or follow-up (Day 21, $VL_{d21\ (whole,\ 3201)}$) was taken from the analysis of plasma HCV RNA levels, as determined by the Roche COBAS Taqman HCV assay. Undetectable levels of HCV RNA were considered to be 5 IU/mL. The percentage of each individual variant group (e.g., A156V/T) at either end-of-dosing (Day 14, %A156V/T$_{d14(3201)}$) or follow-up (Day 21, %A156V/T$_{d21\ (3201)}$) was taken from the sequence analysis described above (Table 1). Next, the fold of net increase in plasma HCV viral load (NVL) for each HCV variant and the whole population of HCV in the plasma in the individual patient after dosing (Day 14 to Day 21) was determined. For example:

$$NVL_{(A156V/T,\ 3201)} = VL_{d21\ (A156V/T,\ 3201)} / VL_{d14\ (A156V/T,\ 3201)}$$

$$NVL_{(whole,\ 3201)} = VL_{d21\ (whole,\ 3201)} / VL_{d14\ (whole,\ 3201)}$$

The NVL of each HCV variant was normalized to that of the whole plasma HCV population in each patient: %NVL$_{(A156V/T,\ 3201)}$ = NVL$_{(A156V/T,\ 3201)}$ / NYL$_{(whole,\ 3201)}$.

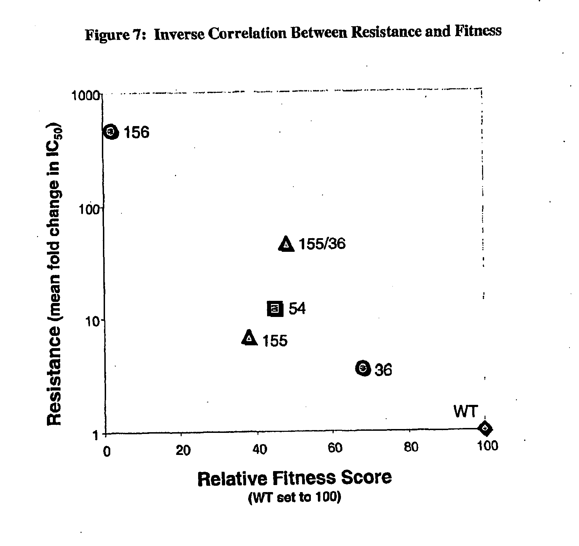

The normalized fold of net increase in viral load of each HCV variant in individual patients were then converted into log$_{10}$-converted values: Log$_{10}$[%NVL$_{(A156V/T,\ 3201)}$], and the average value for a variant was determined from all patients. The fitness of the HCV mutant was then calculated: Fitness for A156V/T mutation =10 $_{Log(fitness,\ A156V/T)}$. This relative fitness score was analyzed with the resistance level of each mutant. As shown in Figure 7, all mutants were less fit than wild-type and, in general, using this analysis there is an inverse correlation between fitness and resistance of single mutants. The double mutant 36/155, however, has a significant increase in resistance with less impact on fitness. This may be due to interaction of the mutations compensating for the loss of fitness, while still conferring resistance.

*Example 17 X-ray structure of the R155K variant protease*

Purified R155K variant of the HCV-H strain protease domain, in a complex with an NS4A peptide co-factor (Kim et al., (1996) Cell 87(2), 343-355), at a concentration of 8.0 mg/ml was used for crystallization trials. The protein crystals were grown over a reservoir liquid of 0.1 M MES (pH 6.2), 1.4 M NaCl, 0.3 M KH$_2$PO4, and 10 mM β-mercaptoethanol. Single crystals were obtained in the hanging droplets after equilibrating over two days. A single crystal with dimensions of 0.15 x 0.15 x 0.35 mm was transferred into cryo-protectant solution of mother liquid with 25% glycerol added shortly prior to be flush-cooled to 100K in nitrogen gas stream. The diffraction images were collected using a CCD4 image plate instrument mounted on an ALS beam line 5,01. Data at 2.5-Å resolution was indexed and integrated using HKL (2000, HKL Incorporated, Charlottesville, VA) and CCP4 software. The crystals belong to space group R32 with unit cell dimensions of a=225.31 Å, b=225.31 Å, c=75.66 A, α=90.00°, β=90.00° and γ= 120.00°. There are 5% of data assigned for testing free R-factor in the later refinements. The crystals of the R155K variant studied here have an identical crystallographic lattice to that of the wild-type protease NS3-4A published previously (Kim et al., *supra*). The published NS3-4A protease domain (PDB code: 1A1R) was used to perform the initial rigid-body and positional refinement of the model. The difference in the side chains of residue 155 was confirmed to be a Lys instead of Arg in the electron density map. The protein molecule was visually inspected against the electron density map using QUANTA programs. Further inclusion of solvent molecules in the refinement and individual B-factor refinement at a resolution range of 20.0 to 2.5 Å reduced the R-factor and free R-factor to 22.0% and 24.7%, respectively. Residues included in the refined model range from amino acids 1 to 181 of the NS3 protease domain and residues 21 to 39 of the NS4A cofactor for crystallographic independent molecule and two Zn metal ions.

*Example 18 Computational Modeling*

Telaprevir was modeled into the active site of the R155K variant NS3 protease domain using the X-ray crystal structure of the R155K variant apo-enzyme in a complex with an NS4A cofactor peptide, following the procedure described previously (Lin et al., *supra*). The ketoamide group of telaprevir was modeled to form a covalent adduct with the Ser[139] side-chain with a si-face attachment. This binding mode was observed for analogous ketoamide inhibitors (Perni et al., (2004) Bioorg. Med. Chem. Lett. 14(6), 1441-1446) and ketoacid inhibitors (Di Marco et al., (2000) J. Biol. Chem. 275 (10), 7152-7157.). The main-chain of the inhibitor was overlaid with the analogous main-chain of these ketoamide and ketoacid inhibitors such that the telaprevir main-chain makes all the following backbone hydrogen bonds: P1 NH with Lys[155] carbonyl, P3 carbonyl with Ala[157] NH, P3 NH with Ala[157] carbonyl, and P4 cap carbonyl with NH of Cys[159]. In this binding mode, the P2 group of telaprevir was placed in the S2 pocket without any need to move the Lys[155] side-chain. The *t*-butyl and the cyclohexyl groups of telaprevir were placed in the S3 and S4 pockets, respectively. The inhibitor was energy minimized in two stages. In the first stage, only the inhibitor and the side-chain atoms of Arg[123], Lys[155] and Asp[168] of the protease were allowed to move during energy minimization for 1000 steps. In the second stage, all the side-chain atoms of the protease were allowed to move along with the inhibitor for 1000 additional steps. This modeled structure closely mimics the telaprevir model in the active site of the wild-type NS3 protease described previously (Lin et al., *supra*). No significant shifts in the positions of Lys[155] side-chain or the other active residue side-chains were observed. The same procedure was repeated for docking telaprevir into the active site of the R155T variant of the NS3 protease domain. However, the enzyme structure in this case is not the X-ray crystal structure, but a model built using the R155K variant crystal structure. The Lys[155] residue was replaced with Thr side-chain and was minimized by holding all the atoms of the enzyme fixed except for the Thr[155] side-chain. In this model, the hydroxyl group of the Thr[155] side-chain forms a hydrogen bond with the side-chain of Asp[81]. All modeling and minimization procedures were carried out using the QUANTA molecular modeling software (Accelrys Incorporated, San Diego, CA).

*Example 19 Study Results*

Results from enzymatic assays and structural studies are presented here, in addition to the discussions above for certain variants of the invention.

1) Substitutions at Arg[155] of the NS3 protease confer low-level resistance to telaprevir in HCV replicon cells

To determine whether the observed substitutions of Arg[155] of the HCV NS3 protease domain are sufficient to confer resistance to telaprevir (VX-950), several substitutions at NS3 residue 155 (R155K, R155T, R155S, R155I, R155M, or R155G) were introduced into a high-efficiency subgenomic replicon plasmid (Con1-mADE). Stable HCV replicon cells were generated for each of these variants, indicating that replacement of NS3 Arg[155] with a different residue did not abolish HCV RNA replication in cells. The average 48-h $IC_{50}$ value of telaprevir in the wild-type HCV replicon Con1-mADE cells was 0.485 ± 0.108 µM, which is slightly higher than what had been determined previously (0.354 µM) in Con1-based HCV replicon cells with a different set of adaptive mutations (24-2) (25,41). Two major Arg[155] variants, R155K and R155T, which were observed in a phase 1b trial of telaprevir alone, had average 48-h telaprevir $IC_{50}$ values of 3.59 ± 0.28 µM for R155K and 9.60 ± 0.87 µM for R155T. This corresponds to a 7.4- or 20-fold increase, respectively, compared to the wild-type Con1-mADE replicons (Table I). Similar decreases in sensitivity to telaprevir were observed in HCV replicon cells containing the other four minor variants at Arg[155]: R155S, R155I, R155M, or R155G; Table I. These 4 variants were found at much lower frequency than R155K/T in the telaprevir phase 1b trial. The replicon 48-h $IC_{50}$ values for these four variants were 1.97 ± 0.21 µM (R155S), 11.7 ± 2.5 µM (R155I), 2.68 ± 0.21 µM (R155M), and 3.58 ± 0.24 µM (R155G), which corresponds to a 4.1-, 24-, 5.5-, and 7.4-fold, respectively, loss of sensitivity to telaprevir (Table I). These results indicate that substitutions of NS3 Arg[155] led to low-level (<25-fold) resistance to telaprevir in HCV replicon cells, independent of the physical properties of the substituted residue, which include a positive charged residue (Lys), a hydrophilic residue (Thr or Ser), a hydrophobic residue (Ile or Met), or a residue that lacks a side chain (Gly).

Table I. Demonstration of Resistance in HCV Replicon Cell assays

| Variants | Replicon $IC_{50}$ of telaprevir (µM) | Fold change |
|---|---|---|
| Wild-type | 0.485 ± 0.108 | 1.0 ± 0.2 |
| R155K | 3.59 ± 0.28 | 7.4 ± 0.6 |
| R155T | 9.60 ± 0.87 | 20 ± 2 |
| R155S | 1.97 ± 0.21 | 4.1 ± 0.4 |
| R155I | 11.7 ± 2.5 | 24.0 ± 5.2 |
| R155M | 2.68 ± 0.21 | 5.5 ± 0.4 |
| R155G | 3.58 ± 0.24 | 7.4 ± 0.5 |

The stable wild-type (mADE) and variant HCV sub-genomic replicon cell lines were generated using the T7 RNA runoff transcripts from the corresponding high efficiency Con1 replicon plasmids. The average $IC_{50}$ values and SD of telaprevir were determined for the HCV replicon cell lines in the 48-h assay in three independent experiments. Fold change was determined by dividing the $IC_{50}$ of a given variant by that of the wild-type HCV replicon.

2) Substitutions at Arg[155] of the HCV NS3 protease resulted in a decreased sensitivity to telaprevir in enzyme assays

To confirm whether substitutions at Arg[155] in the HCV NS3 protease domain are sufficient to cause a loss of sensitivity to telaprevir at the enzyme level, Arg[155] was replaced with Lys, Thr, Ser, or Ile in an NS3 protease domain (genotype 1a) from which the sequences were derived from HCV samples in a patient prior to dosing with telaprevir.

The NS3 serine protease domain proteins containing R155K, R155T, R155S, or R155I mutations were expressed in *E. coli* and purified prior to determination of enzyme sensitivity to telaprevir. Resistance to telaprevir was defined by the fold-change in $Ki_{(app,1h)}$, which is the apparent $K_i$ measured after a 1-h pre-incubation with telaprevir.

A comparison of the sensitivity of telaprevir for the wild-type HCV protease domain co-complexed with the KK4A cofactor peptide versus variants with substitutions at Arg[155] is shown in Table II. The average $K_{i(app,1h)}$ value of telaprevir against the wild-type genotype 1a patient NS3 protease domain complexed with the KK4A peptide was 0.044 ± 0.033 μM (Table II). In contrast, the average $K_{i(app,1h)}$ values of telaprevir were about 11-fold higher for the R155K protease and 9-fold higher for the R155T protease (Table II), the two major variants observed in the phase 1b trial of telaprevir alone. R155S and R155I, two of the minor Arg[155] variants observed in the telaprevir phase 1b trial, showed 22-fold and 16-fold increases in $K_{i(app,1h)}$ values, respectively, compared to that of wild-type protease (Table II). These data indicate that substitutions of Arg[155] with different amino acids, including a conservative substitution of another positively charged residue (Lys), results in a decreased sensitivity to telaprevir.

Table II. Demonstration of Resistance in HCV NS3 Protease Enzyme Assays

| Variants | $K_{i(app,1h)}$ of telaprevir (μM) | Fold change |
|---|---|---|
| Wild-type (n=5) | 0.044 ± 0.033 | 1.0 ± 0.8 |
| R155K (n=4) | 0.49 ± 0.22 | 11 ± 5 |
| R155T (n=5) | 0.38 ± 0.18 | 8.7 ± 4.2 |
| R155S (n=3) | 0.97 ± 0.70 | 22 ± 16 |
| R155I (n=3) | 0.71 ± 0.35 | 16 ± 8 |

The average $K_{i(app, 1h)}$ values and SD oftelaprevir were determined for the purified wild-type and for four variant HCV NS3 serine protease domains using the KK4A cofactor peptide and the FRET substrate in three to five independent experiments. Fold change was determined by dividing the $K_{i(app,1h)}$ of a given variant by that of the wild-type protease.

3) <u>X-ray structure of the R155K HCV NS3 protease</u>

To understand why substitution of Arg[155] with another residue, including a positively charged amino acid (Lys), results in a loss of sensitivity to telaprevir, the X-ray crystal structure of the R155K NS3 protease was determined. The R155K mutation was engineered into a T7-tag fused HCV-H protease construct that had previously been used to determinate the structure of the wild-type protease co-complexed with NS4A cofactor peptide. The ensemble structure of the R155K protease domain co-complexed with NS4A cofactor peptide, with a resolution of 2.5 Å, is very similar to that of wild-type HCV-H strain protease complex described previously (Kim et al., *supra*). Briefly, one molecule of NS3 protease domain (residues 1 to 181) and one molecule of the co-factor NS4A (residues 21 to 39) form the globular entity, which, in turn, forms the homodimer with another globular entity in the asymmetrical unit. One globular unit of the Lys[155] variant protease in complex with the NS4A cofactor was superimposed with the wild-type Arg[155] co-complex. Because the rms deviation of Cα atoms was only 0.314 Å, there is little difference in structure of these two proteases.

A close-up view of the side chains of NS3 protease residues 123, 168 and 155 in the S2 and S4 pockets is shown in Figure 25(B). The overall shift of residue 155 side-chain (Lys[155] versus Arg[155]) is small as evidenced by the distance between the Cδ of residue 155 and the Cββ of the Asp[81], one of the catalytic triad residues: 4.26 Å for Lys[155] and 4.24 Å for Arg[155]. In the R155K protease, the distance between the terminal amine (Nz) of Lys[155] and the Cβ of Asp[81] is 3.5 Å, and the distance between the Cδ of Lys[155] and the Cβ of Asp[81] is 3.6 Å. In the wild-type protease, the NH1 and Nz of Arg[155] are 5.6 Å and 5.3 Å, respectively, away from the Cβ of its Asp[81]. Thus, the terminal amine group of Lys[155] in the R155K protease is closer to the carboxyl group of Asp[81] than the comparative terminal azide group of Arg[155]. In contrast, the distance between Nz of Lys[155] and the carboxyl atom Oε2 of Asp[168] is 5.8 Å in the R155K protease compared to 3.2 Å between the corresponding pair of the terminal NH2 of Arg[155] and the Oε2 of Asp[168] in the wild-type protease. Thus, the terminal amine group of Lys[155] in the R155K protease is further away from the carboxyl group of Asp[168] than the comparative terminal azide group of Arg[155]. Therefore, substitution of Arg with Lys at residue 155 alters the shape of the S2 binding pocket such that the positive charge at the Nz atom of Lys[155] can not be neutralized by the adjacent side-chain of Asp[168] as is the case of the wild-type Arg[155] and Asp[168] pair.

4) <u>Mechanism of resistance of R155K or R155T variant to telaprevir</u>

A structural model of the interactions between telaprevir and NS3 protease has previously been described (e.g., Lin et al., *supra*). In a model of the co-complex of telaprevir with the R155K enzyme, the same interactions were maintained except for differences at the side-chain of residue 155. In the wild-type protease structure, the Arg[155] side-

chain bends over the bicyclic P2 group of telaprevir to make several direct van der Waals contacts, and provides a hydrophobic environment for the P2 group of telaprevir (Figure 26(A)). However, the Lys[155] side-chain of the R155K enzyme has an extended conformation and makes only one or two direct contacts with the P2 group, thus leaving the P2 group of telaprevir more exposed to the solvent (Figure 26(B)). This observation is consistent with the R155K enzyme being less sensitive to telaprevir, as shown with in vitro enzyme assays. It is reasonable to assume that the R155M variant will have an extended conformation of Met[155] side-chain, and therefore similarly fewer interactions with the P2 group of telaprevir, consistent with observed decrease in binding of the inhibitor.

To understand the mechanism of binding of telaprevir to variants with residues with shorter side-chains at position 155, the model of R155T variant enzyme complexed with telaprevir was used. It is obvious from the model that Thr[155] side-chain is too short to provide a hydrophobic cover for the P2 group of the inhibitor (Figure 26(C)). Other shorter side-chains like Ile, Ser and Gly are similar or even shorter in size and are expected to lose interactions with the P2 group and have a decreased binding affinity to the telaprevir.

5) <u>HCV variant replicons with substitutions at Arg[155] of the NS3 protease remain fully sensitive to IFN-$\alpha$</u>

Whether the telaprevir-resistant variant replicon cells with substitution at NS3 residue 155 remain sensitive to IFN-$\alpha$ or ribavirin was also determined. As shown in Table III, the $IC_{50}$ of either IFN-$\alpha$ or ribavirin remained virtually the same for HCV replicon cells containing R155K, R155T, or R155M mutations compared to the wild-type replicon cells. These results suggest that combination with IFN-$\alpha$ with or without ribavirin could be a potential therapeutic strategy to suppress the emergence of HCV variants with substitutions at NS3 protease residue 155.

Table III. Lack of Resistance to Other Anti-HCV Agents in HCV Replicon Cells

| Variants | Replicon $IC_{50}$ | | | |
|---|---|---|---|---|
| | IFN-$\alpha$ (U/ml) | Fold change | Ribavirin ($\mu$M) | Fold change |
| Wild-type | 11.6 $\pm$ 1.1 | 1.0 $\pm$ 0.1 | 58 $\pm$ 18 | 1.0 $\pm$ 0.3 |
| R155K | 15.2 $\pm$ 12.3 | 1.3 $\pm$ 1.1 | 37 $\pm$ 17 | 0.6 $\pm$ 0.3 |
| R155T | 4.8 $\pm$ 3.3 | 0.4 $\pm$ 0.3 | 32 $\pm$ 18 | 0.6 $\pm$ 0.3 |
| R155M | 4.9 $\pm$ 1.0 | 0.4 $\pm$ 0.1 | 39 $\pm$ 5 | 0.7 $\pm$ 0.1 |

The stable wild-type and variant HCV sub-genomic replicon cell lines were generated using the T7 RNA runoff transcripts from the corresponding high efficiency Con1 replicon plasmids. The average $IC_{50}$ values and SD of IFN-$\alpha$ and ribavirin were determined for the HCV replicon cell lines in the 48-h assay in three independent experiments. Fold change was determined by dividing the $IC_{50}$ of a given variant by that of the wild-type HCV replicon.

*Example 20 Summary of the Study Results*

One study was to monitor the possible emergence of drug resistant mutations to VX-950 monotherapy by sequence analysis of the HCV protease NS3-4A region in subjects with genotype 1 hepatitis C who were dosed with VX-950 for 14 days. Traditionally, resistance genotyping has been done by population-based sequencing, which detects the dominant sequence in the plasma virus. Any sequences that constitute less than 20% of the viral population will not be detected by this method. Because drug-resistance mutations may take longer than 14 days to accumulate to this measurable level, a new method was developed to detect minor populations of variants. Sequences were obtained from about 80-85 individual viral clones per subject per time point, so that resistant mutations that may emerge in 14 days of dosing with VX-950 with a sensitivity of down to about 5% of the population can be identified.

In subjects grouped by viral response to VX-950, distinct mutational patterns were observed. In the first group of subjects whose HCV RNA rebounded during the dosing period, wild-type virus was almost completely replaced by 1 of 3 viral variants containing a mutation at position 36, 54, or 155 at ETR and follow up. A V36A mutation was found in genotype 1b subjects, whereas a V36A/M or R155K/T was seen in the genotype 1a subjects. Some variants also contained a double mutation at positions 36 and 155 in 1a subjects. A T54A mutation was seen in both 1a and 1b subtypes. The mutations at positions 36 and 54 appear to be mutually exclusive as they were rarely found together in the same genome. A second group of subjects had an initial HCV RNA decline that leveled off at the end of the 14-day dosing period. These subjects harbored virus that contained a mutation at position 156 from an alanine to either a valine (A156V) or threonine (A156T). This mutation at position 156 has previously been shown to develop in vitro in the presence of VX-950 (6, 7). Some subjects harbored a few variants that also contained a double mutation at positions 36 and 156.

Subject-specific protease clones were expressed and tested for inhibition by VX-950. There were no significant differences in the enzyme $IC_{50}$ values of the baseline proteases derived from different subject isolates within a subtype. However, the IC50 values of the mutant proteases indicate varying degrees of decreased sensitivity to VX- 950. HCV RNA in the last group of subjects continued to decline throughout the dosing period, and some reached levels below the limit of detection of the current assay (10 IU/mL). Due to the limit of sensitivity of our sequencing assay (>100 IU/mL), no viral sequence data are available for these subjects at Day 14 of dosing. However, samples taken 7 to 10 days after the last dose of VX-950 were successfully sequenced for all subjects. In the follow-up samples from the first two response groups, resistant mutations were found to persist in the plasma of all subjects. However, in many cases, the frequency of mutation at position 156 was significantly decreased, as the wild-type as well as the mutations at position 36 or 54 began to increase in proportion. The proportion of virions with a mutation at position 155 remained relatively constant. These shifts in mutation patterns are likely due to fitness differences between variants in the absence of drug selective pressure. It appears that viruses with mutation at positions 36, 54, or 155, although less fit than wild-type, are still reasonably fit, whereas the residue 156 mutants are quite unfit in the absence of drug. From these data, there seems to be an inverse correlation between the level of resistance and the fitness for different single mutants. The data derived from the group of subjects with a plateau in plasma HCV RNA indicated the influences of virologic resistance and fitness in producing a given clinical response. Thus, the clinical response cannot, itself, indicate the underlying virology.

Analysis of the last group of subjects who had continued HCV RNA decline during dosing and reached low levels of HCV RNA reveals that virus present at follow-up consisted of mostly low level resistance mutations at positions 36 and 54, and 3 of the subjects harbored few mutations but were mostly or completely wild-type: Although it is unknown what variants, if any, were present at Day 14, this result suggests that with an optimal response to VX-950, it may be possible to avoid clinical resistance with monotherapy or by the addition of other antiviral compounds such as Peg-IFN. Optimizing dosing regimens may extend this response to a greater number of patients.

In summary, these results indicate that the dosing regimen of VX-950 in this study can result in the selection of different mutations in the NS3 protease with varying levels of drug resistance. Increased concentrations of VX-950 are expected to prevent emergence of virus with low level resistance (at positions 36, 54, and 155). The remaining high level resistant virus (at position 156) may be overcome through different treatment options, including combination therapy. Higher drug concentrations may inhibit viral replication more completely, causing a steeper slope of initial decline; thus reducing the chance that resistant mutations will be selected and cause clinical resistance. The addition of Peg-IFN to VX-950 treatment may enhance immune-mediated clearance of the virus, and the effectiveness of immune-mediated clearance should not be affected by the presence of resistant variants. Although the mutation at position 156 confers high levels of resistance, it appears to come with significant fitness costs, as measured by its relative rate of replication in the absence of VX-950. There is increasing evidence that antiviral drug resistance is associated with impaired viral fitness, which can translate into a clinical benefit (1-3, 9). Resistant virus replicating at such a low level may not accumulate compensatory fitness mutations immediately, allowing the host immune system or other drugs, such as Peg-IFN, to clear the remaining virus.

References

1. Brenner, B. G., D. Turner, and M. A. Wainberg. 2002. HIV-1 drug resistance: can we overcome? Expert Opin. Biol. Ther. 2:751-61.

2. Buckheit, R. W., Jr. 2004. Understanding HIV resistance, fitness, replication capacity and compensation: targeting viral fitness as a therapeutic strategy. Expert Opin. Investig. Drugs 13:933-58.

3. Gilbert, C., J. Bestman-Smith, and G. Boivin. 2002. Resistance of herpesviruses to antiviral drugs: clinical impacts and molecular mechanisms. Drug Resist. Updat. 5:88-114.

4. Grossman, T. H., E. S. Kawasaki, S. R. Punreddy, and M. S. Osburne. 1998. Spontaneous cAMP-dependent derepression of gene expression in stationary phase plays a role in recombinant expression instability. Gene 209: 95-103.

5. Johnson, V. A., F. Brun-Vezinet, B. Clotet, B. Conway, R. T. D'Aquila, L. M. Demeter, D. R. Kuritzkes, D. Pillay, J. M. Schapiro, A. Telenti, and D. D. Richman. 2004. Update of the drug resistance mutations in HIV-1: 2004. Top. HIV Med. 12:119-24.

6. Lin, C., C. A. Gates, B. G. Rao, D. L. Brennan, J. R. Fulghum, Y.-P. Luong, J. D. Frantz, K. Lin, S. Ma, Y.-Y. Wei, R. B. Perni, and A. D. Kwong. 2005. In vitro studies of cross-resistance mutations against two hepatitis C virus

**EP 1 951 748 B1**

serine protease inhibitors, VX-950 and BILN 2061. J. Biol. Chem. 280:36784-36791.

7. Lin, C., K. Lin, Y. P. Luong, B. G. Rao, Y. Y. Wei, D. L. Brennan, J. R. Fulghum, H. M. Hsiao, S. Ma, J. P. Maxwell, K. M. Cottrell, R. B. Perni, C. A. Gates, and A. D. Kwong. 2004. In vitro resistance studies of hepatitis C virus serine protease inhibitors, VX-950 and BILN 2061: Structural analysis indicates different resistance mechanisms. J. Biol. Chem. 279:17508-17514.

8. Lu, L., T. J. Pilot-Matias, K. D. Stewart, J. T. Randolph, R. Pithawalla, W. He, P. P. Huang, L. L. Klein, H. Mo, and A. Molla. 2004. Mutations conferring resistance to a potent hepatitis C virus serine protease inhibitor in vitro. Antimicrob. Agents Chemother. 48:2260-6.

9. Maisnier-Patin, S., and D. I. Andersson. 2004. Adaptation to the deleterious effects of antimicrobial drug resistance mutations by compensatory evolution. Res. Microbiol. 155:360-9.

10. Pawlotsky, J. M., and J. G. McHutchison. 2004. Hepatitis C. Development of new drugs and clinical trials: promises and pitfalls. Summary of an AASLD hepatitis single topic conference, Chicago, IL, February 27-March 1, 2003. Hepatology 39:554-67.

11. Simmonds, P. 2004. Genetic diversity and evolution of hepatitis C virus--15 years on. J. Gen. Virol. 85:3173-88.

12. Strader, D. B., T. Wright, D. L. Thomas, and L. B. Seeff. 2004. Diagnosis, management, and treatment of hepatitis C. Hepatology 39:1147-71.

13. Trozzi, C., L. Bartholomew, A. Ceccacci, G. Biasiol, L. Pacini, S. Altamura, F. Narjes, E. Muraglia, G. Paonessa, U. Koch, R. De Francesco, C. Steinkuhler, and G. Migliaccio. 2003. In vitro selection and characterization of hepatitis C virus serine protease variants resistant to an active-site peptide inhibitor. J. Virol. 77:3669-79.

Table 1. Mutations in the HCV Protease Alone or in Combination Found After Dosing with VX-950

| Subject Group | Subj ID | VX-950 Dose | Geno - type | Δ in VL[a] | Time Point[b] | % Single[c] | | | | % Double[c] | | Average Phenotype[d] |
|---|---|---|---|---|---|---|---|---|---|---|---|---|
| | | | | | | V36 | T54 | R155 | A156 | 36/155 | 36/15 6 | |
| Rebound | 2101 | 450 | 1a | 2.56 | ETR[e] | 9 | 4 | 13 | 0 | 77 | 0 | 36.70 |
| | | | | | FU[r] | 23.5 | 0 | 11.5 | 0 | 60.5 | 0 | 29.43 |
| | 2102 | 450 | 1a | 1.84 | ETR | 35 | 3 | 32 | 8 | 12 | 3 | 69.69 |
| | | | | | FU | 31 | 0 | 26 | 0 | 26 | 0 | 14.81 |
| | 2105 | 450 | 1b | 1.06 | ETR | 86 | 0 | 0 | 5 | 0 | 0 | 26.31 |
| | | | | | FU | 45 | 32 | 0 | 6 | 0 | 0 | 28.94 |
| | 2107 | 450 | 1b | 2.09 | ETR | n/a[g] | n/a | n/a | n/a | n/a | n/a | n/a |
| | | | | | FU | n/a | n/a | n/a | n/a | n/a | n/a | n/a |
| | 3108 | 450 | 1b | 2.08 | ETR | 66 | 31 | 0 | 0 | 0 | 0 | 6.03 |
| | | | | | FU | 62 | 8 | 0 | 0 | 0 | 0 | 3.13 |
| | 3111 | 450 | 1b | 1.82 | ETR | 24 | 67 | 0 | 2 | 0 | 1 | 30.71 |
| | | | | | FU | 31 | 63 | 0 | 0 | 0 | 0 | 8.65 |
| | 2211 | 750 | 1a | 2.35 | ETR | n/a | n/a | n/a | n/a | n/a | n/a | n/a |
| | | | | | FU | 35 | 1 | 31 | 0 | 13 | 0 | 9.33 |
| | 2310 | 1250 | 1a | 0.86 | ETR | 42 | 0 | 33 | 0 | 14 | 0 | 10.15 |
| | | | | | FU | 41 | 1 | 22 | 0 | 14 | 0 | 9.39 |
| | 2312 | 1250 | 1a | 3.20 | ETR | 38 | 14 | 9 | 0 | 52 | 0 | 26.13 |
| | | | | | FU | 36 | 9 | 10 | 0 | 52 | 0 | 26.03 |
| | 3301 | 1250 | 1a | 1.16 | ETR | 8 | 4 | 65 | 0 | 10 | 0 | 9.38 |
| | | | | | FU | 28 | 0 | 34 | 0 | 16 | 0 | 10.65 |
| | 3302 | 1250 | 1a | 2.50 | ETR | 39 | 8 | 15 | 0 | 24 | 0 | 14.39 |
| | | | | | FU | 31 | 10 | 26 | 0 | 18 | 0 | 12.33 |
| | 3303 | 1250 | 1a | 1.93 | ETR | 0 | 1 | 79 | 0 | 20 | 0 | 14.59 |
| | | | | | FU | 6 | 0 | 66 | 0 | 23 | 0 | 15.28 |

EP 1 951 748 B1

| Subject Group | Subj ID | VX-950 Dose | Geno - type | Δ in VL[a] | Time Point[b] | % Single[c] | | | | % Double[c] | | Average Phenotype[d] |
|---|---|---|---|---|---|---|---|---|---|---|---|---|
| | | | | | | V36 | T54 | R155 | A156 | 36/155 | 36/15 6 | |
| | 3305 | 1250 | 1a | 2.25 | ETR | 63 | 2 | 10 | 0 | 12 | 4.5 | 47.49 |
| | | | | | FU | 77 | 0 | 4 | 0 | 10 | 1 | 15.38 |
| | | | | | | | | | | | | |
| Plateau | 2104 | 450 | 1a | 0.65 | ETR | 34 | 0 | 27 | 4 | 9 | 4 | 57.05 |
| | | | | | FU | 82 | 0 | 0 | 0 | 8 | 0 | 6.55 |
| | 2106 | 450 | 1b | 0.41 | ETR | n/a | n/a | n/a | n/a | n/a | n/a | n/a |
| | | | | | FU | 5 | 8 | 0 | 0 | 0 | 0 | 1.14 |
| | 3201 | 750 | 1a | 0.73 | ETR | 0 | 0 | 2 | 100 | 0 | 0 | 466.14 |
| | | | | | FU | 25 | 2 | 24 | 3 | 22 | 3 | 45.74 |
| | 3202 | 750 | 1b | 0.22 | ETR | 0 | 0 | 0 | 100 | 0 | 0 | 466.00 |
| | | | | | FU | 24 | 16 | 0 | 29 | 0 | 2 | 126.48 |
| | 1230 8 | 1250 | 1b | 0.05 | ETR | 0 | 68 | 0 | 30 | 0 | 0 | 147.96 |
| | | | | | FU | 7 | 64 | 0 | 0 | 0 | 0 | 7.93 |
| | 2309 | 1250 | 1b | 0.33 | ETR | n/a | n/a | n/a | n/a | n/a | n/a | n/a |
| | | | | | FU | 19 | 10 | 0 | 0 | 0 | 0 | 1.87 |
| | 2311 | 1250 | 1a | 0.62 | ETR | 72 | 92 | 12 | 2 | 8 | 1 | 25.96 |
| | | | | | FU | n/a | n/a | n/a | n/a | n/a | n/a | n/a |
| | 3306 | 1250 | 1b | 0.26 | ETR | 0 | 0 | 0 | 100 | 0 | 0 | 466.00 |
| | | | | | FU | 52 | 25 | 0 | 4 | 0 | 1 | 30.97 |
| Subject Group | Subj ID | VX-950 Dose | Geno -type | Δ in VL[a] | Time Point[b] | % Single[c] | | | | % Double[c] | | Average Phenotype[d] |
| | | | | | | V36 | T54 | R155 | A156 | 36/155 | 36/15 6 | |
| Continued Response | 3110 | 450 | 1b | - | ETR | nd[h] | nd | nd | nd | nd | nd | nd |
| | | | | | FU | 21 | 53.5 | 0 | 0 | 0 | 0 | 6.80 |

EP 1 951 748 B1

| Subject Group | Subj ID | VX-950 Dose | Geno -type | Δ in VL[a] | Time Point[b] | % Single[c] | | | | % Double[c] | | Average Phenotype[d] |
|---|---|---|---|---|---|---|---|---|---|---|---|---|
| | | | | | | V36 | T54 | R155 | A156 | 36/155 | 36/15 6 | |
| | 3112 | 450 | 1b | - | ETR | nd | nd | nd | nd | nd | nd | nd |
| | | | | | FU | 3 | 6 | 0 | 0 | 0 | 0 | 0.83 |
| | 2207 | 750 | 1b | - | ETR | nd | nd | nd | nd | nd | nd | nd |
| | | | | | FU | n/a | n/a | n/a | n/a | n/a | n/a | n/a |
| | 3203 | 750 | 1b | - | ETR | nd | nd | nd | nd | nd | nd | nd |
| | | | | | FU | 9 | 1 | 0 | 2 | 0 | 0 | 9.65 |
| | 3204 | 750 | 1b | - - | ETR | nd | nd | nd | nd | nd | nd | nd |
| | | | | | FU | 25 | 20 | 0 | 0 | 0 | 0 | 3.17 |
| | 3205 | 750 | 1a | - | ETR | nd | nd | nd | nd | nd | nd | nd |
| | | | | | FU | 62 | 11 | 21 | 0 | 5 | 0 | 4.92 |
| | 3212 | 750 | 1b | - | ETR | nd | nd | nd | nd | nd | nd | nd |
| | | | | | FU | 0 | 0 | 0 | 0 | 0 | 0 | 0.00 |

[a] Log change in HCV RNA from nadir to Day 14 (end of dosing). Patient 3108 did not have a Day 14 HCV RNA value, and so it was inferred using Day 11 and Day 17 values.

[b] Days after first dose of VX-950

[c] Percentages base on an average of 82 clones

[d] Sum of (%mutant at amino acid position) x (average fold change in $IC_{50}$ for amino acid position for all single and double mutants/100

[e] ETR = end of treatment (Day 14)

[f] FU = follow up (7-10 days after ETR)

[g] n/a = not available (in progress)

[h] nd = not detectable

EP 1 951 748 B1

Table 2. $IC_{50}$ Analysis of Single and Double Mutants with VX-950 and BILN 2061

| Mutation | VX950 | | BILN2061 | |
|---|---|---|---|---|
| | $IC_{50}$ [nM] | Fold change | $IC_{50}$ [nM] | Fold change |
| V36M | 110 | 1.7 | nd* | nd |
| V36M | 280 | 4.4 | 2.3 | 0.5 |
| V36M | 156 | 2.4 | 2.0 | 0.4 |
| V36L | 140 | 2.2 | 2.1 | 0.5 |
| V36A | 125 | 2.0 | nd | nd |
| V36A | 275 | 4.3 | 2.3 | 0.5 |
| V36A | 250 | 3.9 | 26 | 5.5 |
| V36A | 264 | 4.1 | nd | nd |
| V36A | 444 | 6.9 | 9.1 | 2.0 |
| | | | | |
| T54S | 120 | 1.9 | 3.6 | 0.8 |
| T54A | 749 | 12 | 10 | 2.3 |
| | | | | |
| R155K | 275 | 4.3 | 632 | 137 |
| R155K | 300 | 4.7 | nd | nd |
| R155K | 410 | 6.4 | >840 | >183 |
| R155M | 425 | 6.6 | nd | nd |
| R155S | 370 | 5.8 | >840 | >183 |
| R155T | 335 | 5.2 | nd | nd |
| R155T | 465 | 7.3 | 696 | 151 |
| R155T | 915 | 14 | 799 | 174 |
| | | | | |
| A156S | 1400 | 22 | 7.4 | 1.6 |
| | | | | |
| A156T | 21500 | 336 | >840 | >183 |
| A156T | 15000 | 234 | nd | nd |
| A156I | >50000 | >781 | nd | nd |
| A156V | >50000 | >781 | nd | nd |
| A156V | 12500 | 195 | nd | Nd |
| | | | | |
| V36A, R155K | 1350 | 21 | nd | nd |
| V36A, R155K | 1800 | 28 | nd | nd |
| V36M, R155K | 3593 | 56 | nd | nd |
| V36M, R155K | 2950 | 46 | >840 | >183 |
| V36M, R155K | 4043 | 63 | nd | nd |
| V36M, R155T | 3810 | 60 | 729 | 158 |

(continued)

| Mutation | VX950 | | BILN2061 | |
|---|---|---|---|---|
| | IC$_{50}$ [nM] | Fold change | IC$_{50}$ [nM] | Fold change |
| V36M, A156T | >50000 | >781 | nd | nd |
| * nd = not determined | | | | |

**Table 3. Mean IC$_{50}$ values for VX-950 of Different Amino Acid Mutations at the Same Position**

| Mutation | IC$_{50}$ mean [nM] | IC$_{50}$ SD [nM] | Fold change mean | Fold change SD |
|---|---|---|---|---|
| V36M/L/A | 227 | 106 | 3.5 | 1.7 |
| T54S | 120 | | 1.9 | |
| T54A | 749 | | 12 | |
| R155K/M/S/T | 437 | 204 | 6.8 | 3.2 |
| A156S | 1400 | | 22 | |
| A156T/V/I | 29800 | 18730 | 466 | 293 |
| | | | | |
| V36M/A, R155K/T | 2924 | 1116 | 46 | 17 |
| V36M, A156T | >50000 | | >781 | |

**Table 4. Fitness of Viral Mutants**

| Viral Mutation | Fitness | Fold Reduction in Fitness Relative to Wild-type | Fitness (wild-type set to 100) |
|---|---|---|---|
| None (wild-type) | 4.17 | -- | 100 |
| V36A/M | 2.82 | -1.48 | 68 |
| R155K/T + V36A/M | 2.0 | -2.09 | 48 |
| T54A | 1.86 | -2.24 | 45 |
| R155K/T | 1.58 | -2.64 | 38 |
| A156V/T | 0.1 | -41.70 | 2.4 |

**Table 5 Summary of Resistance of Mutant HCV Proteases**

| Viral Variant | VX-950 | | SCH 503034 | | BILN 2061 | | ITMN-191 | |
|---|---|---|---|---|---|---|---|---|
| | Replicon Assay[a] | Enzyme Assay[b] | Replicon Assay | Enzyme Assay | Replicon Assay | Enzyme Assay | Replicon Assay | Enzyme Assay |
| V36M | 7.0(1.6) | 5.8 | 2.7(0.9) | 4.4 | 1.4(0.9) | 0.6 | 2.1 | 1.9 |
| V36A | 7.4(2.2) | | 3.2 (0.9) | -- | 1.7(1.1) | -- | 1.5 | -- |
| V36G | 11.2 (0.4) | | 2.4(0.3) | -- | 1.1 | -- | 1.7 | -- |
| V36L | 2.2 (0.4) | 2.2 | 1.1(0.3) | -- | 1.5 | -- | 0.9 | -- |
| T54A | 6.3(1.7) | -- | 3.2(1.1) | -- | 0.9(0.2) | -- | 1.0 | -- |
| V36A+T54A | 20.1(2.9) | -- | 4.5(0.2) | -- | 0.6 (0.3) | -- | 0.5(0.1) | -- |
| R155K | 7.4(0.6) | 11 | 6.2(4.9) | 10 | 355 (213) | >300 | 62.8 | 120 |
| R155T | 19.8(1.8) | 8.7 | 10.2(2.4) | -- | 645 (173) | 72 | 9.2 | -- |
| R155S | 4.1(0.4) | 22 | 1.8(0.6) | -- | 592 (124) | >310 | 8.0 (0.2) | -- |

(continued)

| Viral Variant | VX-950 | | SCH 503034 | | BILN 2061 | | ITMN-191 | |
|---|---|---|---|---|---|---|---|---|
| | Replicon Assay[a] | Enzyme Assay[b] | Replicon Assay | Enzyme Assay | Replicon Assay | Enzyme Assay | Replicon Assay | Enzyme Assay |
| R155I | 24.0(5.2) | 16 | 6.6(2.1) | -- | 36.5 (9.5) | 56 | 1.3(0.2) | -- |
| R155M | 5.5(0.4) | -- | 2.5(0.3) | -- | 42.2(2.1) | -- | -- | -- |
| R155G | 7.4(0.5) | -- | 2.8(0.6) | -- | 821 (208) | -- | 18.7(3.0) | -- |
| V36A+R155K | ~40 | -- | 8.3(0.7) | -- | 757 (99) | -- | 316 (191) | -- |
| V36A+R155T | >62 | -- | 21.9(6.6) | -- | 835 (91) | -- | 33.0 | -- |
| V36M+R155K | ~63 | 74 | 11.3(5.5) | 39 | 791 (343) | >250 | 263 (155) | 220 |
| V36M+R155T | >62 | -- | 21.3(2.6) | -- | 1160 (110) | -- | 66.7 | - |
| V36M+A156T | >62 | - | >37 | - | 1986 (1104) | - | 12.0(2.0) | - |
| A156S | -- | 50 | -- | 37 | -- | 9.7 | | 10 |
| A156T | - | 410 | -- | 310 | -- | >310 | | 12 |
| V170A | 2.6 (0.6) | | 3.3(0.6) | | 1.8(0.3) | | ?? | |
| R109K | 0.8(0.2) | | 0.8(0.2) | 0.8(0.2) | 1.2 (0.4) | | ?? | |

## Claims

1. An isolated HCV polynucleotide encoding an HCV NS3 protease, or a fragment thereof; wherein the protease or the fragment thereof has serine protease activity; and, wherein a codon in the polynucleotide encodes M or G at the codon position that corresponds to codon position 36 of a polynucleotide sequence encoding a wild-type 181-amino acid HCV NS3 protease domain.

2. The HCV polynucleotide of claim 1, wherein the HCV polynucleotide comprises a portion of a polynucleotide sequence encoding a wild-type HCV NS3 protease; wherein said portion encodes a fragment of an HCV NS3 protease that has serine protease activity.

3. An HCV variant comprising a polynucleotide according to any of claims I or 2.

4. An isolated HCV NS3 protease comprising an amino acid sequence in which the amino acid at the position corresponding to amino acid position 36 of a wild-type 181-amino acid HCV NS3 protease domain is an M or G.

5. The HCV protease of claim 4, wherein the HCV NS3 protease comprises a portion of a wild-type HCV NS3 protease amino acid sequence; wherein said portion encodes a fragment of the HCV NS3 protease that has serine protease activity.

6. The HCV protease of claim 4 comprising an NS4A cofactor.

7. An anti-HCV protease antibody that is specific to an HCV NS3 protease encoded by the polynucleotide of claim 1.

8. An expression system comprising the HCV polynucleotide of any one of claims 1 or 2.

9. The expression system of claim 8 comprising a vector, wherein the vector comprises the HCV polynucleotide of any one of claims 1 or 2 operably linked to a promoter.

10. A host cell transfected, transformed, or transduced with the vector of claim 9.

11. The expression system of claim 8 that is an mRNA display system.

12. A method for identifying a candidate compound for treating an HCV infection in a patient comprising:

(a) providing a sample infected with an HCV variant; wherein the HCV variant comprises a nucleic acid encoding an HCV NS3 protease that comprises the polynucleotide of claim 1;
(b) assaying the ability of the candidate compound in inhibiting an activity of the HCV variant in the sample.

13. The method of claim 12, wherein the activity of the HCV variant is replication.

14. An *in vitro* method for identifying a candidate compound for treating or preventing an HCV infection in a patient comprising:

(a) providing a replicon RNA comprising the polynucleotide of claim 1;
(b) determining whether the candidate compound inhibits replication of the replicon RNA of (a).

15. An in vitro method for identifying a candidate compound for treating an HCV infection in a patient comprising:

(a) providing an HCV NS3 protease and a protease substrate, wherein the protease is encoded by the polynucleotide of claim 1, and wherein the protease and the substrate are in a cell-based system or in a cell-free system;
(b) contacting the HCV NS3 protease with the candidate compound in the presence of the substrate; and
(c) determining whether the HCV NS3 protease activity is reduced.

16. A method for evaluating a candidate compound for treating an HCV infection in a patient comprising:

(a) introducing a vector comprising a polynucleotide encoding an HCV NS3 protease, or a fragment thereof; wherein the protease or the fragment thereof has serine protease activity; wherein the protease or fragment thereof is encoded by the polynucleotide of claim 1, and an indicator gene encoding an indicator in vitro into a host cell;
(b) culturing the host cell; and
(c) measuring the indicator in the presence of the candidate compound and in the absence of the candidate compound.

17. An *in vitro* method for identifying a compound able to rescue the activity of VX-950 against an HCV NS3 protease that has become resistant to VX-950 comprising:

(a) contacting the HCV NS3 protease with a candidate compound; wherein the protease is encoded by the polynucleotide of claim 1;
(b) assaying the ability of VX-950 to inhibit the activity of the protease of (a).

18. A method for identifying a compound effective in reducing an HCV NS3 protease activity, comprising:

(a) obtaining a three dimensional model of an HCV NS3 protease encoded by the polynucleotide of claim 1;
(b) designing or selecting a compound;
(c) evaluating the ability of the compound to bind to or interact with the protease of (a).

19. The method of claim 18 further comprising evaluating the ability of a designed or selected compound to inhibit an HCV NS3 protease activity in a cell-free or cell-based assay or assaying the ability of a designed or selected compound to inhibit HCV replication in an infected cell in vitro or in a sample.

20. A method for detecting the presence of an HCV variant in a biological sample comprising detecting the presence of a polynucleotide in the biological sample, wherein said polynucleotide encodes an HCV NS3 protease, or a fragment thereof; wherein the protease or the fragment thereof has serine protease activity; and, wherein the protease or fragment thereof is encoded by the polynucleotide of claim 1.

21. A diagnostic kit comprising the antibody of claim 7.

22. The method of any one of claims 12 and 14, wherein the HCV polynucleotide comprises a portion of a polynucleotide sequence encoding a wild-type HCV NS3 protease; wherein the portion encodes a fragment of an HCV NS3 protease that has serine protease activity.

23. The method of any one of claims 15 to 18, 19 and 20, wherein the wild-type HCV NS3 protease comprises a portion of a wild-type HCV NS3 protease amino acid sequence; wherein the portion encodes a fragment of the HCV NS3 protease that has serine protease activity.

**Patentansprüche**

1. Isoliertes HCV-Polynucleotid, das eine HCV-NS3-Protease oder ein Fragment davon codiert; wobei die Protease oder das Fragment davon Serinprotease-Aktivität hat; und wobei ein Codon im Polynucleotid M oder G an der Codonposition codiert, die der Codonposition 36 einer Polynucleotidsequenz entspricht, die eine 181-Aminosäuren-Domäne der HCV-NS3-Protease vom Wildtyp codiert.

2. HCV-Polynucleotid nach Anspruch 1, wobei das HCV-Polynucleotid einen Teil einer Polynucleotidsequenz umfasst, die eine HCV-NS3-Protease vom Wildtyp codiert; wobei der Teil ein Fragment einer HCV-NS3-Protease codiert, das Serinprotease-Aktivität hat.

3. HCV-Variante, umfassend ein Polynucleotid nach einem der Ansprüche 1 oder 2.

4. Isolierte HCV-NS3-Protease, umfassend eine Aminosäuresequenz, in der die Aminosäure an der Position, die der Aminosäureposition 36 einer 181-Aminosäuren-Domäne der HCV-NS3-Protease vom Wildtyp entspricht, ein M oder G ist.

5. HCV-Protease nach Anspruch 4, wobei die HCV-NS3-Protease einen Teil einer Aminosäuresequenz einer HCV-NS3-Protease vom Wildtyp umfasst, wobei der Teil ein Fragment der HCV-NS3-Protease codiert, das Serinprotease-Aktivität hat.

6. HCV-Protease nach Anspruch 4, umfassend einen NS4A-Cofaktor.

7. Anti-HCV-Protease-Antikörper, der für eine HCV-NS3-Protease spezifisch ist, die von dem Polynucleotid nach Anspruch 1 codiert wird.

8. Expressionssystem, umfassend das HCV-Polynucleotid nach einem der Ansprüche 1 oder 2.

9. Expressionssystem nach Anspruch 8, das einen Vektor umfasst, wobei der Vektor das HCV-Polynucleotid nach einem der Ansprüche 1 oder 2 funktionell mit einem Promotor verknüpft umfasst.

10. Wirtszelle, die mit dem Vektor nach Anspruch 9, transfiziert, transformiert oder transduziert ist.

11. Expressionssystem nach Anspruch 8, das ein mRNA-Display-System ist.

12. Verfahren zum Identifizieren einer Kandidatenverbindung für die Behandlung einer HCV-Infektion bei einem Patienten, umfassend:

    (a) Bereitstellen einer Probe, die mit einer HCV-Variante infiziert ist, wobei die HCV-Variante eine Nucleinsäure umfasst, die eine HCV-NS3-Protease codiert, die das Polynucleotid nach Anspruch 1 umfasst;
    (b) Testen der Fähigkeit der Kandidatenverbindung, eine Aktivität der HCV-Variante in der Probe zu inhibieren.

13. Verfahren nach Anspruch 12, wobei die Aktivität der HCV-Variante Replikation ist.

14. In vitro-Verfahren zum Identifizieren einer Kandidatenverbindung für die Behandlung oder Prävention einer HCV-Infektion bei einem Patienten umfassend:

    (a) Bereitstellen einer Replikon-RNA, umfassend das Polynucleotid nach Anspruch 1;
    (b) Bestimmen, ob die Kandidatenverbindung die Replikation der Replikon-RNA aus (a) inhibiert.

15. *In vitro*-Verfahren zum Identifizieren einer Kandidatenverbindung für die Behandlung einer HCV-Infektion bei einem Patienten, umfassend:

    (a) Bereitstellen einer HCV-NS3-Protease und eines Proteasesubstrats, wobei die Protease von dem Polynucleotid nach Anspruch 1 codiert wird, und wobei die Protease und das Substrat sich in einem zellbasierten oder in einem zellfreien System befinden;

(b) Inkontaktbringen der HCV-S3-Protease mit der Kandidatenverbindung in Anwesenheit des Substrats; und

(c) Bestimmen, ob die HCV-NS3-Protease-Aktivität verringert ist.

16. Verfahren zum Evaluieren einer Kandidatenverbindung für die Behandlung einer HCV-Infektion bei einem Patienten, umfassend:

(a) Einbringen eines Vektors, der ein Polynucleotid, das eine HCV-NS3-Protease oder ein Fragment davon codiert; wobei die Protease oder das Fragment davon Serinprotease-Aktivität hat; wobei die Protease oder das Fragment davon von dem Polynucleotid nach Anspruch 1 codiert wird, und ein Indikatorgen umfasst, das einen Indikator codiert, *in vitro* in eine Wirtszelle;

(b) Züchten der Wirtszelle; und

(c) Messen des Indikators in Anwesenheit der Kandidatenverbindung und in Abwesenheit der Kandidatenverbindung.

17. *In vitro*-Verfahren zum Identifizieren einer Verbindung, die fähig ist, die Aktivität von VX-950 gegen eine HCV-NS3-Protease, das gegen VX-950 resistent geworden ist, wiederherzustellen, umfassend:

(a) Inkontaktbringen der HCV-NS3-Protease mit einer Kandidatenverbindung; wobei die Protease von dem Poylnucleotid nach Anspruch 1 codiert wird;

(b) Testen der Fähigkeit von VX-950, die Aktivität der Protease aus (a) zu inhibieren.

18. Verfahren zum Identifizieren einer Verbindung, die wirksam ist, eine HCV-NS3-Protease-Aktivität zu verringern, umfassend:

(a) Erhalt eines dreidimensionalen Modells einer HCV-NS3-Protease, die von dem Polynucleotid nach Anspruch 1 codiert wird;

(b) Entwickeln oder Auswählen einer Verbindung;

(c) Evaluieren der Fähigkeit der Verbindung, an die Protease aus (a) zu binden oder mit dieser zu interagieren.

19. Verfahren nach Anspruch 18, des Weiteren umfassend das Evaluieren der Fähigkeit einer entwickelten oder ausgewählten Verbindung, eine HCV-NS3-Proteaseaktivität in einem zellfreien oder zellbasierten Test zu inhibieren oder Testen der Fähigkeit einer entwickelten oder ausgewählten Verbindung, die HCV-Replikation in einer infizierten Zelle *in vitro* oder in einer Probe zu inhibieren.

20. Verfahren zum Nachweis der Anwesenheit einer HCV-Variante in einer biologischen Probe, umfassend den Nachweis der Anwesenheit eines Polynucleotids in der biologischen Probe, wobei das Polynucleotid eine HCV-NS3-Protease oder ein Fragment davon codiert; wobei die Protease oder das Fragment davon Serinprotease-Aktivität hat; und wobei die Protease oder das Fragment davon von dem Polynucleotid nach Anspruch 1 codiert wird.

21. Diagnosekit, umfassend den Antikörper nach Anspruch 7.

22. Verfahren nach einem der Ansprüche 12 und 14, wobei das HCV-Polynucleotid einen Teil einer Polynucleotidsequenz umfasst, die eine HCV-NS3-Protease vom Wildtyp codiert, wobei der Teil ein Fragment einer HCV-NS3-Protease codiert, das Serinprotease-Aktivität hat.

23. Verfahren nach einem der Ansprüche 15 bis 18, 19 und 20, wobei die HCV-NS3-Protease vom Wildtyp einen Teil einer Aminosäuresequenz einer HCV-NS3-Protease vom Wildtyp umfasst; wobei der Teil ein Fragment der HCV-NS3-Protease codiert, das Serinprotease-Aktivität hat.

**Revendications**

1. Polynucléotide de VHC isolé codant pour une protéase NS3 de VHC, ou un fragment de celle-ci; la protéase ou le fragment de celle-ci ayant une activité sérine protéase ; et, un codon dans le polynucléotide codant pour M ou G à la position de codon qui correspond à la position de codon 36 d'une séquence polynucléotidique codant pour un domaine de protéase NS3 de VHC de type sauvage de 181 acides aminés.

**2.** Polynucléotide de VHC de la revendication 1, le polynucléotide de VHC comprenant une partie d'une séquence polynucléotidique codant pour une protéase NS3 de VHC de type sauvage ; ladite partie codant pour un fragment d'une protéase NS3 de VHC qui a une activité sérine protéase.

**3.** Variant de VHC comprenant un polynucléotide selon l'une quelconque des revendications 1 ou 2.

**4.** Protéase NS3 de VHC isolée comprenant une séquence d'acides aminés dans laquelle l'acide aminé à la position correspondant à la position d'acide aminé 36 d'un domaine de protéase NS3 de VHC de type sauvage de 181 acides aminés est M ou G.

**5.** Protéase de VHC de la revendication 4, la protéase NS3 de VHC comprenant une partie d'une séquence d'acides aminés de protéase NS3 de VHC de type sauvage ; ladite partie codant pour un fragment de la protéase NS3 de VHC qui a une activité sérine protéase.

**6.** Protéase de VHC de la revendication 4 comprenant un cofacteur NS4A.

**7.** Anticorps anti-protéase de VHC qui est spécifique pour une protéase NS3 de VHC codée par le polynucléotide de la revendication 1.

**8.** Système d'expression comprenant le polynucléotide de VHC de l'une quelconque des revendications 1 ou 2.

**9.** Système d'expression de la revendication 8 comprenant un vecteur, le vecteur comprenant le polynucléotide de VHC de l'une quelconque des revendications 1 ou 2 fonctionnellement lié à un promoteur.

**10.** Cellule hôte transfectée, transformée, ou transduite avec le vecteur de la revendication 9.

**11.** Système d'expression de la revendication 8 qui est un système de présentation d'ARNm.

**12.** Procédé pour identifier un composé candidat pour traiter une infection par le VHC chez un patient comprenant :

(a) la production d'un échantillon infecté par un variant de VHC ; le variant de VHC comprenant un acide nucléique codant pour une protéase NS3 de VHC qui comprend le polynucléotide de la revendication 1 ;
(b) l'évaluation de la capacité du composé candidat à inhiber une activité du variant de VHC dans l'échantillon.

**13.** Procédé de la revendication 12, dans lequel l'activité du variant de VHC est la réplication.

**14.** Procédé *in vitro* pour identifier un composé candidat pour traiter ou prévenir une infection par le VHC chez un patient comprenant :

(a) la fourniture d'un ARN de réplicon comprenant le polynucléotide de la revendication 1 ;
(b) la détermination que le composé candidat inhibe ou non la réplication de l'ARN de réplicon de (a).

**15.** Procédé *in vitro* pour identifier un composé candidat pour traiter une infection par le VHC chez un patient comprenant :

(a) la fourniture d'une protéase NS3 de VHC et d'un substrat de protéase, la protéase étant codée par le polynucléotide de la revendication 1, et la protéase et le substrat étant dans un système à base de cellules ou dans un système sans cellules ;
(b) la mise en contact de la protéase NS3 de VHC avec le composé candidat en présence du substrat ; et
(c) la détermination que l'activité de protéase NS3 de VHC est réduite ou non.

**16.** Procédé pour évaluer un composé candidat pour traiter une infection par le VHC chez un patient comprenant :

(a) l'introduction d'un vecteur comprenant un polynucléotide codant pour une protéase NS3 de VHC, ou un fragment de celle-ci ; la protéase ou le fragment de celle-ci ayant une activité sérine protéase ; la protéase ou le fragment de celle-ci étant codé par le polynucléotide de la revendication 1, et d'un gène indicateur codant pour un indicateur *in vitro* dans une cellule hôte ;
(b) la culture de la cellule hôte ; et
(c) la mesure de l'indicateur en présence du composé candidat et en l'absence du composé candidat.

**17.** Procédé *in vitro* pour identifier un composé capable de restaurer l'activité de VX-950 contre une protéase NS3 de VHC qui est devenue résistante à VX-950 comprenant :

(a) la mise en contact de la protéase NS3 de VHC avec un composé candidat ; la protéase étant codée par le polynucléotide de la revendication 1 ;
(b) l'évaluation de la capacité de VX-950 à inhiber l'activité de la protéase de (a).

**18.** Procédé pour identifier un composé efficace pour réduire une activité de protéase NS3 de VHC, comprenant :

(a) l'obtention d'un modèle tridimensionnel d'une protéase NS3 de VHC codée par le polynucléotide de la revendication 1 ;
(b) la conception ou la sélection d'un composé ;
(c) l'évaluation de la capacité du composé à se lier ou interagir avec la protéase de (a).

**19.** Procédé de la revendication 18 comprenant en outre l'évaluation de la capacité d'un composé conçu ou sélectionné à inhiber une activité de protéase NS3 de VHC dans un essai sans cellules ou à base de cellules ou l'évaluation de la capacité d'un composé conçu ou sélectionné à inhiber la réplication de VHC dans une cellule infectée *in vitro* ou dans un échantillon.

**20.** Procédé pour détecter la présence d'un variant de VHC dans un échantillon biologique comprenant la détection de la présence d'un polynucléotide dans l'échantillon biologique, ledit polynucléotide codant pour une protéase NS3 de VHC, ou un fragment de celle-ci ; la protéase ou le fragment de celle-ci ayant une activité sérine protéase ; et, la protéase ou le fragment de celle-ci étant codé par le polynucléotide de la revendication 1.

**21.** Trousse de diagnostic comprenant l'anticorps de la revendication 7.

**22.** Procédé de l'une quelconque des revendications 12 et 14, le polynucléotide de VHC comprenant une partie d'une séquence polynucléotidique codant pour une protéase NS3 de VHC de type sauvage ; la partie codant pour un fragment d'une protéase NS3 de VHC qui a une activité sérine protéase.

**23.** Procédé de l'une quelconque des revendications 15 to 18, 19 et 20, la protéase NS3 de VHC de type sauvage comprenant une partie d'une séquence d'acides aminés de protéase NS3 de VHC de type sauvage ; la partie codant pour un fragment de la protéase NS3 de VHC qui a une activité sérine protéase.

**Figure 1: Phylogenetic Analysis of Baseline Sequences.** Phylogenetic tree of the N-terminal 543 nucleotides of the NS3 protein (protease domain) from untreated genotype 1 HCV-infected subjects. Sequences cluster into subtypes 1a and 1b. The tree is rooted with the reference lab strain HCV-H as an outgroup (accession number M67463).

EP 1 951 748 B1

**Figure 2: Baseline IC$_{50}$s for Genotype 1a and 1b.** Protease variants from untreated subjects were expressed, purified, and tested for sensitivity to VX-950. The enzyme IC$_{50}$ of VX-950 against each protease is shown (values are the mean of 2 independent experiments). Results are grouped by genotype 1a and genotype 1b. The line indicates the mean of all data points. Included for comparison are K$_i$ values of previously published wild-type proteases and protease mutants known to confer resistance to VX-950 (A156V/T/S).

EP 1 951 748 B1

| Subject Group | # of Subj | Definition |
|---|---|---|
| Placebo ■ | 6 | No VL change |
| Rebound ◆ | 13 | > 0.75 log VL change from nadir to day 14 |
| Plateau ● | 8 | < 0.75 log VL change from nadir to day 14 |
| Continued Response ▲ | 7 | Continued decline throughout 14 days of VX-950 dosing |

**Figure 3: Subject Groups by Viral Response.** Subject groups are divided by HCV RNA response to 14 days of dosing with VX-950 based on mutational patterns. HCV RNA values shown are the mean values for each group of subjects.

**Figure 4: Summary of Viral Response and Mutation Patterns.** Pie charts show the average percent of mutations at a given amino acid position for all subjects within a given response group.

| | Placebo | Rebound[a] | | Plateau | Continued Response |
|---|---|---|---|---|---|
| | | 1a | 1b | | |
| **End Of Treatment** | | | | | Not detectable |
| **Follow Up** | | | | | |

[a] This rebound group is split by subtype to depict the fact that mutations at position 155 are only seen in 1a patients. All mutations at position 155 in plateau and continued response groups are found only in the genotype 1a patients of that group.

V36    WT    A156
T54    R155    36/155

EP 1 951 748 B1

Figure 5: Enzymatic IC50 and Fold Change from Reference 1a Strain HCV-H of Protease Single Resistance Mutants to VX-950. Protease mutants selected in subjects dosed with VX-950 were expressed, purified and tested for sensitivity to VX-950. Values are the mean of 2 independent experiments for a given protease mutant sequence. The IC50 of VX-950 against each protease is shown (A) and compared to the reference strain HCV-H (B).

EP 1 951 748 B1

**Figure 6: Enzymatic IC50 and Fold Change from Reference 1a Strain HCV-H of Protease Double Resistant Mutants to VX-950.** Protease mutants selected in subjects dosed with VX-950 were expressed, purified and tested for sensitivity to VX-950. Values are the mean of 2 independent experiments for a given protease mutant sequence. The IC50 of VX-950 against each protease is shown (A) and compared to the reference strain HCV-H (B).

EP 1 951 748 B1

EP 1 951 748 B1

Figure 7: Inverse Correlation Between Resistance and Fitness

VX-950 (MW. 680)    BILN 2061 (MW. 775)

FIGURE 4   8

# Location of Telaprevir-resistant Mutations in the HCV Protease

EP 1 951 748 B1

Figure 9

# Methods for Phenotypic Analyses of Variants

## Phenotypic analyses

### Replicon analysis

| Sub-genomic replicon |
| Genotype 1b (Con 1) |
| ↓ |
| 48-h $IC_{50}$ of telaprevir |

### Enzymatic analysis

| NS3 protease domain + 4A peptide |
| Genotype 1a |
| ↓ |
| $K_i$ of telaprevir |

### Structural analysis

| NS3 protease domain + 4A peptide |
| Genotype 1a (HCV-H) |
| ↓ |
| X-ray structure Computational modeling |

Figure 10

VERTE

EP 1 951 748 B1

# V36 Substitutions Confer Low-level Resistance to Telaprevir

Replicon IC$_{50}$

Enzyme K$_i$

Figure 11

EP 1 951 748 B1

# X-ray Structure of the V36M Variant Protease

**Wild-type protease**    **V36M variant protease**

M36

V36

Figure 12

<u>V36M protease</u>

no difference from wild-type protease

- except for residues 36 and 43

EP 1 951 748 B1

# V36 Does not Make Direct Contact with Telaprevir (VX-950)

- V36 does not make direct contact with telaprevir (VX-950)

- V36 is located next to the NS4A cofactor ß-strand

- V36 (and T54) make hydrophobic contacts with F43

- F43, in turn, interacts with the P1' group of telaprevir (VX-950)

Figure 13

EP 1 951 748 B1

# V36M Variant in G1a: Low-level Resistance, More Fi

- **Phenotype**
  - **6-7 fold resistance to telaprevir**
  - Average % of HCV RNA at day 14 in patients dosed with telaprevir alone
    - 43% in G1a (1-nt change)
    - 0% in G1b (presumably due to 2-nt change)

- **Mechanism**
  - Loss of hydrophobic interaction with F43 ➡ increased flexibility of F43 and P1' pocket
  - Loss of interaction with telaprevir P1' group
  - May gain additional interaction within the protease

Figure 14

VERTE

EP 1 951 748 B1

# V36A Variant in G1a/b: Low-level Resistance, Less F

- **Phenotype**
  - Similar resistance as V36M
  - Average % of HCV RNA at day 14 in patients dosed with telaprevir alone
    - <10% in G1a (1-nt change)
    - 15% in G1b (1-nt change)
  - Less fit than V36M

- **Mechanism**
  - Loss of hydrophobic interaction with F43 ➡ increased flexibility of F43 and P1' pocket
  - Loss of interaction between F43 and telaprevir P1' group
  - Does NOT gain additional interaction with protease

Figure 15

EP 1 951 748 B1

VERTE

# V36G Variant in G1b: Low-level Resistance, Less Fit

- Phenotype
  - Similar resistance as V36M/A
  - Average % of HCV RNA at day 14 in patients dosed with telaprevir alone
    - 0% in G1a (1-nt change)
    - 14% in G1b (1-nt change)
  - Less fit than V36M

- Mechanism
  - Loss of hydrophobic interaction with F43 ➡ increased flexibility of F43 and P1' pocket
  - Loss of interaction between F43 and telaprevir P1' group
  - Does NOT gain additional interaction with protease

Figure 16

VERTE

EP 1 951 748 B1

# V36L Variant: No Resistance, Rare in G1 (Polymorphism)

- **Phenotype**
  - No resistance
  - Average % of HCV RNA at day 14 in patients dosed with telaprevir alone
    - 0.7% in G1a (polymorphism)
    - 0% in G1b
  - L36 exists in G2, G3, or G4 HCV NS3 protease

- **Mechanism**
  - Little **loss of hydrophobic interaction with F43**
  - Little **loss of interaction** between F43 and telaprevir P1' group

Figure 17

EP 1 951 748 B1

# Methods for Phenotypic Analyses of Variants

## Phenotypic analyses

Replicon analysis     Enzymatic analysis     Structural analysis

| Genotype 1b | Genotype 1a | Genotype 1a |
|---|---|---|
| ↓ | ↓ | ↓ |
| Inhibition of HCV replication by telaprevir | Inhibition of HCV protease by telaprevir | Binding of telaprevir to HCV protease |

Definition of resistance (in fold change):
| | |
|---|---|
| < 3-fold | (no resistance) |
| 3- to 25-fold | (low-level resistance) |
| 25- to 50-fold | (intermediate-level resistance) |
| >50-fold | (high-level resistance) |

Figure 18

VERTEX

# R155 Substitutions Confer Low-level Resistance to Telaprevir

## Replicon $IC_{50}$

## Enzyme $K_i$

Figure 19

EP 1 951 748 B1

# X-ray Structure of the R155K Variant Protease

Wild-type protease

R155K variant protease

K155

R155

### R155K protease

no difference from wild-type protease

- except for residue 155 itself

VERTEX

Figure 20

# Computer Model of Telaprevir Binding to the R155K Variant Protease

## R155K protease

- Conformation of bound telaprevir remains virtually unchanged

- Loss of some interactions with telaprevir P2 group

- Similar models for other R155 variant proteases

Figure 21

EP 1 951 748 B1

# V36 or T54 Substitutions Confer Low-level Resistance to Telaprevir

## Replicon $IC_{50}$

## Enzyme $K_i$

Figure 22

EP 1 951 748 B1

# Computer Model of Telaprevir Binding to the V36M Variant Protease

Figure 23

## V36M protease

- Conformation of bound telaprevir remains virtually unchanged

- Loss of interactions with telaprevir P1' group

- Similar models for other V36 variant proteases

VERTEX

# V36M and R155K Substitutions Are Additive in Conferring Resistance to Telaprevir

Figure 24

EP 1 951 748 B1

Figure 25

Figure 26

# Telaprevir-resistant Replicon Variants Remain Fully Sensitive to IFN-alpha

IFN-alpha

Fold change in Replicon $IC_{50}$

| WT | V36M | V36A | T54A | R155K | R155T | R155M | V36M+ R155K | V36M+ R155T | V36A+ R155K | V36A+ R155T |
|----|------|------|------|-------|-------|-------|-------------|-------------|-------------|-------------|
| 1.0 | 1.0 | 0.9 | 0.3 | 1.3 | 0.4 | 0.4 | 0.9 | 0.3 | 0.9 | 0.3 |

Figure 27

EP 1 951 748 B1

# Telaprevir-resistant Replicon Variants Remain Fully Sensitive to Ribavirin

Figure 28

EP 1 951 748 B1

# Telaprevir Combination Therapy Suppressed Emergence of Viral Resistance and Prevented Viral Break-through during Dosing

**Phase 1b:** Telaprevir + PEG-IFN

**Phase 2a:** Telaprevir + PEG-IFN/RBV

At Day 14 (telaprevir + PEG-IFN):
- 4/8 patients undetectable (<10 IU/mL)

At Day 28 (telaprevir + PEG-IFN/RBV):
- All 12 patients undetectable (<10 IU/mL)

No subjects had viral breakthrough during dosing of telaprevir in combination with PEG-IFN +/- RBV

Figure 29

EP 1 951 748 B1

# HCV Sequence Diversity and Resistance Mutations

- Sequence changes (mutations) in the HCV genome occur spontaneously and frequently as a natural result of viral replication
  - HCV polymerase has a high error rate (~1 error per genome)
  - High rate of HCV replication (~$10^{12}$ virions produced per day)
- Therefore, variants with mutations conferring resistance to a specifically targeted antiviral most likely exist at a low frequency prior to drug treatment
- Strong inhibition of wild-type virus with a specifically-targeted antiviral drug selects for drug-resistant virus
- NS3 protease mutations observed in Ph 1b trial of telaprevir alone
  - V36M/A/G
  - T54A
  - R155K/T/M/S/I/G
  - A156S/V/T (observed previously in replicon-based in vitro studies)
  - V36M/A+R155K/T

Figure 30

VERTEX

EP 1 951 748 B1

# Mechanisms of Resistance to HCV Protease Inhibitors

- ## In the active site

  - A156S/V/T (Lin 2004, Lu 2004, & Lin 2005)
    - Steric hindrance (direct) to inhibitor binding
  - D168A/V (Trozzi 2003, Lin 2004 & Lu 2004)
    - Steric hindrance (indirect) to inhibitor binding
  - R155K/T/S/I/M/G (Lin AASLD 2006)
    - Loss of contact (direct) to inhibitor

- ## Outside the active site

  - V36M/A/G   ??
  - T54A

Figure 31

VERTE

# Conclusions

- **Mechanism of resistance to HCV protease inhibitors**

  - A156S/V/T: Steric hindrance (direct) to telaprevir binding

  - D168A/V: Steric hindrance (indirect) to BILN 2061 binding

  - R155K/T: Loss of contact (direct) to telaprevir

  - **V36M/A/G: Loss of contact (indirect) to telaprevir**

    - **V36M:** Low-level resistance, more fit than V36A/G

    - **V36A/G:** Low-level resistance, less fit than V36M

    - **V36L:** No resistance (polymorphism)

Figure 32

VERTE

EP 1 951 748 B1

# Summary

- Telaprevir-resistant variants can be classified into two groups:
  - Low-level resistance (<25–fold): V36, T54, R155, A156S
  - High-level resistance (>50–fold): A156V/T or V36+R155

- Mechanism of resistance to telaprevir:
  - Loss of interaction to the P1' group (V36)
  - Loss of interaction to the P2 group (R155)

- Telaprevir-resistant variants remain sensitive to IFN-alpha and ribavirin in replicon cells

- No subjects had viral breakthrough during dosing of telaprevir in combination with PEG-IFN +/- ribavirin

Figure 33

VERTEX

**EP 1 951 748 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6653127 B
- US 6211338 B
- WO 9203918 A
- US 6258558 B
- US 6361943 B
- US 5843701 A
- US 20050136400 A
- US 20030162169 A
- WO 9811134 A
- EP 404097 A
- WO 9311161 A
- US 20040214994 A
- US 6924270 B
- US 6849254 B
- WO 0059929 A
- US 6608027 B
- WO 04039970 A
- US 9743310 W
- US 20020016294 A
- WO 0181325 A
- WO 0208198 A
- WO 0177113 A
- WO 0208187 A
- WO 0208256 A
- WO 0208244 A
- WO 03006490 A
- WO O174768 A
- WO 9950230 A
- WO 9817679 A
- WO 0248157 A
- US 20020177725 A
- WO 02060926 A
- US 20030008828 A
- WO 0248116 A
- WO 0164678 A
- WO 0107407 A
- WO 9846630 A
- WO 9907733 A
- WO 0009588 A
- US 20020016442 A
- WO 0009543 A
- WO 9907734 A
- US 20020032175 A
- US 20050080017 A
- WO 9822496 A
- WO 02079234 A
- WO 0031129 A
- WO 9938888 A
- WO 9964442 A

- WO 2004072243 A
- WO 0218369 A
- US 6018020 A
- US 6265380 A
- US 6608027 A
- US 5866684 A
- US 20050249702 A
- US 20050153900 A
- US 20050245458 A
- US 20050222047 A
- US 20050209164 A
- US 20050197301 A
- US 20050176648 A
- US 20050164921 A
- US 20050119168 A
- US 20050085425 A
- US 20050059606 A
- US 20030207861 A
- US 20020147139 A
- US 20050143439 A
- US 20050107304 A
- US 20050090450 A
- US 20040147483 A
- US 20040142876 A
- US 20040077600 A
- US 20040018986 A
- US 20030236242 A
- US 20030216325 A
- US 6962932 B
- US 6914122 B
- US 6911428 B
- US 6846802 B
- US 6838475 B
- US 5807876 A
- US 6498178 B
- US 6344465 B
- US 6054472 A
- WO 9740028 A
- WO 9840381 A
- WO 0056331 A
- US 6541496 B
- US 4835168 A
- US 6127422 A
- WO 9414436 A
- US 6037157 A
- US 5484801 A
- US 402690 A
- WO 9507696 A
- WO 9509614 A

**Non-patent literature cited in the description**

- **PAWLOTSKY, J. M. ; J. G. MCHUTCHISON.** Development of new drugs and clinical trials: promises and pitfalls. *Hepatitis C,* 2004
- Summary of an AASLD hepatitis single topic conference. *Hepatology,* 27 February 2003, vol. 39, 554-67
- **STRADER et al.** Diagnosis, management, and treatment of hepatitis C. *Hepatology,* 2004, vol. 39, 1147-71
- **SIMMONDS, P.** Genetic diversity and evolution of hepatitis C virus - 15 years on. *J. Gen. Virol.,* 2004, vol. 85, 3173-88
- **JOHNSON et al.** *Top. HIV Med.,* 2004, vol. 12, 119-24
- **LIN et al.** In vitro studies of cross-resistance mutations against two hepatitis C virus serine protease inhibitors, VX-950 and BILN 2061. *J. Biol. Chem.,* vol. 280, 36784-36791
- **LIN et al.** In vitro resistance studies of hepatitis C virus serine protease inhibitors, VX-950 and BILN 2061: Structural analysis indicates different resistance mechanisms. *J. Biol. Chem.,* 2004, vol. 279, 17508-17514
- **LU et al.** *Antimicrob. Agents Chemother,* vol. 48, 2260-6
- **TROZZI et al.** In vitro selection and characterization of hepatitis C virus serine protease variants resistant to an active-site peptide inhibitor. *J. Virol.,* 2003, vol. 77, 3669-79
- **BARTENSCHLAGER et al.** *J. Virology,* 1994, vol. 68, 5045-55
- **FAILLA et al.** *J. Virology,* 1995, vol. 69, 1769-77
- **ZAPATA et al.** *Protein Eng.,* 1995, vol. 8 (10), 1057-1062
- **PLUCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **M. LLINAS-BRUNET et al.** *Bioorg. Med. Chem. Lett.,* 1998, vol. 8, 1713-18
- **W. HAN et al.** *Bioorg. Med. Chem. Lett.,* 2000, vol. 10, 711-13
- **R. DUNSDON et al.** *Bioorg. Med. Chem. Lett.,* 2000, vol. 10, 1571-79
- **M. LLINAS-BRUNET et al.** *Bioorg. Med. Chem. Lett.,* 2000, vol. 10, 2267-70
- **S. LAPLANTE et al.** *Bioorg. Med. Chem. Lett.,* 2000, vol. 10, 2271-74
- **W. MARKLAND et al.** *Antimicrobial & Antiviral Chemotherapy,* 2000, vol. 44, 859
- **REDDY, K. R. et al.** Effcacy and Safety of Pegylated (40-kd) interferon alpha-2a compared with interferon alpha-2a in noncirrhotic patients with chronic hepatitis C. *Hepatology,* 2001, vol. 33, 433-438
- **KAO, J. H. et al.** efficacy of Consensus Interferon in the Treatment of Chronic Hepatitis. *J. Gastroenterol. Hepatol.,* 2000, vol. 15, 1418-1423
- **CLAYETTE, P. et al.** IFN-tau, A New Interferon Type I with Antiretroviral activity. *Pathol. Biol. (Paris,* 1999, vol. 47, 553-559
- **DAVIS, G.L. et al.** Future Options for the Management of Hepatitis C. *Seminars in Liver Disease,* 1999, vol. 19, 103-112
- **TAZULAKHOVA, E. B. et al.** Russian Experience in Screening, analysis, and Clinical Application of Novel Interferon Inducers. *J. Interferon Cytokine Res.,* vol. 21, 65-73
- **SAUDER, D. N.** Immunomodulatory and Pharmacologic Properties of Imiquimod. *J. Am. Acad. Dermatol.,* 2000, vol. 43, S6-11 1
- **TAZULAKHOVA et al.** *J. Interferon Cytokine Res.,* vol. 21, 65-73
- **SAUDER.** *J. Am. Arad. Dermatol.,* 2000, vol. 43, 6-11
- **YOUNOSSI et al.** *Seminars in Liver Disease,* 1999, vol. 19, 95-102
- **COLACINO et al.** *Antimicrobial Agents & Chemotherapy,* 1990, vol. 34, 2156-2163
- **F. G. HAYDEN et al.** Goodman & Gilman's The Pharmacological Basis of Therapeutics. McGraw-Hill, 1996, 1191-1223
- **BENET et al.** Goodman & Gilman's The Pharmacological Basis of Therapeutics. McGraw-Hill, 1996, 3-27
- *Acta Biochim Pol.,* 1996, vol. 43, 25-36
- *Swed. Nucleosides Nucleotides,* 1996, vol. 15, 361-378
- *Synthesis,* 1995, vol. 12, 1465-1479
- *Carbohyd. Chem.,* 1995, vol. 27, 242-276
- *Chena Nucleosides Nucleotides,* 1994, vol. 3, 421-535
- Ann. Reports in Med. Chena. Academic Press
- *Exp. Opin. Invest. Drugs,* 1995, vol. 4, 95-115
- **FELSENSTEIN, J.** *PHYLIP,* 1993
- **KIM et al.** *Cell,* 1996, vol. 87 (2), 343-355
- **PERNI et al.** *Bioorg. Med. Chem. Lett.,* 2004, vol. 14 (6), 1441-1446
- **DI MARCO et al.** *J. Biol. Chem.,* 2000, vol. 275 (10), 7152-7157
- **BRENNER, B. G. ; D. TURNER ; M. A. WAINBERG.** HIV-1 drug resistance: can we overcome?. *Expert Opin. Biol. Ther.,* 2002, vol. 2, 751-61
- **BUCKHEIT, R. W., JR.** Understanding HIV resistance, fitness, replication capacity and compensation: targeting viral fitness as a therapeutic strategy. *Expert Opin. Investig. Drugs,* 2004, vol. 13, 933-58

- **GILBERT, C. ; J. BESTMAN-SMITH ; G. BOIVIN.** Resistance of herpesviruses to antiviral drugs: clinical impacts and molecular mechanisms. *Drug Resist. Updat.,* 2002, vol. 5, 88-114
- **GROSSMAN, T. H. ; E. S. KAWASAKI ; S. R. PUNREDDY ; M. S. OSBURNE.** Spontaneous cAMP-dependent derepression of gene expression in stationary phase plays a role in recombinant expression instability. *Gene,* 1998, vol. 209, 95-103
- **JOHNSON, V. A. ; F. BRUN-VEZINET ; B. CLOTET ; B. CONWAY ; R. T. D'AQUILA ; L. M. DEMETER ; D. R. KURITZKES ; D. PILLAY ; J. M. SCHAPIRO ; A. TELENTI.** Update of the drug resistance mutations in HIV-1. *Top. HIV Med.,* 2004, vol. 12, 119-24
- **LIN, C. ; C. A. GATES ; B. G. RAO ; D. L. BRENNAN ; J. R. FULGHUM ; Y.-P. LUONG ; J. D. FRANTZ ; K. LIN ; S. MA ; Y.-Y. WEI.** In vitro studies of cross-resistance mutations against two hepatitis C virus serine protease inhibitors, VX-950 and BILN 2061. *J. Biol. Chem.,* 2005, vol. 280, 36784-36791
- **LIN, C. ; K. LIN ; Y. P. LUONG ; B. G. RAO ; Y. Y. WEI ; D. L. BRENNAN ; J. R. FULGHUM ; H. M. HSIAO ; S. MA ; J. P. MAXWELL.** In vitro resistance studies of hepatitis C virus serine protease inhibitors, VX-950 and BILN 2061: Structural analysis indicates different resistance mechanisms. *J. Biol. Chem.,* 2004, vol. 279, 17508-17514
- **LU, L ; T. J. PILOT-MATIAS ; K. D. STEWART ; J. T. RANDOLPH ; R. PITHAWALLA ; W. HE ; P. P. HUANG ; L. L. KLEIN ; H. MO ; A. MOLLA.** Mutations conferring resistance to a potent hepatitis C virus serine protease inhibitor in vitro. *Antimicrob. Agents Chemother,* 2004, vol. 48, 2260-6
- **MAISNIER-PATIN, S. ; D. I. ANDERSSON.** Adaptation to the deleterious effects of antimicrobial drug resistance mutations by compensatory evolution. *Res. Microbiol.,* 2004, vol. 155, 360-9
- **PAWLOTSKY, J. M ; J. G. MCHUTCHISON.** Hepatitis C. Development of new drugs and clinical trials: promises and pitfalls. Summary of an AASLD hepatitis single topic conference, Chicago. *Hepatology,* 27 February 2003, vol. 39, 554-67
- **SIMMONDS, P.** Genetic diversity and evolution of hepatitis C virus--15 years on. *J. Gen. Virol.,* 2004, vol. 85, 3173-88
- **STRADER, D. B ; T. WRIGHT ; D. L. THOMAS ; L. B. SEEFF.** Diagnosis, management, and treatment of hepatitis C. *Hepatology,* vol. 39, 1147-71
- **TROZZI, C. ; L. BARTHOLOMEW ; A. CECCACCI ; G. BIASIOL ; L. PACINI ; S. ALTAMURA ; F. NARJES ; E. MURAGLIA ; G. PAONESSA ; U. KOCH.** In vitro selection and characterization of hepatitis C virus serine protease variants resistant to an active-site peptide inhibitor. *J. Virol.,* 2003, vol. 77, 3669-79